# EUROPEAN PATENT APPLICATION

(11) **EP 4 556 030 A1**
(43) Date of publication of application: **21.05.2025**
(21) Application number: 23838651.0
(22) Date of filing: 19.06.2023
(51) Int. Cl.: A61K 51/08, C07K 7/06

(54) **POLYPEPTIDE COMPOUND, AND COMPLEX AND USE THEREOF**

(30) Priority: 11.07.2022 CN 202210809938; 05.06.2023 CN 202310661213
(71) Applicant: Hangzhou Healthytide Biotechnology Co., Ltd., Hangzhou, Zhejiang 310051 (CN)
(72) Inventor: GAO, Jian, Hangzhou, Zhejiang 310051 (CN); ZHONG, Guoqing, Hangzhou, Zhejiang 310051 (CN); LI, Sijun, Hangzhou, Zhejiang 310051 (CN); ZUO, Cheng, Hangzhou, Zhejiang 310051 (CN); HUI, Mingliang, Hangzhou, Zhejiang 310051 (CN); LIU, Yanyang, Hangzhou, Zhejiang 310051 (CN)
(74) Representative: V.O.
(86) International application number: PCT/CN2023/101027
(87) International publication number: WO 2024/012157

(57) **Abstract**

Provided are a polypeptide compound, and a complex and use thereof, specifically relating to the technical field of pharmaceutical chemistry. The polypeptide compound is shown as formula I. Compared with the prior art, the provided peptoid compound can target integrin proteins such as αvβ5, α5β1, and αvβ3, is enriched in tumor cells having high integrin expression, and has an important function in diagnosis and/or treatment of tumors.

## Description

This application claims the priority of Chinese Patent Application No. 202210809938.2, filed with the China National Intellectual Property Administration on July 11, 2022, and titled with "POLYPEPTIDE COMPOUND, AND COMPLEX AND USE THEREOF", and the priority of Chinese Patent Application No. 202310661213.8, filed with the China National Intellectual Property Administration on June 5, 2023, and titled with "POLYPEPTIDE COMPOUND, AND COMPLEX AND USE THEREOF", the disclosure of which are hereby incorporated by reference in they entirety.

### FIELD

The present disclosure relates to the technical field of pharmaceutical chemistry, and in particular to a polypeptide-like compound, complex and use thereof.

### BACKGROUND

Integrin, as a transmembrane glycoprotein commonly found on cells, is one of the members of the cell adhesion molecule family, and a predominant molecule mediating cell to extracellular matrix (ECM) interaction. Integrin is a cell-surface receptor, which can recognize and bind to the corresponding ligands in the extracellular matrix, provide attachment sites for cell adhesion, regulate cell motility, proliferation and apoptosis, and mediate cell-cell and cell-extracellular matrix recognition and adhesion, thereby playing a role in linking extracellular environment with the internal structure of the cell. Integrins are heterodimers that are formed through non-covalent association of two subunits, alpha (α) and beta (β), in a 1:1 ratio. Integrin consists of three parts: an extracellular region, a transmembrane region, and an intracellular region, which form a spherical region through linking in the extracellular region, and contain a divalent cation-binding domain. Based on the specificity of the integrin recognizing ligands, integrins can be broadly classified into arginine-glycine-aspartate (RGD) tripeptide-recognizing integrins, laminin-binding integrins, leukocyte integrins, and collagen-binding integrins. Integrins bind to ECM proteins-collagen, laminin, vitronectin, fibronectin or some cell surface molecules through the extracellular domain. Integrins are involved in many important physiological processes, including embryogenesis, blood coagulation, and maintenance of tissue and organ integrity in vivo. Many pathological response processes, such as inflammation, thrombosis, neoangiogenesis, and malignant tumor growth, infiltration and metastasis, are associated with aberrant regulation of integrins.

Dysregulation of integrin expression on cancer cells has been extensively studied in cellular and animal models and it has been shown that targeting integrins can inhibit tumor growth, reduce resistance to chemotherapy or radiotherapy, and attenuate invasion and metastasis. Studies using transgenic mouse models or using transplanted tumor immunodeficient mouse modelshave shown that tumor growth, metastasis and resistance to chemotherapy or radiotherapy, can be interfered by the absence of integrins in cancer cells or blocking integrin function by blocking antibodies or peptides. β1 integrins have dual roles in growth and metastasis, suggesting that they could be potential therapeutic targets. Integrins, such as αvβ3, αvβ5 and α5β1, are not only expressed on tumor cells but also induced on endothelial cells during angiogenesis. These integrins have been shown to be targets for anti-angiogenic therapy in cancer.

Recent studies have identified a series of other subtypes of integrins involved in carcinogenesis, including pre-metastatic niche, epithelial-mesenchymal transition (EMT), metabolic reorganization, and the establishment of stemness and dormancy in cancer cells. The TGFβ activation involved by integrin αvβ6 is related to SOX4-mediated cancer immune evasion, in which αvβ6-blocking antibodies inhibit SOX4 expression and sensitize a triple-negative breast cancer mouse model to T-cell-mediated killing responses induced by immune checkpoint inhibitor. Integrin αvβ8 can be used as a target to modulate anti-tumor immunity. The αvβ8 blocking antibody or specific blockade of the integrin αvβ8 on the surface of regulatory T cells can attenuate TGFβ-mediated inhibition on CD8⁺ T cells, thereby restoring the tumor-killing capacity of CD8⁺ T cells, and synergistically working with radiation therapy or immunotherapy.

Expression of the integrin αvβ3 is widely associated with the progression of melanoma from the early radial growth stage to aggressive vertical growth and metastasis. Studies have found that αvβ3 is significantly upregulated in malignant primary neuroendocrine prostate cancer and its lung metastatic lesions, and αVβ3 may serve as a biomarker for early detection of malignant transformation of prostate cancer. Expression of integrin αvβ5 is considered a predictive biomarker for several types of cancer, including breast, liver, and gastric cancers. Elevated levels of αvβ5 have been detected in patients with glioblastoma or colorectal cancer, and overexpression of αvβ5 has been associated with unfavorable overall survival rate. Integrin α5β1 is involved in tumor-promoting processes such as angiogenesis, proliferation and metastasis. For patients with early-stage breast cancer, expression of α5β1 in the primary tumor is associated with the presence of diffuse tumor cells in bone marrow aspirates and poor metastasis-free survival. Likewise, studies showed that α5 gene silencing or pharmacological inhibition of α5β1 with volociximab attenuated bone colonization of mice after intravenous injection of tumor cells. Integrin α5β1 has also been found to be upregulated in some gastrointestinal tumors, where enhanced expression of ITGAS corresponds to poor prognosis.

Integrin proteins such as αvβ3, αvβ5 and α5β1 become drug targets in targeted therapy of tumor due to their abnormal expression in various tumors and their roles in tumor development. The drug molecules targeting integrin can either kill the tumor cells directly, or act as a targeting carrier to deliver cytotoxins, nuclides, and the like, to the tumor cells so as to achieve tumor killing. In addition, the combination of nuclides, fluorescent molecules and integrin-targeting molecules can be applied in the fields such as tumor diagnosis and surgical navigation.

Therefore, the study on drugs inhibiting integrin is of great significance.

### SUMMARY

In view of this, an object of the present disclosure is to provide a polypeptide-like compound, a coordination complex thereof, and use thereof. The polypeptide-like compound provided by the present disclosure can target integrin proteins such as αvβ5, α5β1, and αvβ3, which inhibit the growth and proliferation of tumor cells, and play an important role in the diagnosis or treatment of tumors.

In order to achieve the above objects, the present disclosure adopts the following technical solutions.

The present invention provides a polypeptide-like compound, wherein the polypeptide-like compound is prepared from PD and ZT, or from PD, ZT and linker;
wherein, ZT has a structure represented by formula I-ZT: 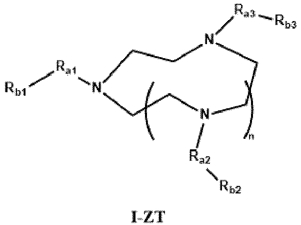
in formula I-ZT, n is an integer selected from 1 to 8,
Rₐ₁, Rₐ₂, and Rₐ₃ are each selected from the group consisting of substituted or unsubstituted alkylidene hypophosphorous acid group, substituted or unsubstituted alkylidene phosphorous acid group, substituted or unsubstituted C1-C6 alkylene, substituted or unsubstituted C5-C20 arylidene, substituted or unsubstituted C1-C20 heteroarylidene, substituted or unsubstituted C1-C6 alkyleneoxy, substituted or unsubstituted C5-C20 aryleneoxy, substituted or unsubstituted C1-C6 alkylidene sulfonyl, substituted or unsubstituted C1-C6 alkylidene sulfonic acid group, substituted or unsubstituted C1-C6 alkylidene sulfonate group, and substituted or unsubstituted C1-C6 alkylidene phosphate group,
R_{b1}, R_{b2}, and R_{b3} are each selected from the group consisting of hydrogen, hydroxyl, carboxyl, halogen, mercapto, seleno, amido, amino, cyano, nitro, substituted or unsubstituted hypophosphorous acid group, substituted or unsubstituted phosphorous acid group, substituted or unsubstituted C1-C6 hydrocarbyl, substituted or unsubstituted C5-C20 aryl, substituted or unsubstituted C1-C20 heteroaryl, substituted or unsubstituted C1-C6 alkoxy, substituted or unsubstituted C5-C20 aryloxy, substituted or unsubstituted C1-C6 sulfonyl, substituted or unsubstituted C1-C6 sulfonic acid group, substituted or unsubstituted C1-C6 sulfonate group, and substituted or unsubstituted C1-C6 phosphate group,
at least one of R_{b1} and R_{b3} is selected from the group consisting of carboxyl, amino and hydroxyl;
linker has a structure represented by formula I-linker: 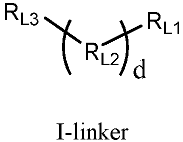
   in formula I-linker, d is an integer selected from 0 to 17,
   R_{L1}, and R_{L3} are each selected from the group consisting of -(CH₂)_{d1}COOH, amino, mercapto, carboxyl, and hydroxyl, wherein d1 is selected from 0 to 13,
   R_{L2} is selected from the group consisting of -Arg-Gly-Asp-, -Arg-Gly-, -Arg-, imino, nitrilo, imido, substituted or unsubstituted C1-C20 alkylene, substituted or unsubstituted C1-C20 alkylidene ether, and substituted or unsubstituted C5-C20 arylidene ether;
   PD is a compound and/or a derivative thereof obtained by condensing one or more of: 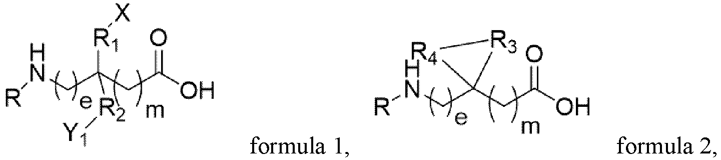 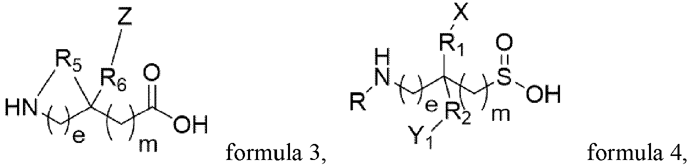 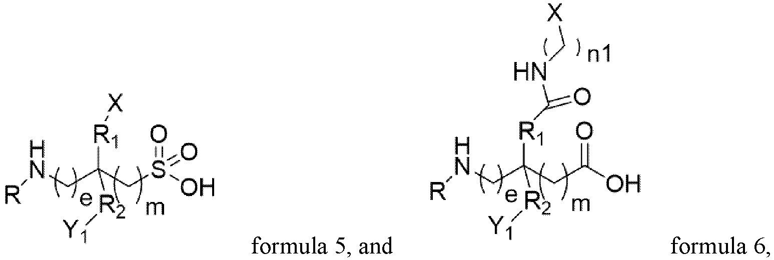
      in formula 1 to formula 6, m, e and n₁ are each an integer independently selected from 0 to 5,
      R is selected from the group consisting of hydrogen, -OH, -NH₂, substituted or unsubstituted C1-C6 hydrocarbyl, substituted or unsubstituted C5-C20 aryl, and substituted or unsubstituted C1-C20 heteroaryl,
      R₁, R₂, R₃, R₄, R₅, and R₆ are independently selected from the group consisting of substituted or unsubstituted C1-C6 alkylene, substituted or unsubstituted C5-C20 arylidene, and substituted or unsubstituted C1-C20 heteroarylidene;
      X, Y₁, and Z are independently selected from the group consisting of hydrogen, halogen, substituted or unsubstituted C1-C6 alkyl, substituted or unsubstituted C1-C6 alkoxy, substituted or unsubstituted C5-C20 aryloxy, -O-(CH2)q-O-R", mercapto, seleno, amido, amino, hydroxyl, cyano, nitro, carboxyl , substituted or unsubstituted C1-C6 ester, substituted or unsubstituted C1-C6 sulfonyl, substituted or unsubstituted C1-C6 sulfonic acid group, substituted or unsubstituted C1-C6 sulfonate group, sulfonamido group, phosphoric acid group, substituted or unsubstituted C1-C6 phosphate group, substituted or unsubstituted C5-C20 aryl, substituted or unsubstituted C5-C20 aryl heterocyclyl, substituted or and unsubstituted C2-C6 heterocyclyl; wherein, the substituted C1-C6 alkyl, substituted C1-C6 alkyl ether, and substituted C5-C20 aryl ether have a substituent group selected from the group consisting of -SH, SeH, hydroxyl, amino, carboxyl, sulfonic acid group , phosphoric acid group, halogen, and a combination thereof; q is 1 or 2; and R" is a C1-C6 alkyl;
      when R₁, R₂, and R₆ are H, X, Y₁, Z and 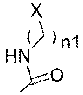 are absent; and
      when R₃, R₄, and R₅ are H, R₃ and R₄ do not form a ring, and R₅ together with the C atom to which it is bounded do not form a ring.

In the present disclosure, in formula I-ZT, Rₐ₁, Rₐ₂, and Rₐ₃ are each selected from the group consisting of substituted or unsubstituted alkylidene hypophosphorous acid group, substituted or unsubstituted alkylidene phosphorous acid group, substituted or unsubstituted C1-C6 alkylene, substituted or unsubstituted C6-C20 arylidene, substituted or unsubstituted C1-C20 heteroarylidene, substituted or unsubstituted C1-C6 alkyleneoxy, substituted or unsubstituted C6-C20 aryleneoxy, substituted or unsubstituted C1-C6 alkylidene sulfonyl, substituted or unsubstituted C1-C6 alkylidene sulfonic acid group, substituted or unsubstituted C1-C6 alkylidene sulfonate group, and substituted or unsubstituted C1-C6 alkylidene phosphate group.

R_{b1}, R_{b2}, and R_{b3} are each selected from the group consisting of hydrogen, hydroxyl, carboxyl, halogen, mercapto, seleno, amido, amino, cyano, nitro, substituted or unsubstituted hypophosphorous acid group, substituted or unsubstituted phosphorous acid group, substituted or unsubstituted C1-C6 hydrocarbyl, substituted or unsubstituted C6-C20 aryl, substituted or unsubstituted C1-C20 heteroaryl, substituted or unsubstituted C1-C6 alkoxy, substituted or unsubstituted C6-C20 aryloxy, substituted or unsubstituted C1-C6 sulfonyl, substituted or unsubstituted C1-C6 sulfonic acid group, substituted or unsubstituted C1-C6 sulfonate group, and substituted or unsubstituted C1-C6 phosphate group;
at least one of R_{b1} and R_{b3} is selected from the group consisting of carboxyl, amino and hydroxyl.

In the present disclosure, in formula I-linker, R_{L1} is an amino or carboxyl.

R_{L2} is selected from the group consisting of imino, nitrile, imido, substituted or unsubstituted C1-C20 alkylene, substituted or unsubstituted C1-C20 alkylidene ether, and substituted or unsubstituted C6-C20 arylidene ether.

In the present disclosure, in formula 1 to formula 6, R is selected from the group consisting of hydrogen, -OH, -NH₂, substituted or unsubstituted C1-C6 hydrocarbyl, substituted or unsubstituted C6-C20 aryl, and substituted or unsubstituted C1-C20 heteroaryl;
R₁, R₂, R₃, R₄, R₅, and R₆ are independently selected from the group consisting of substituted or unsubstituted C1-C6 alkylene, substituted or unsubstituted C6-C20 arylidene, and substituted or unsubstituted C1-C20 heteroarylidene;
X, Y₁, and Z are independently selected from the group consisting of hydrogen, halogen, substituted or unsubstituted C1-C6 alkyl, substituted or unsubstituted C1-C6 alkoxy, substituted or unsubstituted C6-C20 aryloxy, -O-(CH₂)_{q}-O-R", mercapto, seleno, amido, amino, hydroxyl, cyano, nitro, carboxyl, substituted or unsubstituted C1-C6 ester, substituted or unsubstituted C1-C6 sulfonyl, substituted or unsubstituted C1-C6 sulfonic acid group, substituted or unsubstituted C1-C6 sulfonate group, sulfonamido group, phosphoric acid group, substituted or unsubstituted C1-C6 phosphate group, substituted or unsubstituted C6-C20 aryl, substituted or unsubstituted C1-C20 arylheterocyclyl, substituted or unsubstituted C3-C6 heterocyclyl; wherein, the substituted C1-C6 alkyl, substituted C1-C6 alkyl ether, and substituted C6-C20 aryl ether have a substituent group selected from the group consisting of -SH, SeH, hydroxyl, amino, carboxyl, sulfonic acid group , phosphoric acid group, halogen, and a combination thereof; q is 1 or 2; and R" is a C1-C6 alkyl;
when R₁, R₂, and R₆ are H, X, Y₁, Z, and 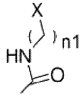 are absent;
when R₃, R₄, and R₅ are H, R₃ and R₄ do not form a ring, and R₅ together with C atom to which it is bounded do not form a ring.

In the present disclosure, in formula I-ZT, n is an integer selected from 1 to 4;
Rₐ₁, Rₐ₂, and Rₐ₃ are each selected from the group consisting of substituted or unsubstituted alkylidene hypophosphorous acid group, substituted or unsubstituted alkylidene phosphorous acid group, substituted or unsubstituted C1-C6 alkylene, and substituted or unsubstituted C1-C6 alkyleneoxy;
R_{b1}, R_{b2}, and R_{b3} are each selected from the group consisting of hydrogen, hydroxyl, carboxyl, halogen, amino, substituted or unsubstituted hypophosphorous acid group, substituted or unsubstituted phosphorous acid group, substituted or unsubstituted C1-C6 hydrocarbyl, and substituted or unsubstituted C1-C6 alkoxy;
at least one of R_{b1} and R_{b3} is selected from the group consisting of carboxy, amino and hydroxyl.

In the present disclosure, the linkage of PD and ZT, or the linkage of PD, ZT and linker form an amide bond, a sulfonamide bond, a sulfenamide bond, an ester bond, a carbonate bond, or a urea structure, and the condensation of amino preferably includes the condensation of amino and carboxyl to a amide bond;
the condensation of amino and sulfonic acid group to a sulfonamide bond;
the amino-amino condensation to a urea structure;
the condensation of amino and hydroxyl to a carbonate bond;
the condensation of carboxyl and hydroxyl to a ester bond; and/or;
the condensation of amino and sulfinic acid group to a sulfinamide bond.

In the present disclosure, Rₐ₁, Rₐ₂, and Rₐ₃ are each selected from the group consisting of C1-C3 alkylidene, C1-C3 alkyleneoxy, C1-C3 alkylidene hypophosphorous acid group, and C1-C3 alkylidene phosphorous acid group.

R_{b1}, R_{b2}, R_{b3} are each selected from the group consisting of hydrogen, hydroxyl, carboxyl, amino, C1-C3 alkylidene hypophosphorous acid group, C1-C3 alkylidene phosphorous acid group, C1-C3 hydrocarbyl, and C1-C3 alkoxy.

At least one of R_{b1} and R_{b3} is selected from the group consisting of carboxy, amino and hydroxyl.

In the present disclosure, ZT has a structure selected from formula ZT1 to ZT4:

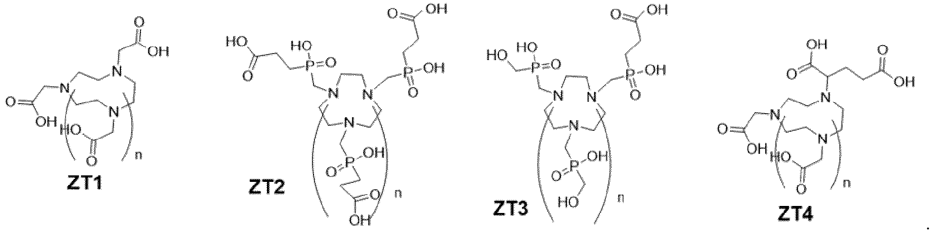

In formula ZT1 to ZT4, n is an integer from 0 to 4.

Preferably, ZT has a structure represented by formula ZT1, wherein n is 1 or 2;

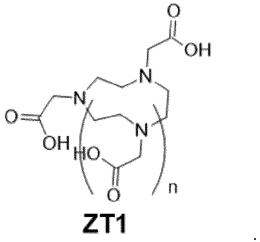

Preferably, linker has a structure selected from the group consisting of formula linker 1 to linker 9:

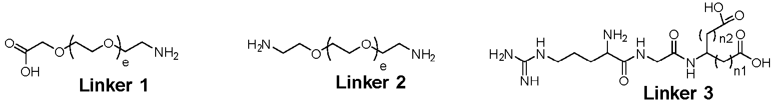

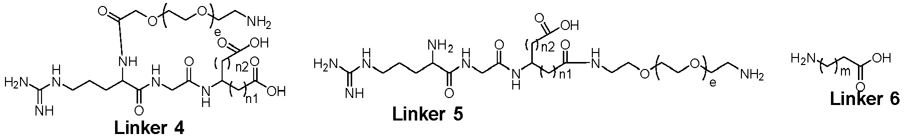

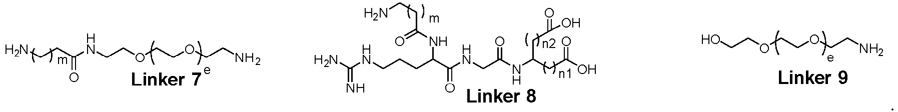

In formula linker 1 to linker 9, e, m, n₁, and n₂ are each an integer independently selected from 0 to 16.

Preferably, the polypeptide-like compound is selected from the group consisting of formula L1 to formula L14:

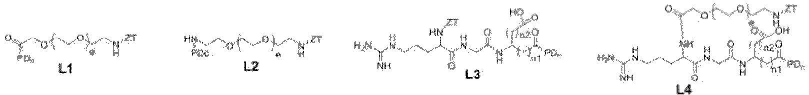

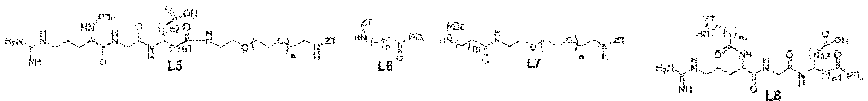

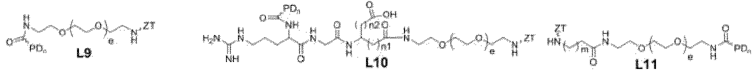

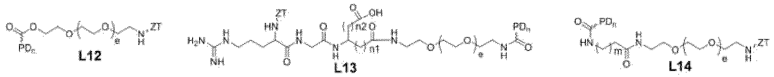

In formula L1 to formula L14, linker is connected to PD and ZT through an amide bond, ester bond, carbonate bond or urea structure; PDc indicates that C-terminal end of the PD fragment forms an amide bond with the N-terminal end of linker, and PDn indicates that the N-terminal end of the PD fragment forms an amide bond with C-terminal end of linker.

Preferably, PD is synthesized from one or several amino acids or amino acid-like substances of formula 1 to 6 by the condensation of the amino group with the carboxyl group to an amide bond, and/or by the condensation of amino and sulfonic acid group to a sulfonamide bond; and/or by the condensation of amino and sulfinic acid group to a sulfinamide bond. Preferably, the polypeptide-like compound has preferably four amide bonds, sulfonamide bonds and/or sulfinamide bonds, preferably by condensation of five amino acids or amino acid-like substances. The structure of the amino acids or amino acid-like substances has preferably N-terminal end at the left end and C-terminal end at the right end.

Preferably, m, e, and n₁ in formula 1 to formula 6 are independently selected from the group consisting of 0, 1, 2, 3, 4 and 5.

Preferably, R in formula 1 to formula 6 is an alkyl with 1 to 20 carbon atoms, more preferably an alky with 1 to 15 carbon atoms, more preferably an alkyl with 1 to 10 carbon atoms, more preferably an alkyl with 1 to 5 carbon atoms, and most preferably methyl or ethyl.

Preferably, the substituents in the substituted alkylene, substituted arylidene, and substituted heteroarylidene in R₁, R₂, R₃, R₄, R₅, and R₆ in formula 1 to formula 6 are each selected from the group consisting of halogen, halogenated alkyl, lower alkyl, alkoxy, -O-(CH₂)_{q}-O-R", alkylthio (or seleno) substituent, halogenated alkylthio (or seleno)substituent, mercapto (or selen), alkylthio (or seleno) ether, arythio (or seleno) ether, hydroxyl, cyano, nitro, carboxylic derivative group, aryloxy, amido, acyl amine, amino, hydroxylamine, alkyl amine, dialkyl amine, trifluoroalkoxy, trifluoromethyl,, ester, sulfonyl, alkyl sulfonyl, halogenated alkyl sulfonyl, sulfonic acid group, sulfonamido group, phosphoric acid group, phosphate group, unsubstituted aryl" aryl and heterocyclic, monocyclic heterocyclic group, N, O, SH, SeH, hydrogen,, carboxyl, methylthio (or seleno) ether group, ethylthio (or seleno) ether group, -SOMe, -SO₂Me, -CONH₂, - COOMe, -COOEt, -NHOCH, -NHOCCH₃, -NHSO₂Me, p-toluenesulfonamido group, and sulfonate group. Wherein, q is 1 or 2, and R" is a C1-C6 alkyl.

The alkylene in R₁, R₂, R₃, R₄, R₅, and R₆ in formula 1 to formula 6 is a hydrocarbyl with 1 to 20 carbon atoms, more preferably a hydrocarbyl with 1 to 10 carbon atoms. The hydrocarbyl is preferably an alkyl, an alkynyl, an alkenyl or an aliphatic chain.

The substituent group in the substituted alkylene in R₁, R₂, R₃, R₄, R₅, and R₆ in formula 1 to formula 6 is selected from the group consisting of N, O, SH and SeH.

The arylidene in R₁, R₂, R₃, R₄, R₅, and R₆ in formula 1 to formula 6 is selected from the group consisting of an aliphatic ring and an aromatic ring.

The substituent group in the substituted arylidene or substituted heteroarylidene in R₁, R₂, R₃, R₄, R₅, and R₆ in formula 1 to formula 6 is selected from the group consisting of -F, -Cl, -Br, - I, -OH, -NH₂, -NHOH, -COOH, -COH, -SO₃H, -SH, -SMe, -SEt, -OPO₃H, -NO₂, -NO, -SOMe, - SO₂Me, -SO₂Me, -SOMe, -SO₂Me, -SOMe, -SOMe SO₂Me, -SOEt, -SO₂Et, -CONH₂, -COOMe, -COOEt, -NHOCH, -NHOCCH₃, -NHSO₂Me, -OSO₂Ph, a phosphate group, and a sulfonate group.

X, Y₁, and Z in formula 1 to formula 6 are independently selected from the group consisting of hydrogen, amino, carboxyl, mercapto, -SMe, -SEt, phosphoric acid group, nitro, -F, - Cl, -Br, -I, -CHF₂, -CF₃, -NHOH, -SO₃H, -SOMe, -SO₂Me, -SOEt, -SO₂Et, -CONH₂, -COOMe, - COOEt, -NHOCH, -NHOCCH₃, -NHSO₂Me, -SeMe, -SeEt, p-toluenesulfonamido group, phosphate group, sulfonate group, halogen, halogenated alkyl, lower alkyl, alkoxy, methylenedioxy, ethylenedioxy, alkylthio (or seleno) substituent, halogenated alkylthio (or seleno) substituent, mercapto (or selen), alkylthio (or seleno) ether, arythio (or seleno) ether, hydroxyl, cyano, carboxylic derivative group, aryloxy, amido, acyl amine, hydroxylamine, alkyl amine, dialkyl amine, trifluoroalkoxy, trifluoromethyl, ester, sulfonyl, alkyl sulfonyl, halogenated alkyl sulfonyl, sulfonic acid group, sulfonamido group, phosphoric acid group, unsubstituted aryl, aryl and heterocyclic, monocyclic heterocyclic group, methylthio (or seleno) group, ethylthio (or seleno) group, and -NHOH.

The compound (amino acid or amino acid-like substance) having the structure represented by formula 1 to formula 6 is selected from the group consisting of Gln, Glu, Asn, Asp, homoaspartic acid, homoasparagine, homoglutamine, homoglutamic acid, Arg-Gly-Asp, Gly, Ala, Leu, Val, Leu, Phe, Ser, Thr, Phe, Tyr, Tyr (Me), Tyr (Et), and a combination thereof. In the compound of formula 1 to formula 6; the amino acids preferably has one or two structures of L-configuration and D-configuration.

Preferably, PD has a structure selected from the group consisting of formula II to formula VII:

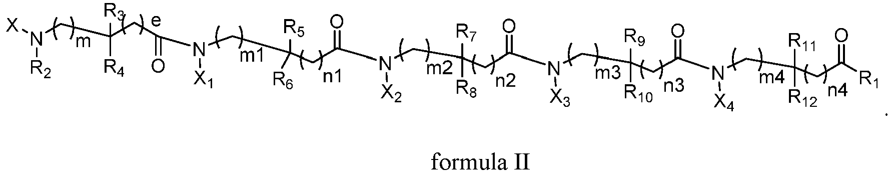

In formula II, the selection ranges of m, e, n₁, n₂, n₃, n₄, m₁, m₂, m₃, and m₄ are consistent with the selection ranges of m, e, and n₁ in formulas 1 to 6 in the technical solution above.

R1 is selected from the group consisting of -H, -OH, -NH₂, -NHNZ¹Z², -NHZ¹, -NZ¹Z², -OZ¹, -NHOZ¹, -SH, -SeH, -SZ¹, -SeZ¹, -Arg-Gly-Asp-Z³, aliphatic derivatized group formed by linking with carbonyl, and an amine derivatized group. Z¹ and Z² are independently selected from alkyl, and Z³ is selected from the group consisting of hydrogen, hydroxyl, amino, ester derivatized group linked to Asp C-terminal end, amine derivative group, and hydrocarbyl or substituted hydrocarbyl.

R₂ is selected from the group consisting of hydrogen, -OH, -NH₂, hydrocarbyl or substituted hydrocarbyl, acyl derivative group, sulfonyl derivative group, and XS-Arg-Gly-Asp-.

R₃, R₄, R₅, R₆, R₇, R₈, R₉, R₁₀, R₁₁, and R₁₂ are independently selected from the group consisting of hydrogen, hydrocarbyl or substituted hydrocarbyl, heteroatom-substituted hydrocarbyl, alkene group or alkene derivatives, alkynyl or alkynyl derivatives, aryl or substituted aryl, and heteroaryl or substituted heteroaryl.

X, X₁, X₂, X₃, X₄, and X₅ are independently selected from the group consisting of hydrogen, hydroxyl, amino, hydrocarbyl, and substituted hydrocarbons.

Preferably, PD has a structure represented by formula III:

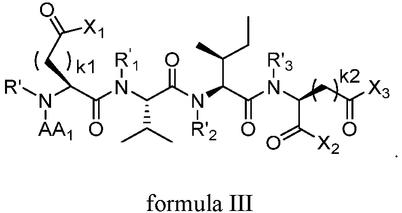

In formula III, k₁ and k₂ are independently selected from 0 to 16;
R', R'₁, R'₂, and R'₃ are independently selected from the group consisting of hydrogen, hydroxyl, amino, and hydrocarbyl or substituted hydrocarbyl.

X₁, X₂ and X₃ are independently selected from the group consisting of -H, -OH, -NH₂, - NHNZ¹Z², -NHZ¹, -NZ¹Z², -OZ¹, -NHOZ¹, -SH, -SeH, -SZ¹, -SeZ¹, -Arg-Gly-Asp-Z³, aliphatic derivatized group formed by linking with carbonyl, and amine derivatized group. Z¹ and Z² are independently selected from alkyl, and Z³ is selected from the group consisting of hydrogen, hydroxyl, amino, ester derivatized group linked to Asp C-terminal end, amine derivative group, and hydrocarbyl or substituted hydrocarbyl.

AA₁ has a structure selected from the group consisting of formula 7 to formula 12:

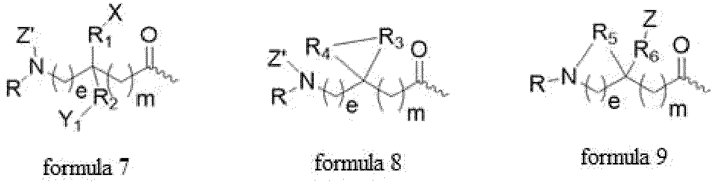

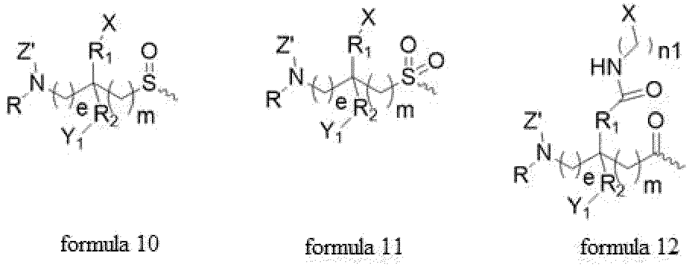

In formula 7 to formula 12, the acyl end of AA1 is coupled to the N-terminal of the main chain;
m, e, and n₁ are independently selected from 0 to 5;
R and Z' are independently selected from the group consisting of hydrogen, -OH, -NH2, hydrocarbyl or substituted hydrocarbyl, acyl derivative group, and sulfonyl derivative group; and
R₁, R₂, R₃, R₄, R₅, and R₆ are independently selected from the group consisting of hydrogen, alkylene or a substituted alkylene, alkenylene or alkenylene derivative, ynylene or ynylene derivative, arylidene and substituted arylidene, and heteroarylidene or substituted heteroarylidene.

When R₁, R₂, R₃, R₄, R₅, and R₆ are selected from hydrogen substitution, X, Y₁, and Z are unsubstituted.

When R₁, R₂, R₃, R₄, R₅, and R₆ are selected from non-hydrogen substituted group, X, Y₁, and Z are independently selected from the group consisting of hydrogen, halogen, halogenated alkyl, lower alkyl, alkoxy, methylenedioxy, ethylenedioxy, alkylthio (or seleno) substituent, halogenated alkylthio (or seleno)substituent, mercapto (or selen), alkylthio (or seleno) ether, arythio (or seleno) ether, hydroxyl, cyano, nitro, carboxylic derivative group, aryloxy, amido, acyl amine, amino, hydroxylamine, alkyl amine, dialkyl amine, trifluoroalkoxy, trifluoromethyl, ester, sulfonyl, alkyl sulfonyl, halogenated alkyl sulfonyl, sulfonic acid group, sulfonamido group, phosphoric acid group, phosphate group, unsubstituted aryl, aryl and heterocyclic, and monocyclic heterocyclic group.

Preferably, PD has a structure represented by formula IV:

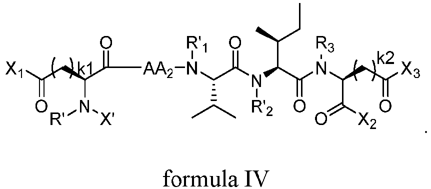

In formula IV, k₁ and k₂ are independently selected from 0 to 16.

R', R'₁, R'₂ and R₃ are independently selected from the group consisting of hydrogen, hydroxyl, and hydrocarbyl or substituted hydrocarbyl.

X' is selected from the group consisting of hydrogen, and hydrocarbyl, or substituted hydrocarbyl.

X₁, X₂ and X₃ are independently selected from -H, -OH, -NH₂, -NHNZ¹Z², -NHZ¹, - NZ¹Z², -OZ¹, -NHOZ¹, -SH, -SeH, -SZ¹, -SeZ¹, -Arg-Gly-Asp-Z³, aliphatic derivatized group formed by linking with carbonyl, and amine derivatized group. Z¹ and Z² are independently selected from alkyl, and Z³ is selected from the group consisting of hydrogen, hydroxyl, amino, ester derivatized group linked to Asp C-terminal end, amine derivative group, and hydrocarbyl or substituted hydrocarbyl.

AA₂ has a structure selected from the group consisting of formula 13 to formula 18:

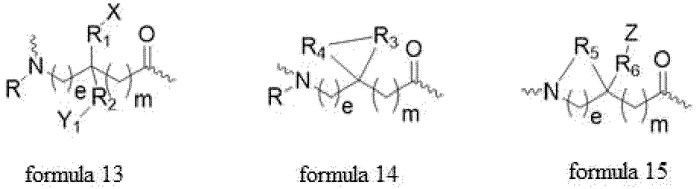

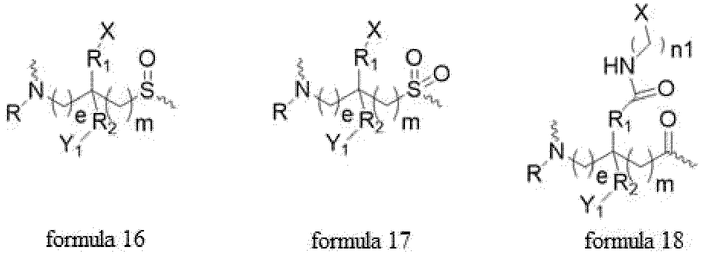

In formula 13 to formula 18, the acyl end of AA₂ is coupled to the N-terminal end of the main chain; and the acyl end of the main chain is coupled to the N-terminal end of the AA₂ general formula.

m, e, and n₁ are independently selected from 0 to 5.

R is selected from the group consisting of hydrogen, -OH, -NH2, and hydrocarbyl or substituted hydrocarbyl.

R₁, R₂, R₃, R₄, R₅, and R₆ are independently selected from the group consisting of hydrogen, alkylene or a substituted alkylene, alkenylene or alkenylene derivative, ynylene or ynylene derivative, arylidene and substituted arylidene, and heteroarylidene or substituted heteroarylidene.

When R₁, R₂, R₃, R₄, R₅, and R₆ are selected from hydrogen substitution, X, Y₁, and Z are unsubstituted.

When R₁, R₂, R₃, R₄, R₅, and R₆ are selected from non-hydrogen substituted group, X, Y₁, and Z are independently selected from the group consisting of hydrogen, halogen, halogenated alkyl, lower alkyl, alkoxy, methylenedioxy, ethylenedioxy, alkylthio (or seleno) substituent, halogenated alkylthio (or seleno)substituent, mercapto (or selen), alkylthio (or seleno) ether, arythio (or seleno) ether, hydroxyl, cyano, nitro, carboxylic derivative group, aryloxy, amido, acyl amine, amino, hydroxylamine, alkyl amine, dialkyl amine, trifluoroalkoxy, trifluoromethyl, ester, sulfonyl, alkyl sulfonyl, halogenated alkyl sulfonyl, sulfonic acid group, sulfonamido group, phosphoric acid group, phosphate group, unsubstituted aryl, aryl and heterocyclic, and monocyclic heterocyclic group.

PD has a structure represented by formula V:

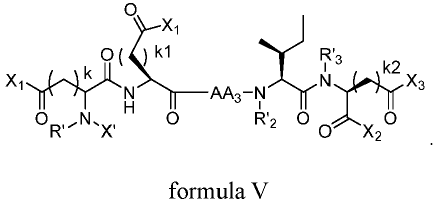

In formula V, k, k₁ and k₂ are independently selected from 0 to 16.

R', R'₂ and R₃ are independently selected from the group consisting of hydrogen, hydroxyl, amino, and hydrocarbyl or substituted hydrocarbyl.

X' is selected from the group consisting of hydrogen, and hydrocarbyl, or substituted hydrocarbyl.

X₁, X₂ and X₃ are independently selected from -H, -OH, -NH₂, -NHNZ¹Z², -NHZ¹, - NZ¹Z², -OZ¹, -NHOZ¹, -SH, -SeH, -SZ¹, -SeZ¹, -Arg-Gly-Asp-Z³, aliphatic derivatized group formed by linking with carbonyl, and amine derivatized group. Z¹ and Z² are independently selected from alkyl, and Z³ is selected from the group consisting of hydrogen, hydroxyl, amino, ester derivatized group linked to Asp C-terminal end, amine derivative group, and hydrocarbyl or substituted hydrocarbyl.

The selection range of AA₃ is consistent with the selection range of AA₂ in formula III described in the above technical solution.

PD has a structure represented by formula VI:

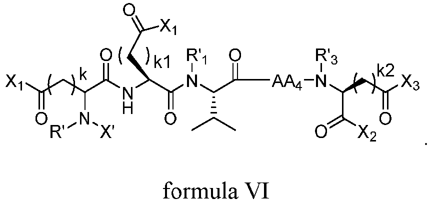

In formula VI, k, k₁ and k₂ are independently selected from 0 to 16.

R', R'₁, and R₃ are independently selected from the group consisting of hydrogen, hydroxyl, amino, and hydrocarbyl or substituted hydrocarbyl.

X' is selected from the group consisting of hydrogen, hydrocarbyl, and substituted hydrocarbyl.

X₁, X₂ and X₃ are independently selected from -H, -OH, -NH₂, -NHNZ¹Z², -NHZ¹, - NZ¹Z², -OZ¹, -NHOZ¹, -SH, -SeH, -SZ¹, -SeZ¹, -Arg-Gly-Asp-Z³, aliphatic derivatized group formed by linking with carbonyl, and amine derivatized group. Z¹ and Z² are independently selected from alkyl, and Z³ is selected from the group consisting of hydrogen, hydroxyl, amino, ester derivatized group linked to Asp C-terminal end, amine derivative group, and hydrocarbyl or substituted hydrocarbyl.

The selection range of AA₄ is consistent with the selection range of AA₂ in formula III described in the above technical solution.

PD has a structure represented by formula VII:

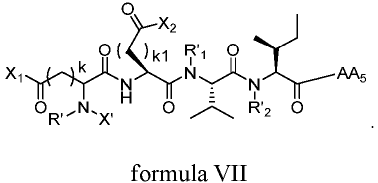

In formula VII, k and k₁ are independently selected from 0 to 16.

R', R'₁, and R'₂ are independently selected from the group consisting of hydrogen, hydroxyl, amino, and hydrocarbyl or substituted hydrocarbyl.

X' is selected from the group consisting of hydrogen, and hydrocarbyl, or substituted hydrocarbyl.

X₁, and X₂ are independently selected from -H, -OH, -NH₂, -NHNZ¹Z², -NHZ¹, -NZ¹Z², -OZ¹, -NHOZ¹, -SH, -SeH, -SZ¹, -SeZ¹, -Arg-Gly-Asp-Z³, aliphatic derivatized group formed by linking with carbonyl, and amine derivatized group. Z¹ and Z² are independently selected from alkyl, and Z³ is selected from the group consisting of hydrogen, hydroxyl, amino, ester derivatized group linked to Asp C-terminal end, amine derivative group, and hydrocarbyl or substituted hydrocarbyl.

AA₅ has a structure selected from the group consisting of formula 19 to formula 24:

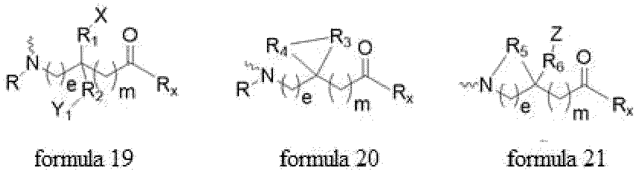

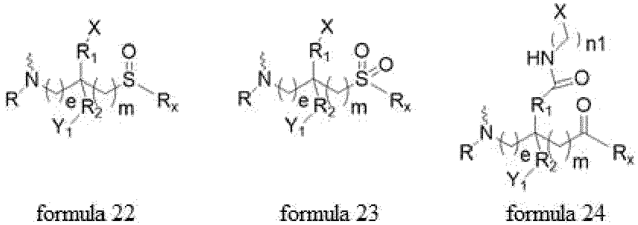

In formula 19 to formula 24, the acyl end of the main chain is coupled to the N-terminal end of the AA₅ general formula.

m, e, and n₁ are independently selected from 0 to 5.

R is selected from the group consisting of hydrogen, -OH, -NH2, and hydrocarbyl or substituted hydrocarbyl.

R₁, R₂, R₃, R₄, R₅, and R₆ are independently selected from the group consisting of hydrogen, alkylene or a substituted alkylene, alkenylene or alkenylene derivative, ynylene or ynylene derivative, arylidene and substituted arylidene, and heteroarylidene or substituted heteroarylidene.

When R₁, R₂, R₃, R₄, R₅, and R₆ are selected from hydrogen substitution, X, Y₁, and Z are unsubstituted.

When R₁, R₂, R₃, R₄, R₅, and R₆ are selected from non-hydrogen substituted group, X, Y₁, and Z are independently selected from the group consisting of hydrogen, halogen, halogenated alkyl, lower alkyl, alkoxy, methylenedioxy, ethylenedioxy, alkylthio (or seleno) substituent, halogenated alkylthio (or seleno)substituent, mercapto (or selen), alkylthio (or seleno) ether, arythio (or seleno) ether, hydroxyl, cyano, nitro, carboxylic derivative group, aryloxy, amido, acyl amine, amino, hydroxylamine, alkyl amine, dialkyl amine, trifluoroalkoxy, trifluoromethyl, ester, sulfonyl, alkyl sulfonyl, halogenated alkyl sulfonyl, sulfonic acid group, sulfonamido group, phosphoric acid group, phosphate group, unsubstituted aryl, aryl and heterocyclic, and monocyclic heterocyclic group.

Rₓ is independently selected from -H, -OH, -NH₂, -NHNZ¹Z², -NHZ¹, -NZ¹Z², -OZ¹, - NHOZ¹, -SH, -SeH, -SZ¹, -SeZ¹, -Arg-Gly-Asp-Z³, aliphatic derivatized group formed by linking with carbonyl, amine derivatized group, alkene group or alkene derivatives, alkynyl or alkynyl derivatives, aryl or substituted aryl, and heteroaryl or substituted heteroaryl. Z¹ and Z² are independently selected from alkyl, and Z³ is selected from the group consisting of hydrogen, hydroxyl, amino, ester derivatized group linked to Asp C-terminal end, amine derivative group, and hydrocarbyl or substituted hydrocarbyl.

Further preferably, PD has a structure selected from the group consisting of:

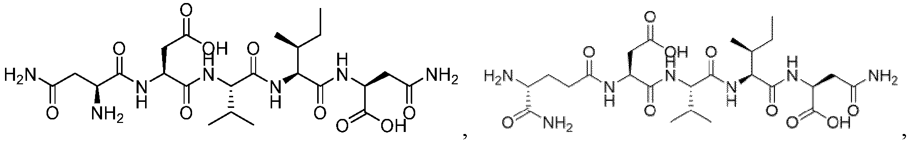

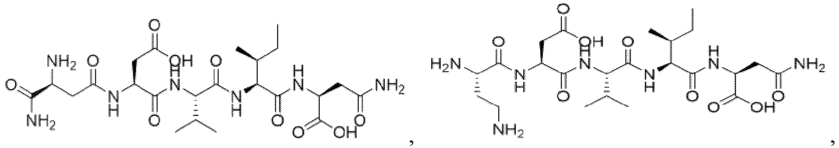

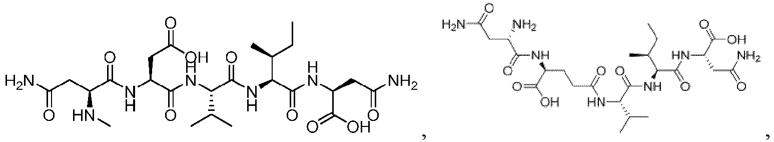

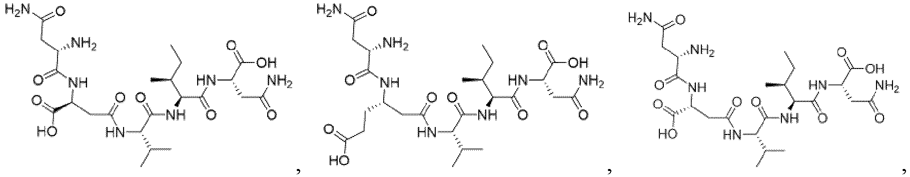

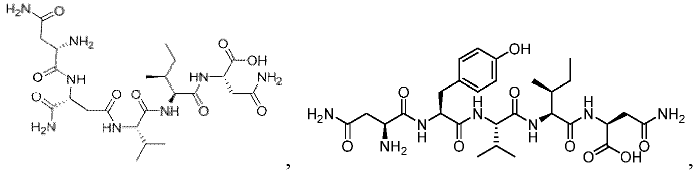

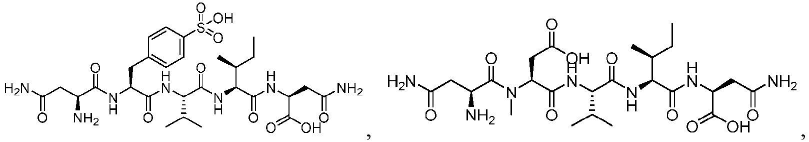

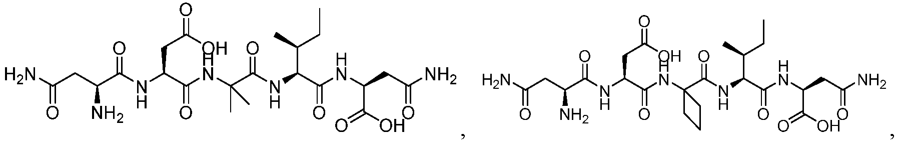

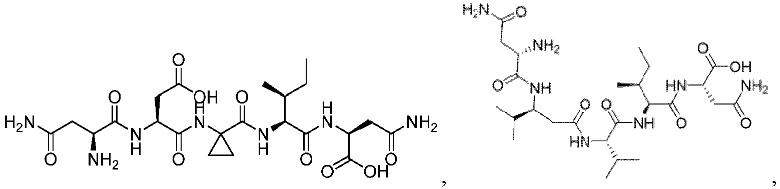

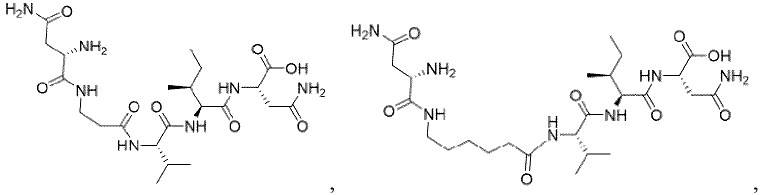

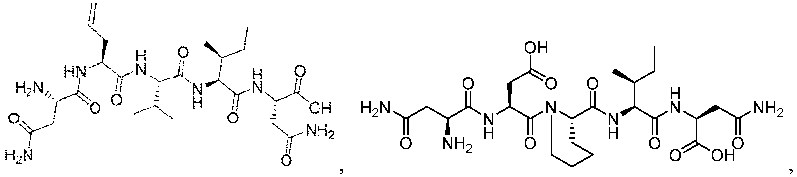

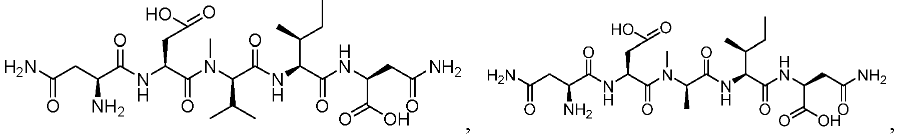

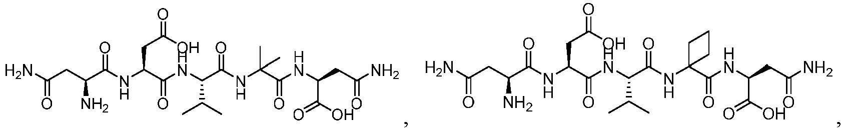

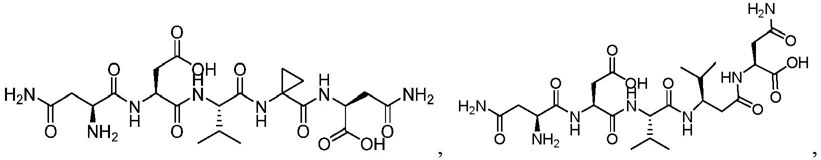

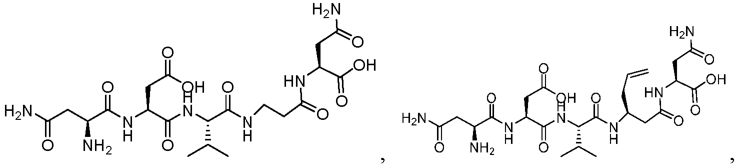

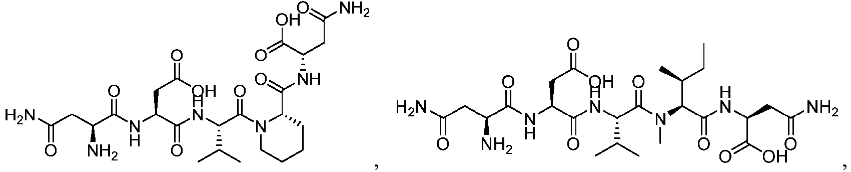

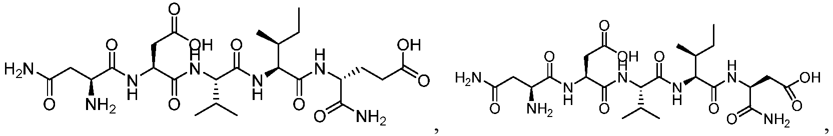

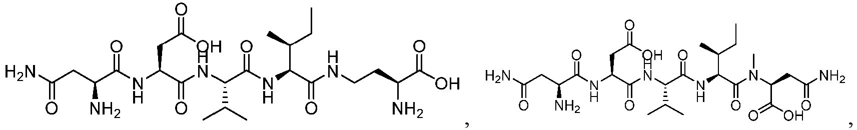

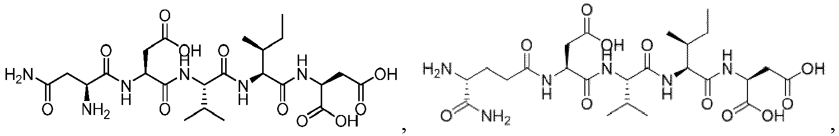

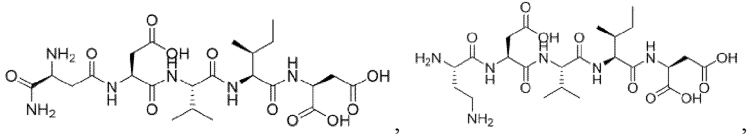

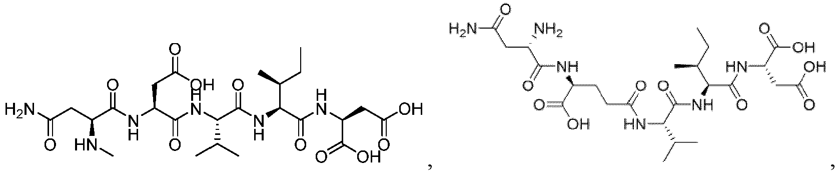

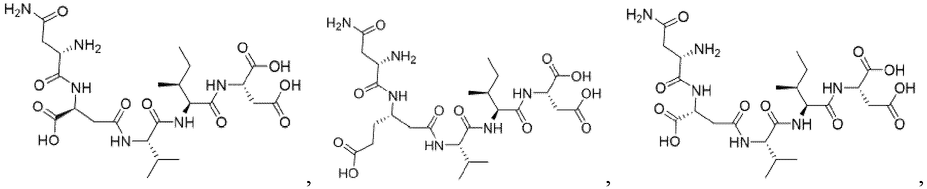

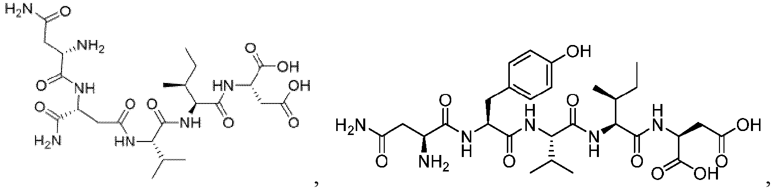

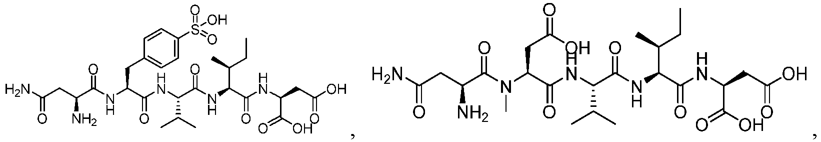

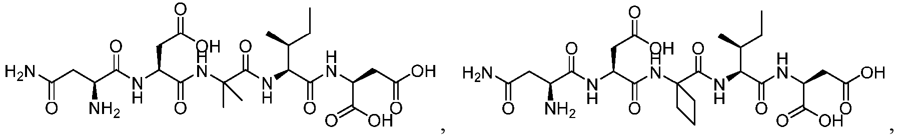

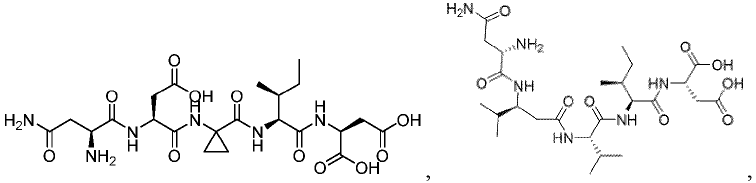

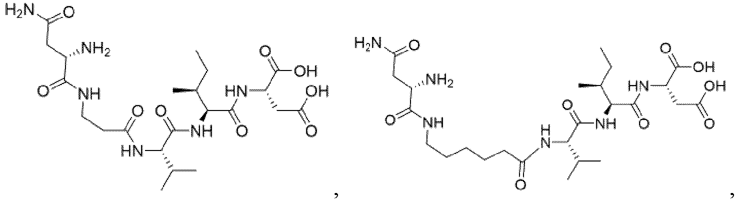

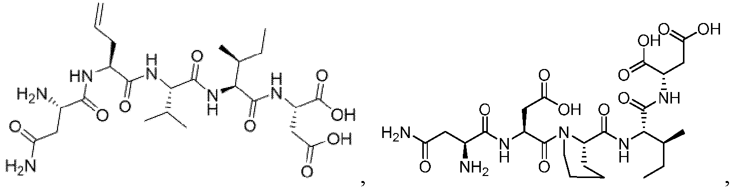

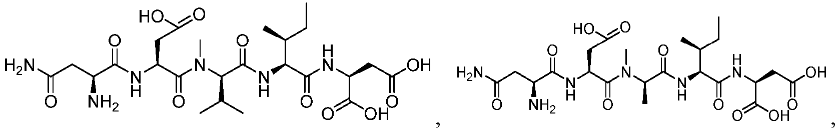

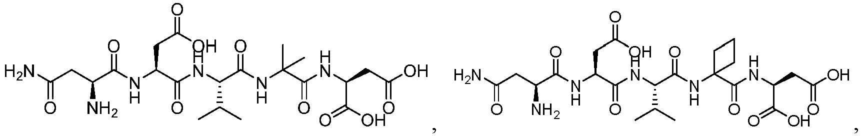

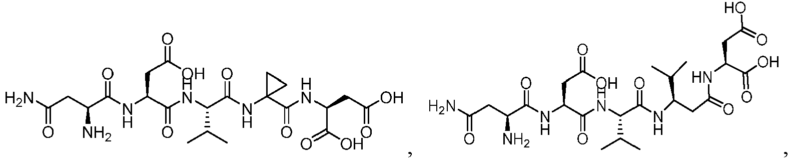

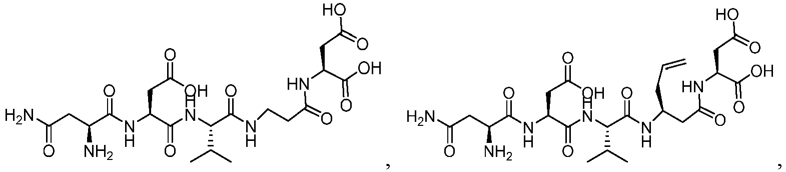

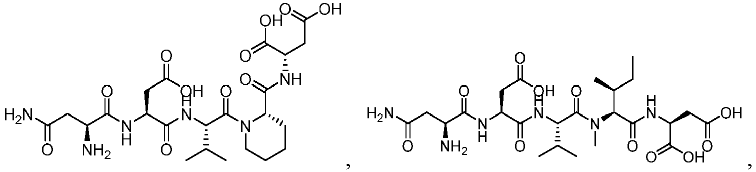

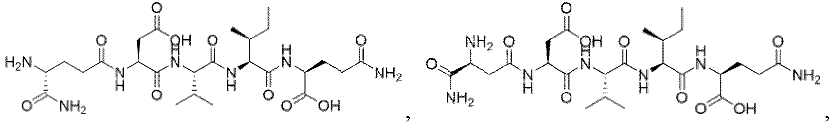

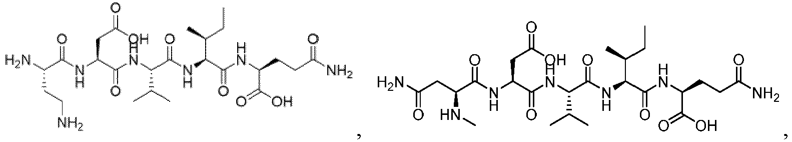

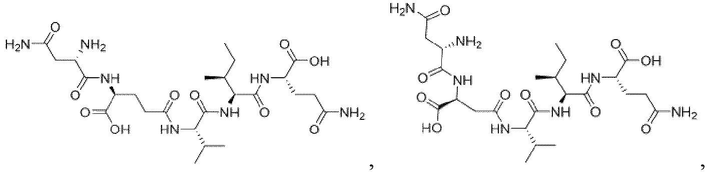

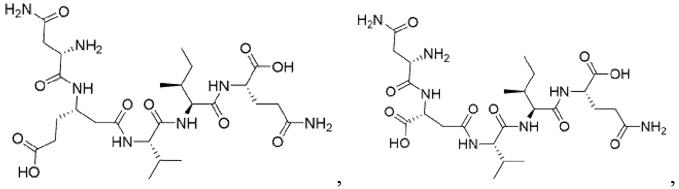

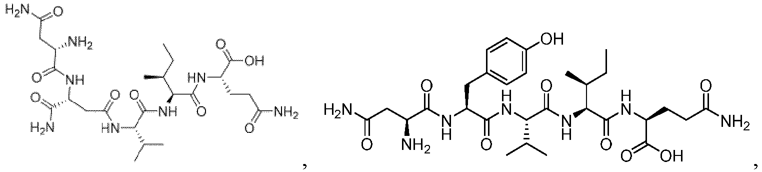

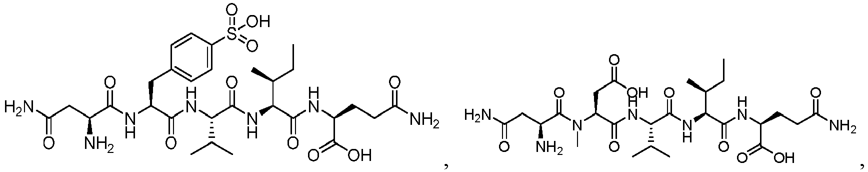

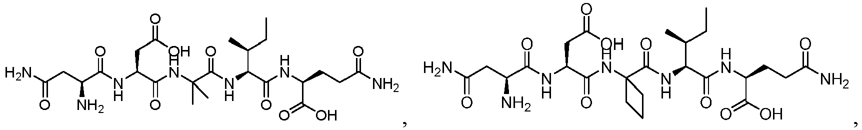

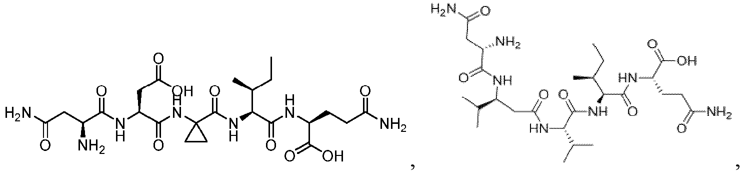

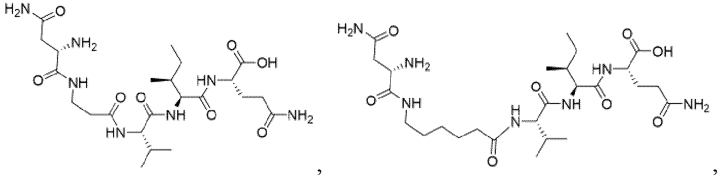

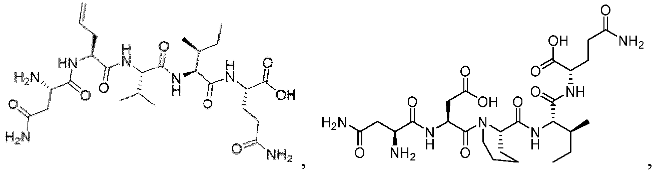

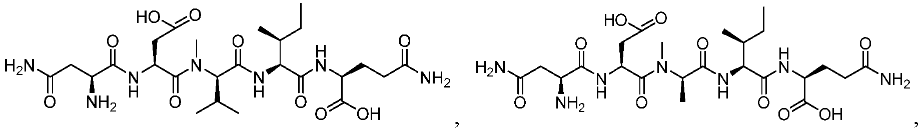

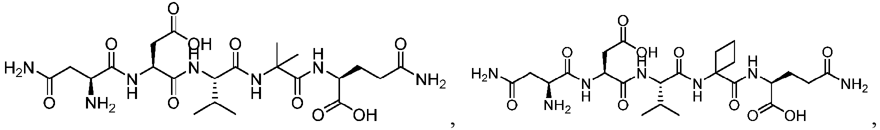

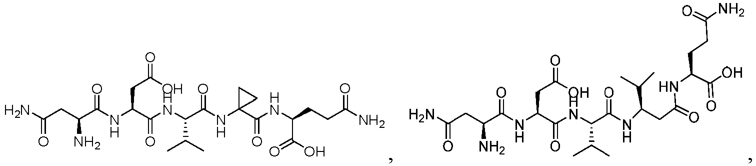

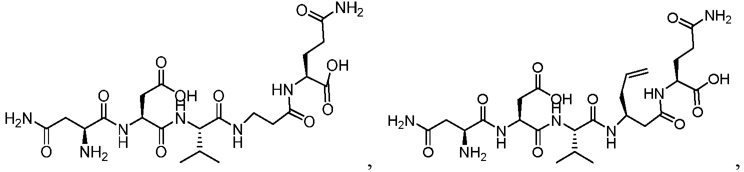

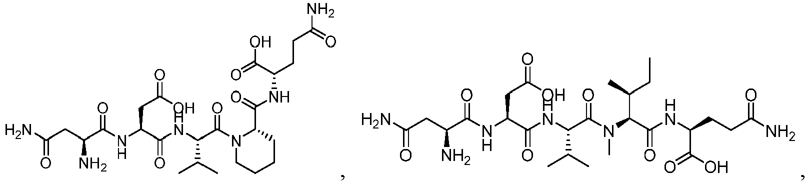

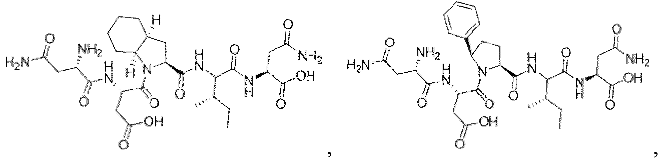

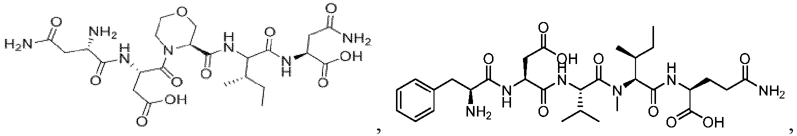

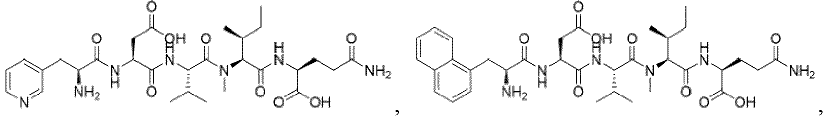

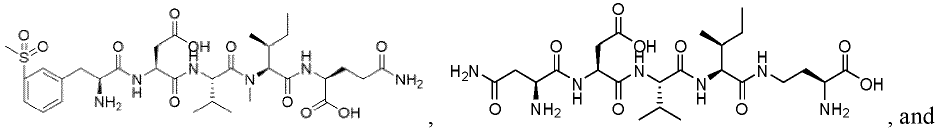

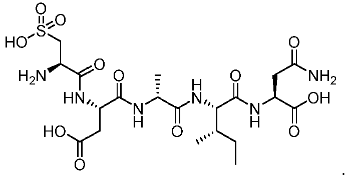

Preferably, the polypeptide-like compound has a structure selected from the group consisting of: 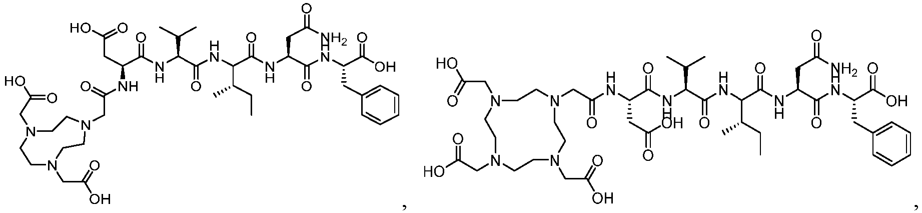 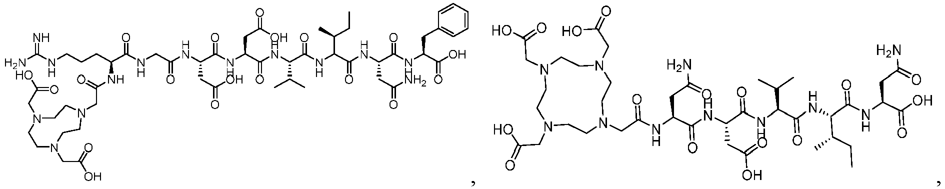 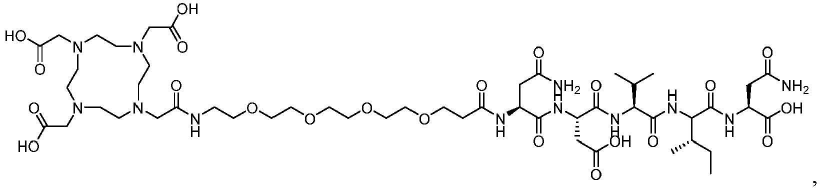 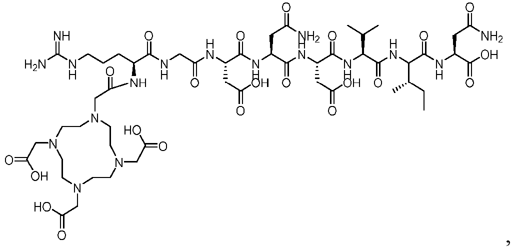 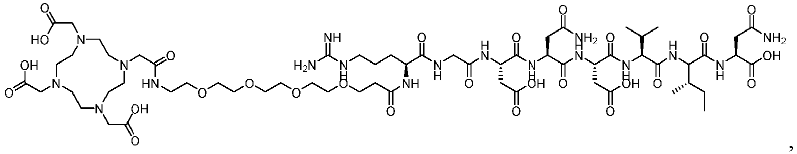 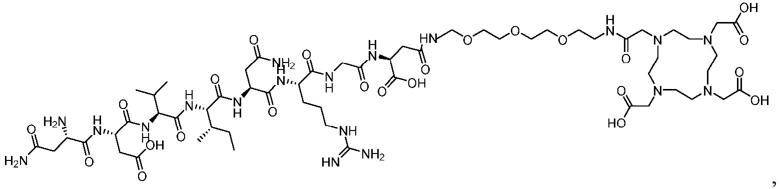 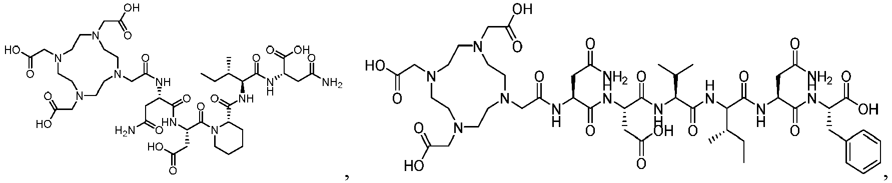 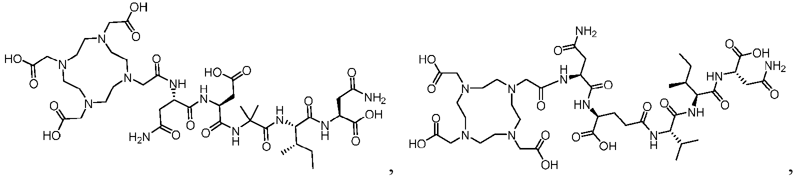 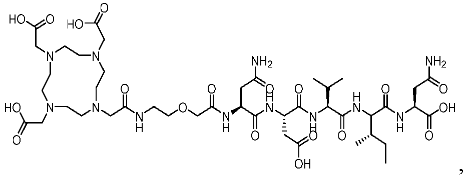 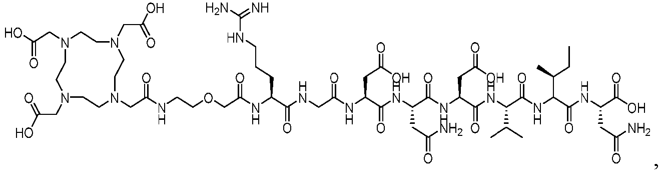 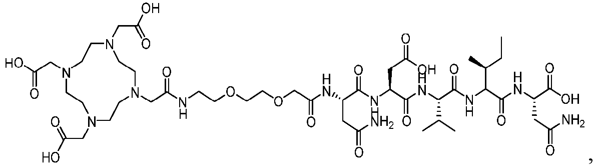 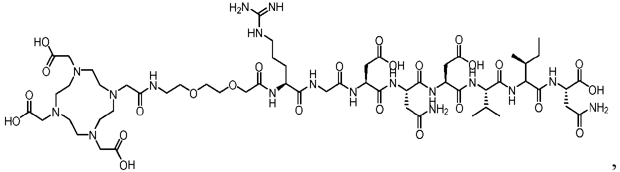 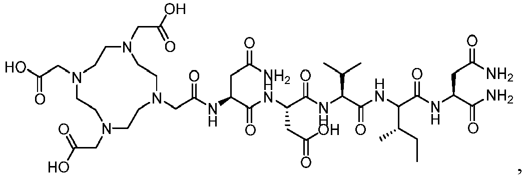 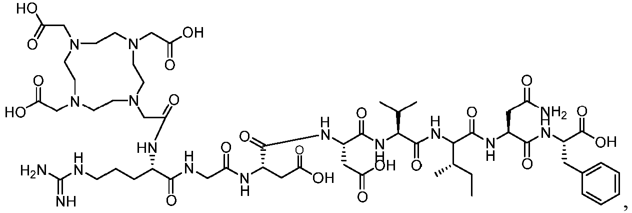 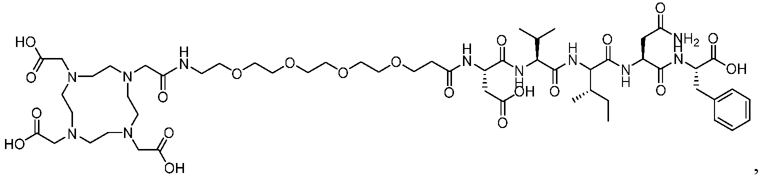 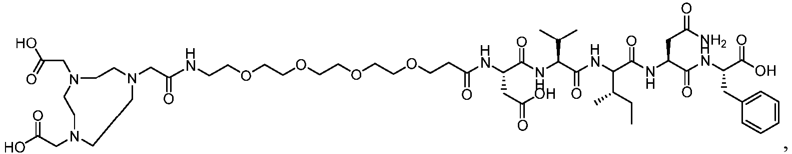 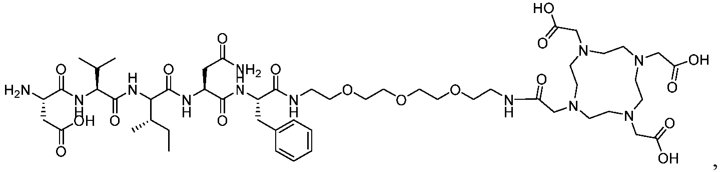 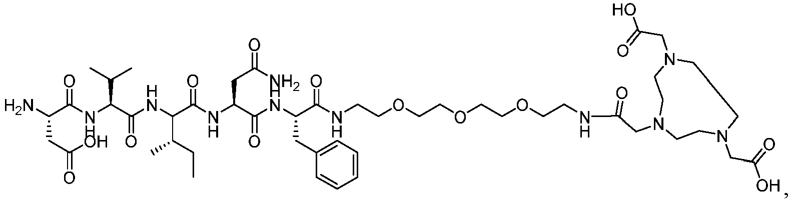 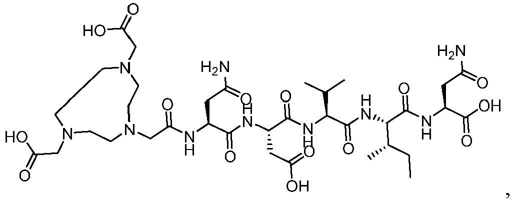 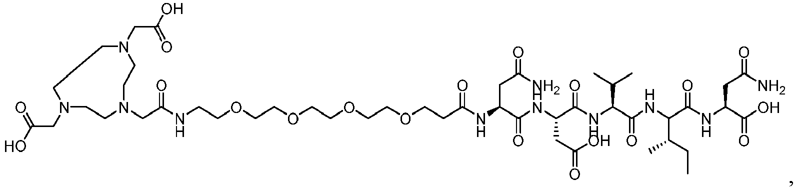 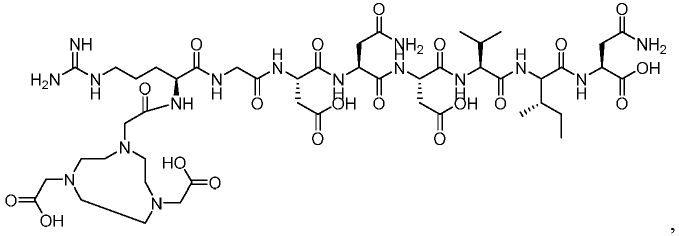 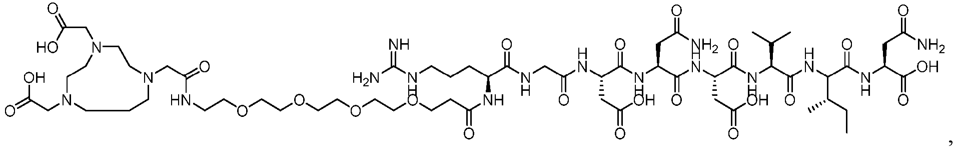 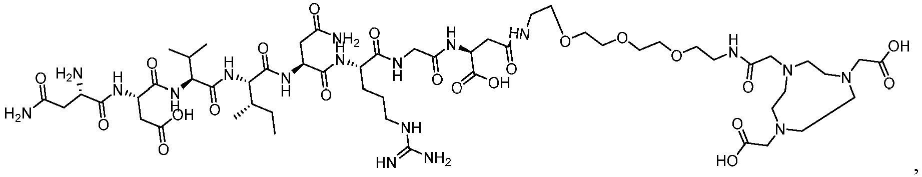 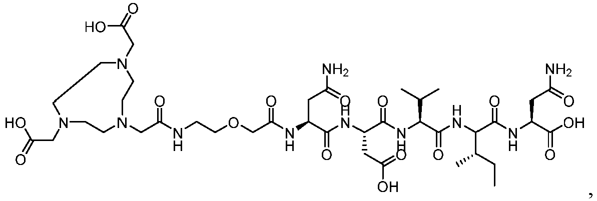 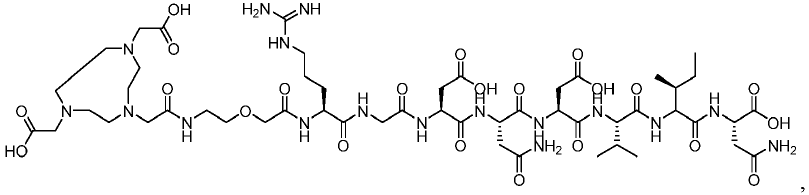 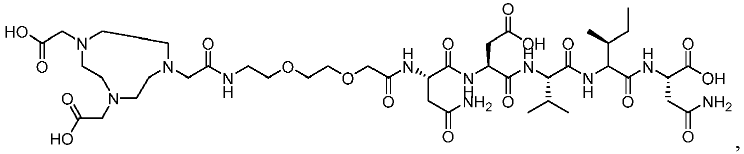 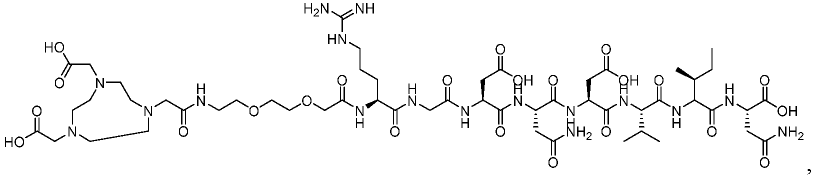 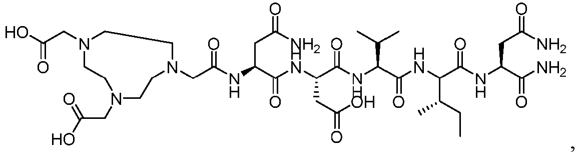 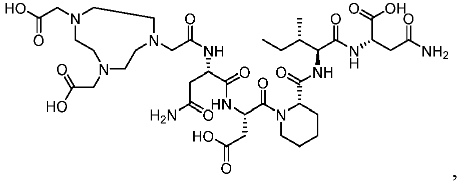 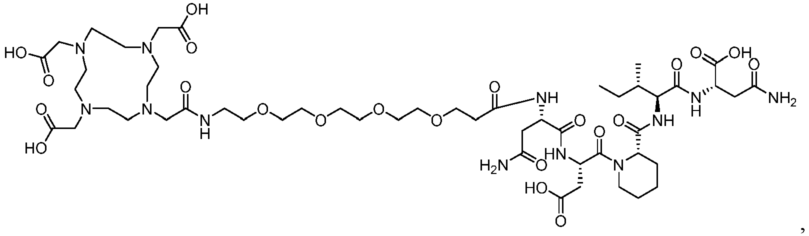 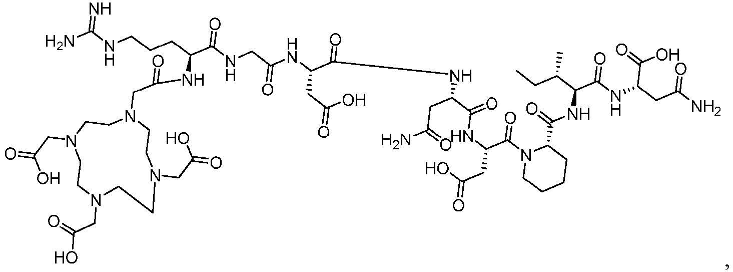 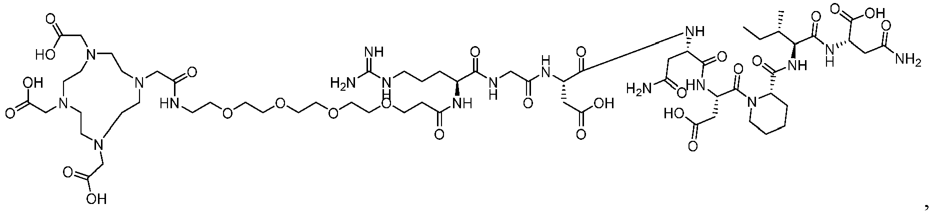 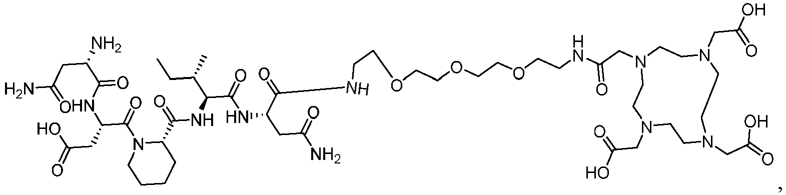 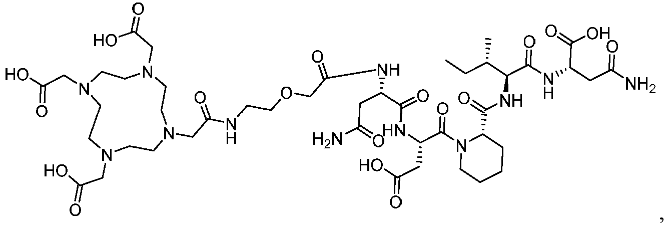 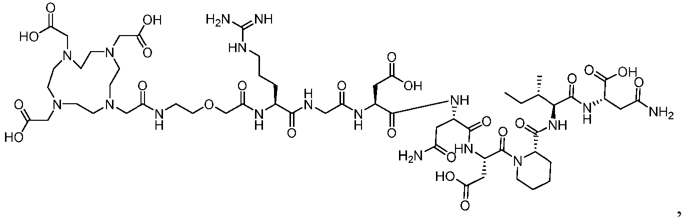 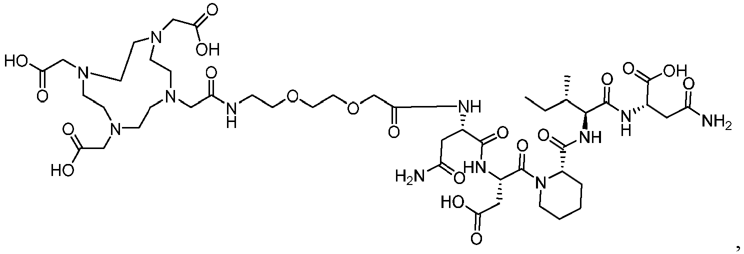 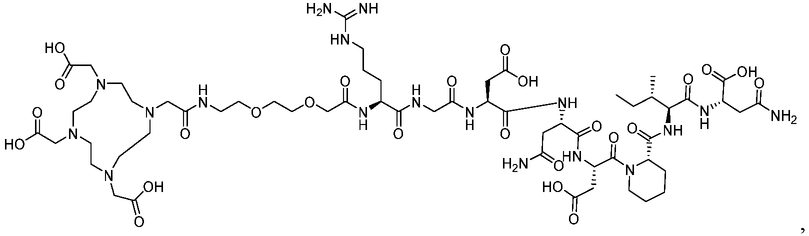 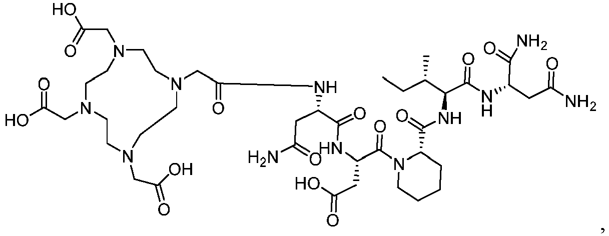 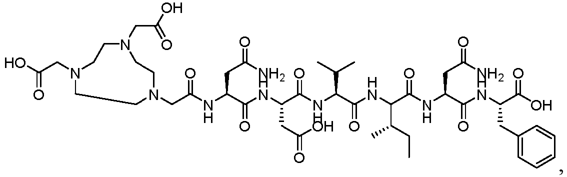 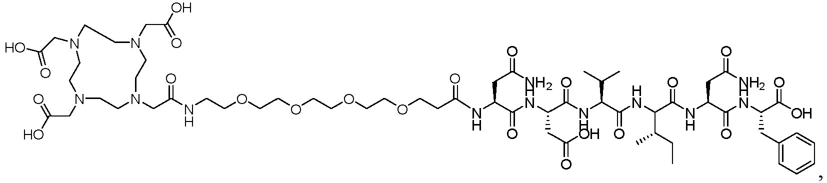 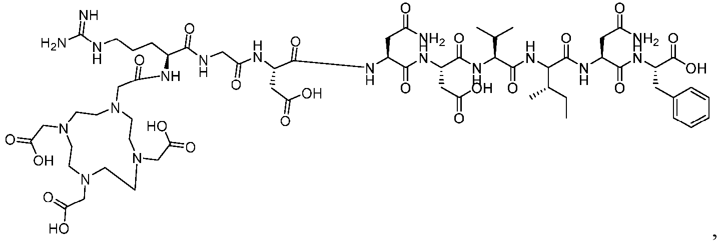 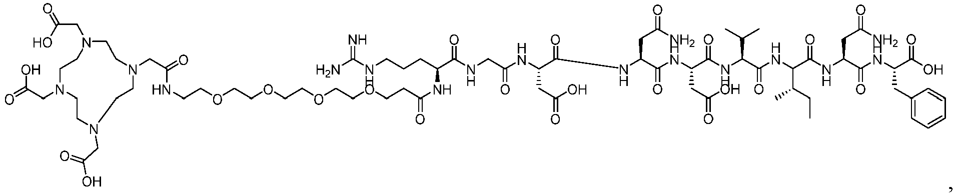 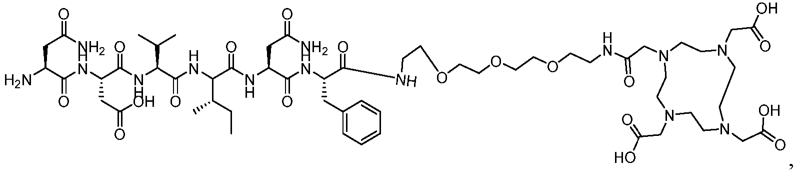 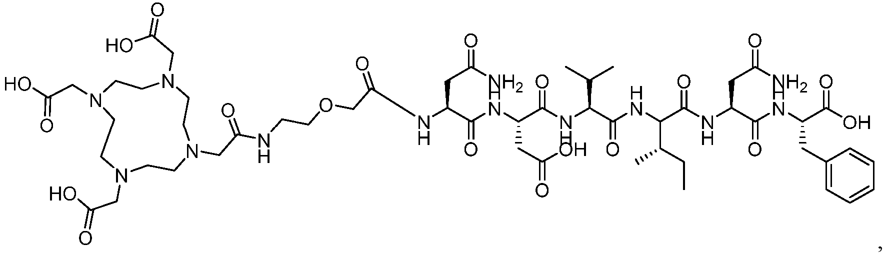 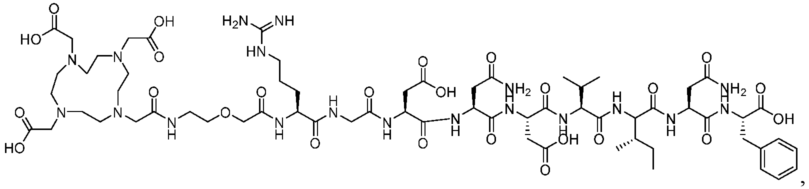 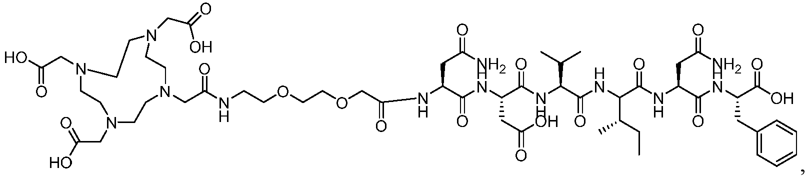 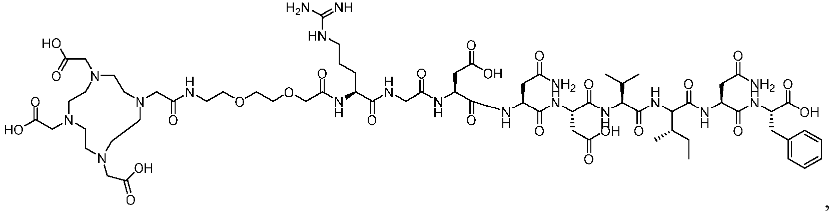 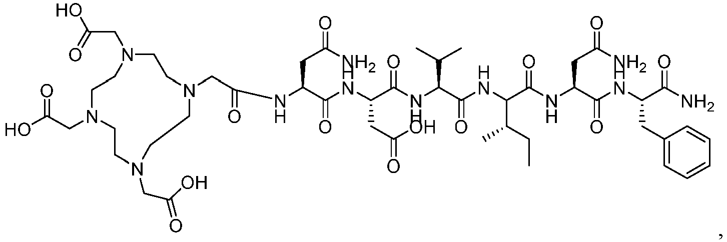 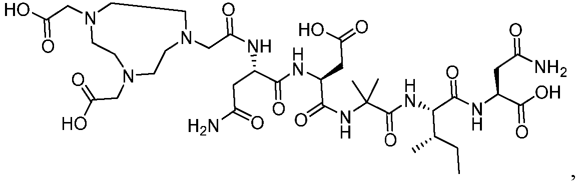 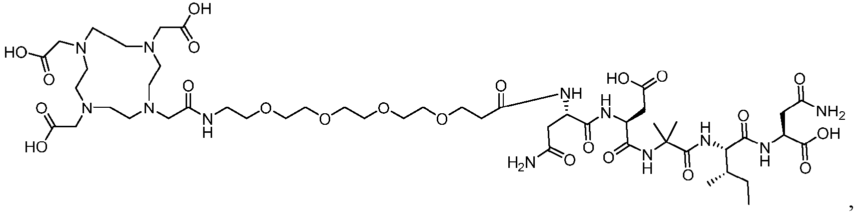 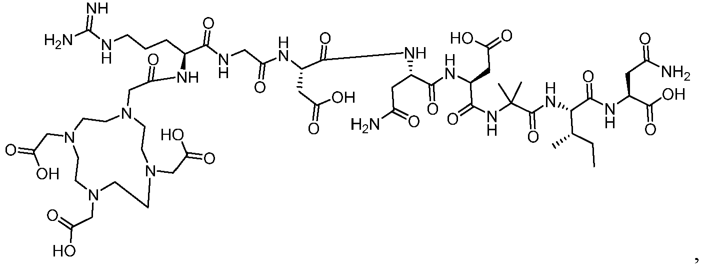 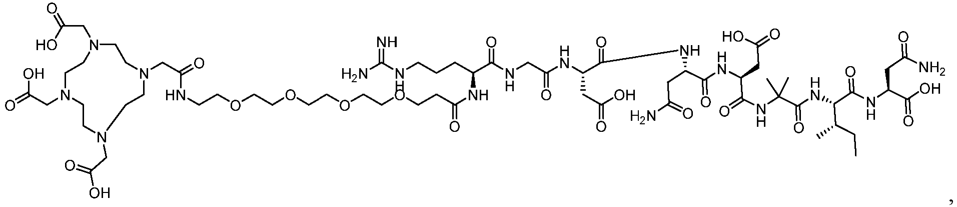 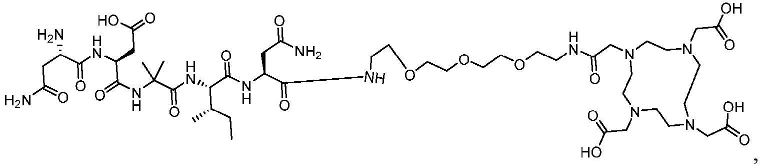 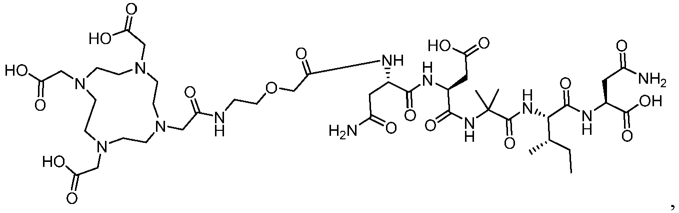 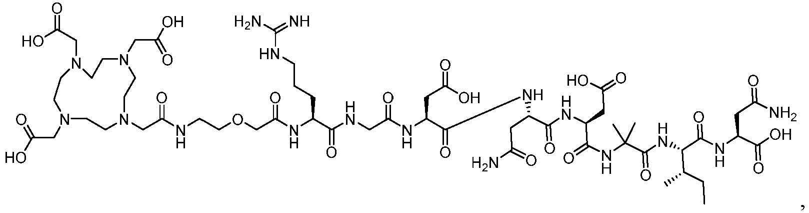 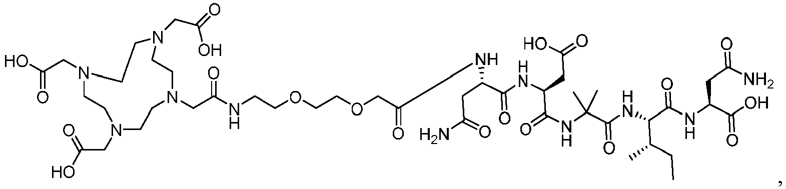 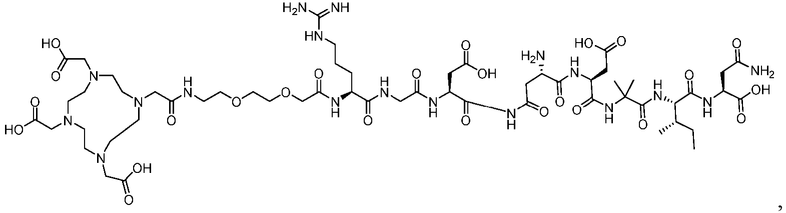 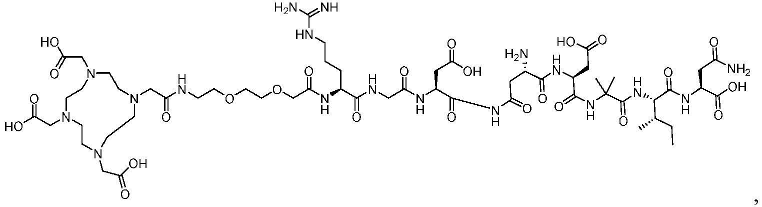 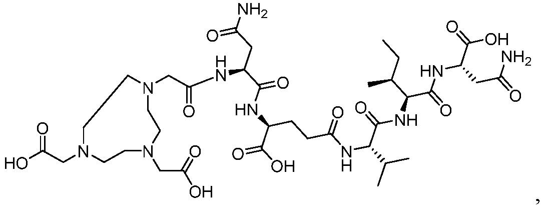 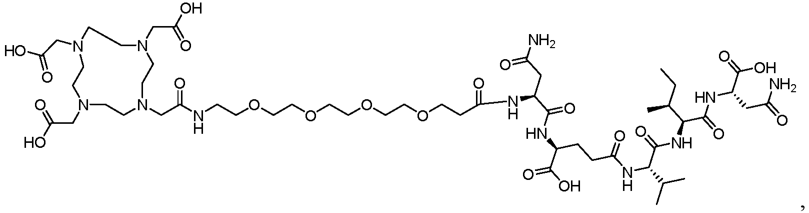 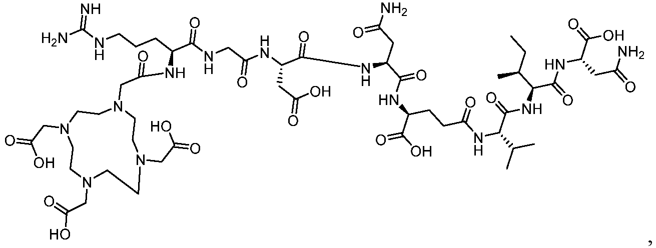 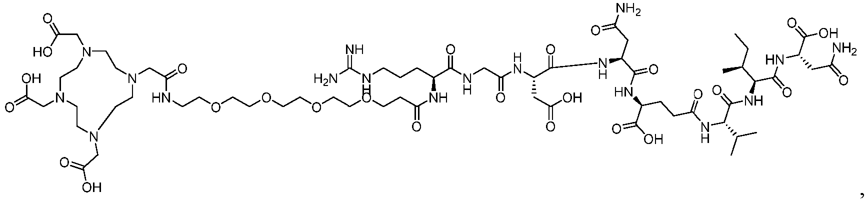 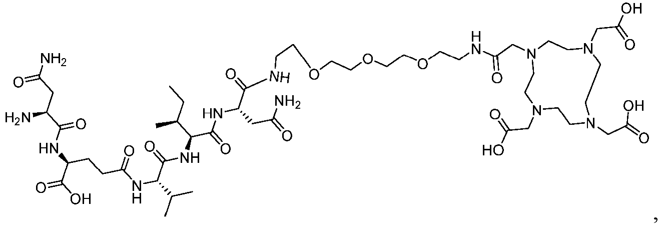 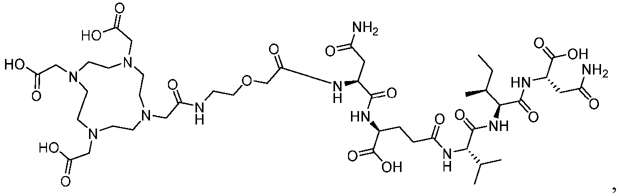 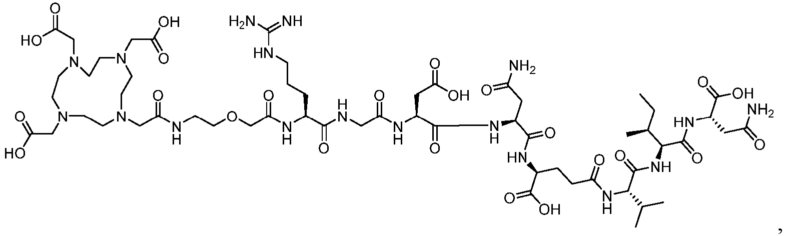 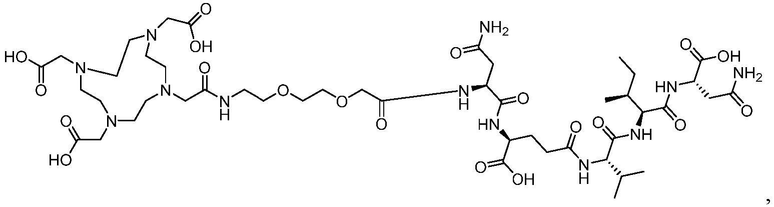 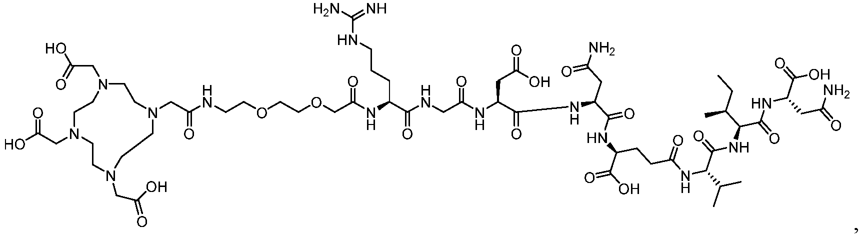 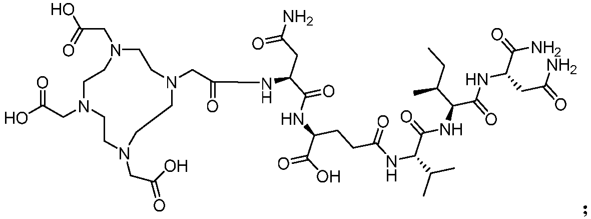 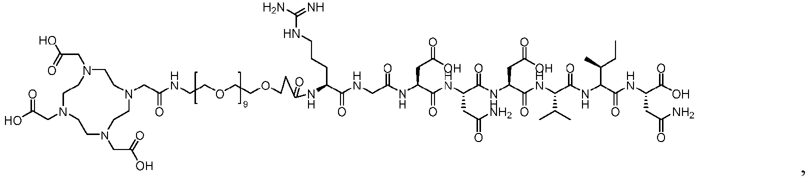 and

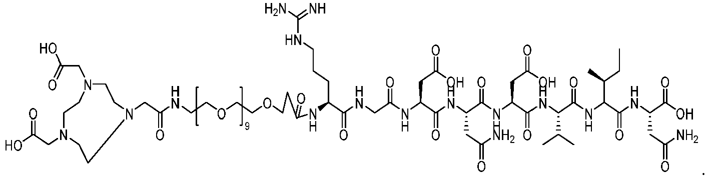

In the present disclosure, the term "halogen" refers to fluorine (F), chlorine (Cl), bromine (Br) and iodine (I).

In the present disclosure, the term "alkyl" refers to a linear or branched aliphatic hydrocarbyl, for example, "C1-C6 alkyl" refers to an alkyl with 1 to 6 carbon atoms. In the present disclosure, the alkyl includes, but is not limited to methyl, ethyl, n-propyl, isopropyl, n-butyl, sec-butyl, iso-butyl, tert-butyl, n-pentyl, and n-hexyl. The alkyl in the present disclosure may be optionally substituted by one or more substituents (e.g., halogen) as described herein.

In the present disclosure, the term "halogenated alkyl" refers to an alkyl substituted by one or more (e.g., 1 to 3) identical or different halogen atoms. For example, the term "C1-C6 halogenated alkyl" refers to a halogenated alkyl with 1 to 6 carbon atoms. In the present disclosure, the halogenated alkyl includes, but is not limited to -CH₂F, -CHF₂, -CF₃, -CH₂CF₃, -CF₂CF₃, - CH₂CH₂CF₃, and -CH₂Cl. The halogenated alkyl of the present disclosure can be optionally substituted by one or more substituents described herein.

In the present disclosure, the term "alkoxy" refers to an alkyl attached to the rest of the molecule through an oxygen atom. In the present disclosure, alkoxy includes, but is not limited to, methoxy, ethoxy, n-propoxy, isopropoxy, n-butoxy, isobutoxy, tert-butoxy, pentyloxy, and hexoxy. Alkoxy in the present disclosure can be optionally substituted by one or more of the substituents described herein.

In the present disclosure, the term "cycloalkyl" refers to a saturated or partially saturated, monocyclic or polycyclic (such as bicyclic) non-aromatic hydrocarbyl. For example, the term "C3-C8 cycloalkyl" refers to a cycloalkyl having 3 to 8 carbon atoms. In the present disclosure, cycloalkyl includes (but is not limited to) monocyclic cycloalkyl, such as cyclopropyl, cyclobutyl, cyclopentyl, cyclohexyl, cycloheptyl, cyclooctyl, and cyclononyl; or bicycloalkyl, including fused ring, bridged ring or spiro ring, such as bicyclo[1.1.1]pentyl, bicyclo[2.2.1]heptyl, bicyclo[3.2.1]octyl, bicyclo[5.2.0]nonyl, and decalin. The cycloalkyl in the present disclosure can be optionally substituted by one or more substituents described herein (such as methyl).

In the present disclosure, the term "heterocycloalkyl" refers to a saturated or partially saturated, monocyclic or polycyclic (such as bicyclic) nonaromatic group, the ring atoms of which are composed of carbon atoms and at least one heteroatom selected from N, O and S. The heterocycloalkyl may be attached to the rest of the molecule through any ring atom, provided the valence requirements are met. For example, the term "3-8 membered heterocycloalkyl" refers to a heterocycloalkyl with 3 to 8 ring atoms. In the present disclosure, the heterocycloalkyl includes (but is not limited to), oxiranyl, aziridinyl, azetidinyl, oxetanyl, tetrahydrofuryl, dioxolinyl, pyrrolidinyl, pyrrolidinonyl, imidazolidinyl, pyrazolidinyl, tetrahydropyranyl, piperidinyl, piperazinyl, morpholinyl, thiomorpholinyl, dithianyl, and trithianyl. The heterocycloalkyl in the present disclosure can be optionally substituted by one or more substituents described herein.

In the present disclosure, the term "aryl" refers to a monocyclic or fused polycyclic aromatic hydrocarbyl having a conjugated π electron system. For example, the term "C6-C20 aryl" refers to an aryl with 6 to 20 carbon atoms. In the present disclosure, the aryl includes (but is not limited to) phenyl, naphthyl, anthracenyl, phenanthryl, acenaphthenyl, azulenyl, fluorenyl, indenyl, and pyrenyl. The aryl in the present disclosure can be optionally substituted by one or more substituents described herein (such as halogen, hydroxyl, cyano, nitro, and C1-C6 alkyl).

In the present disclosure, the term "heteroaryl" refers to an aromatic group having a single ring or a fused polycyclic ring (especially a benzofused polycyclic ring) with a conjugated π electron system, and its ring atoms are composed of carbon atoms and at least one heteroatom selected from N, O and S. The heterocycloalkyl may be attached to the rest of the molecule through any ring atom, provided the valence requirements are met. For example, the term "C6-C20 heteroaryl" refers to a heteroaryl with 6 to 20 carbon atoms. In the present disclosure, the heteroaryl includes (but is not limited to), thienyl, furyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidinyl, pyrazinyl, triazinyl and benzo derivative thereof. The heteroaryl in the present disclosure can be optionally substituted by one or more substituents described herein (such as halogen and C1-C6 alkyl).

In the present disclosure, the term "alkenyl" refers to a straight or branched aliphatic hydrocarbyl group having at least one carbon-carbon double bond. For example, the term "C2-C6 alkenyl" refers to an alkenyl with 2 to 6 carbon atoms. In the present disclosure, the alkenyl includes (but is not limited to) vinyl, propenyl, n-butenyl, 3-methylbutyl-2-alkenyl, n-pentenyl, n-octenyl, and n-decenyl. The alkenyl in the present disclosure can be optionally substituted by one or more substituents described herein.

In the present disclosure, the term "alkynyl" refers to a straight or branched aliphatic hydrocarbyl group having at least one carbon-carbon triple bond. For example, the term "C2-C6 alkynyl" refers to an alkynyl with 2 to 6 carbon atoms. In the present disclosure, the alkynyl includes (but is not limited to) ethynyl, 2-propynyl, 2-butynyl, and 1,3-butadiynyl. The alkynyl in the present disclosure can be optionally substituted by one or more substituents described herein.

In the present disclosure, the term " substituted" means that one or more (e.g., 1, 2, 3 or 4) atoms (e.g., hydrogen atoms) or radicals (e.g., trifluoromethanesulfonate groups) on the specified group are replaced by other atoms or radicals, provided that the specified group meets the valence requirements in the current situation and forms a stable compound after substitution. Combinations of substituents and/or variables are allowed only if they can form stable compounds. If a substituent is described as "optionally substituted by ... ", the substituent may be unsubstituted or substituted. If the first substituent is described as being optionally substituted by one or more substituents in the second substituent list, one or more hydrogen atoms in the first substituent can be individually or independently substituted by one or more substituents in the second substituent list, or not.

In the present disclosure, the term "alkylene" refers to a group obtained by removing two hydrogen atoms from straight, branched or cyclic, saturated or unsaturated hydrocarbons.

In the present disclosure, the term "arylidene" refers to a group obtained by removing two hydrogen atoms from aromatic hydrocarbons; and "heteroarylidene" refers to a group obtained by removing two hydrogen atoms from an aromatic compound containing heteroatoms.

In the present disclosure, the term "alkyleneoxy" refers to a group obtained by removing one hydrogen atom from an alkoxyl.

In the present disclosure, the term "alkylidene hypophosphorous acid group" refers to a group in which alkylidene and hypophosphorous acid group are connected by a covalent bond. The alkylidene refers to a group obtained by removing two hydrogen atoms from a straight-chain or branched-chain hydrocarbon compound, and the hypophosphorous acid group refers to a group obtained by removing a hydrogen atom connected to a phosphorus atom from hypophosphorous acid. The "alkylidene phosphorous acid group" refers to a group in which alkylidene and phosphorous acid group are connected by a covalent bond. The phosphorous acid group refers to a group obtained by removing a hydrogen atom connected to a phosphorus atom from phosphorous acid.

In the present disclosure, the term "alkylidene ether" refers to a group obtained by removing one hydrogen atom from alkyl ether.

In the present disclosure, the term "arylidene ether" refers to a group obtained by removing one hydrogen atom from aryl ether.

In the present disclosure, the term "alkylidene sulfonyl" refers to a group in which alkylidene and sulfonyl groups are linked by a covalent bond. The "alkylidene sulfonic acid group" refers to a group in which alkylidene and sulfonic acid group are linked by a covalent bond. The "alkylidene sulfonate group" refers to a group in which alkylidene and sulfonate group are linked by a covalent bond. The "alkylidene phosphate group" refers to a group in which alkylidene and phosphate group are linked by a covalent bond.

In the present disclosure, the term "each independently" means that at least two groups (or ring systems) in a structure with the same or similar value range may have the same or different meanings in a specific circumstance. For example, if substituent 1 and substituent 2 are each independently hydrogen, halogen, hydroxyl, cyano, alkyl or aryl, when substituent 1 is hydrogen, substituent 2 is either hydrogen, or halogen, hydroxyl, cyano, alkyl or aryl; similarly, when substituent 2 is hydrogen, substituent 1 is either hydrogen, or halogen, hydroxyl, cyano, alkyl or aryl.

In the present disclosure, the derivative is preferably a pharmaceutically acceptable form of the compound. For example, it is selected from the group consisting of salt, ester, stereoisomer, tautomer, polymorph, solvate, nitrogen oxide, isotope label, metabolite, prodrug of the compound, and a combination thereof.

In the present disclosure, the term "polymorph" (or "polymorphic form") refers to the solid crystal form of a compound or complex. Polymorphs of molecules can be obtained by a person skilled in the art through many known methods. These methods include (but are not limited to) melt recrystallization, melt cooling, solvent recrystallization, desolvation, rapid evaporation, rapid cooling, slow cooling, vapor phase diffusion and sublimation.

In the present disclosure, the term "solvate" refers to a substance formed by combination of a compound of the present disclosure (or a pharmaceutically acceptable salt thereof) with at least one solvent molecule through a non-covalent intermolecular force. Common solvates include (but are not limited to) hydrates (including hemihydrate, monohydrate, dihydrate, trihydrate, etc.), alcoholates, and acetonates.

In the present disclosure, the term "nitrogen oxide" refers to a compound formed by oxidation of nitrogen atoms in the structure of tertiary amines or nitrogen-containing (aromatic) heterocyclic compounds. For example, nitrogen atoms in the parent nucleus structure can form corresponding nitrogen oxides.

In the present disclosure, the term "isotope label" refers to a derivative compound formed by replacing a specific atom in the compound of the present disclosure with its isotope atom. Unless otherwise specified, the compound of the present disclosure includes various isotopes of H, C, N, O, F, P, S and Cl, such as ²H(D), ³H(T), ¹³C, ¹⁴C, ¹⁵N, ¹⁷O, ¹⁸O, ¹⁸F, ³¹P, ³²P, ³⁵S, ³⁶S and ³⁷Cl.

In the present disclosure, the term "metabolite" refers to a derivative compound formed after metabolism of the compound of the present disclosure.

The term "prodrug" means a derivative compound capable of directly or indirectly providing a compound according to the application upon administration to an individual. Particularly, preferred derivative compounds or prodrugs are compounds that, when administered to a subject, increase the bioavailability of the compounds of the present application (e.g., better absorption into the blood), or facilitate delivery of the parent compound to the site of action (e.g., the lymphatic system). Unless otherwise indicated, all prodrug forms of the compounds according to the present application are within the scope of the present application, and various prodrug forms are known in the art.

The term "a pharmaceutically acceptable salt" refers to a salt of the compound of the present disclosure that is substantially non-toxic to an organism. Pharmaceutically acceptable salts generally include (but are not limited to) salts formed by reacting the compound of the present disclosure with a pharmaceutically acceptable inorganic acid/organic acid, or inorganic base/organic base, such salts are also known as acid addition salts or base addition salts.

The term "a pharmaceutically acceptable ester" refers to an ester that is substantially non-toxic to an organism and hydrolyzed into the compound of the present application or a salt thereof in the organism. In the present disclosure, pharmaceutically acceptable esters generally include (but are not limited to) esters formed by the compound of the present disclosure with pharmaceutically acceptable carboxylic or sulfonic acids, such esters also known as carboxylic or sulfonic esters.

The term "isomer" refers to a compound that has the same molecular weight due to the same number and type of atoms, but differs in the spatial arrangement or configuration of the atoms. The term "stereoisomer" (or "optical isomer") refers to a stable isomer having a vertical asymmetric plane resulted by at least one chiral factor (including chiral center, chiral axis, chiral plane, etc.) and thereby being capable of rotating plane-polarized light. Since the compounds of the present disclosure have asymmetric centers and other chemical structures that may lead to stereoisomerism, the present disclosure also includes these stereoisomers and mixtures thereof. Since the compounds of the present disclosure (or pharmaceutically acceptable salts thereof) comprise asymmetric carbon atoms, they may exist in the form of a single stereoisomer, a racemate, a mixture of enantiomers and diastereomers. Generally, these compounds can be prepared in the form of racemates. However, if desired, such compounds can be prepared or isolated as a pure stereoisomer, i.e. single enantiomer or diastereomer, or a mixture enriched with a single stereoisomer (purity≥98%, ≥95%, ≥93%, ≥90%, ≥88%, ≥85% or ≥80%). A single stereoisomer of the compound is prepared synthetically from optically active starting materials containing the desired chiral center, or obtained by separation or resolution after the mixture of enantiomeric products is prepared, for example, after a mixture of diastereomers is obtained by conversion, it is subjected to separation or recrystallization, chromatography treatment, treatment by chiral resolving agent, or direct separation of enantiomers on chiral chromatography column. The starting compound with specific stereochemistry is either commercially available or can be prepared and resolved by methods well known in the art. The term "enantiomers" refers to a pair of stereoisomers with mirror images that are not superimposable between each other. The term "diastereoisomers" or "diastereomers" refers to optical isomers that do not form mirror images between each other. The term "racemic mixture" or "racemate" refers to a mixture containing equal parts of single enantiomers (i.e., an equimolar mixture of the two R and S enantiomers). The term "non-racemic mixture" refers to a mixture containing unequal parts of single enantiomers. Unless otherwise indicated, all stereoisomeric forms of the compounds of the present disclosure are within the scope of the present disclosure. The term "tautomers" (or "tautomeric forms") refers to structural isomers with different energies that can be interconverted through a low energy barrier. A chemical equilibrium of tautomers can be achieved if tautomerism is possible (e.g., in solution). For example, proton tautomers (or proton transfer tautomers) include, but are not limited to, interconversion by proton transfer, such as keto-enol isomerization, imine-enamine isomerization, amido-imino alcohol isomerization, etc. Unless otherwise indicated, all tautomeric forms of the compounds of the present disclosure are within the scope of the present disclosure.

In the present disclosure, there is no special restrictions on the preparation method of the polypeptide-like compound, and it is sufficient to adopt the solid-phase slice-by-slice, fragment synthesis, and liquid-phase synthesis of polypeptide-like compound using resins by those skilled in the art, and to perform synthesizing using the desired amino acids, amino acid-like substances, or polypeptide-like substances according to the structure of the compound. The general steps in the preparation process of the polypeptide-like compound preferably comprise: initial resin synthesis, removal of protective groups (Fmoc protective groups), coupling amino acids, repeating the operation of removal of protective groups and coupling amino acids to gradually synthesize the desired polypeptide-like compound. Wherein, the PD, ZT and linker can be connected to each other via common functional group structures, such as amido, carbonate, urea, sulfonamido, sulfuamido, and amine, formed by reaction of functional groups in their structure.

The present disclosure further provides a polypeptide-like coordination compound formed by reacting the polypeptide-like compound and a metal; wherein the metal is selected from the group consisting of Cu, Ga, In, Bi, Gd, ¹⁷⁷Lu, ⁶⁴Cu, ⁶⁸Ga, ¹¹¹In , ²¹¹At, ²²⁵Ac, ⁹⁰Y, ²¹³Bi, At, and an isotope thereof, more preferably selected from the group consisting of ¹⁷⁷Lu, Gd, ⁶⁸Ga, and an isotope thereof.

Preferably, the polypeptide-like coordination compound has a structure selected from the group consisting of: 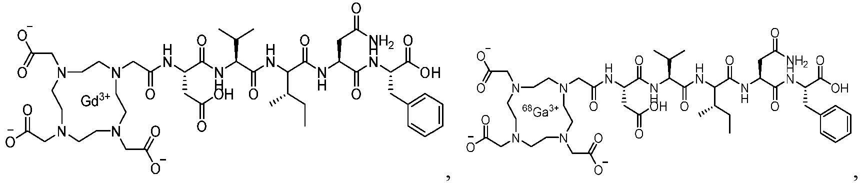 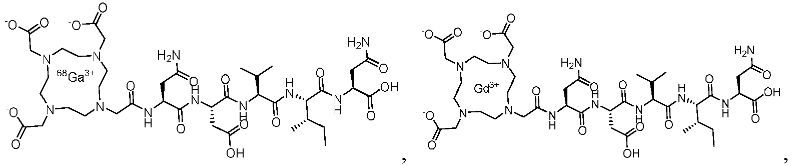 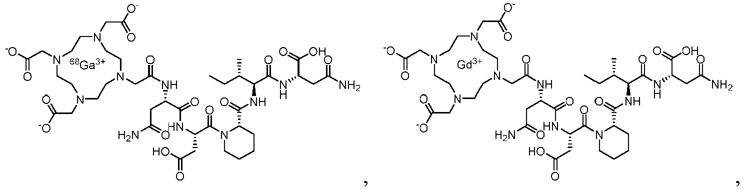 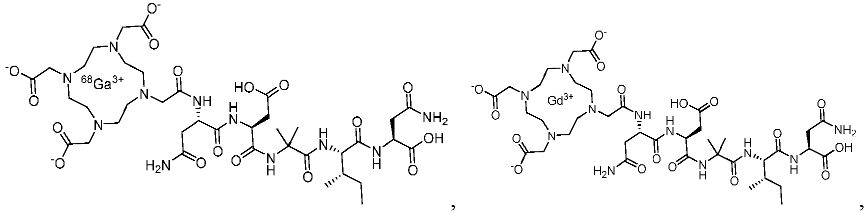 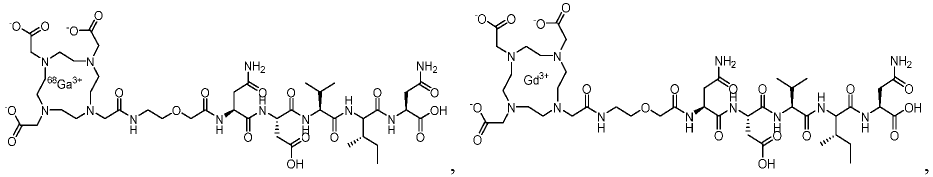 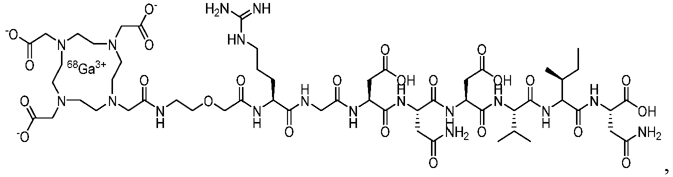 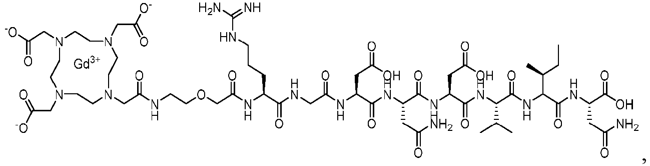 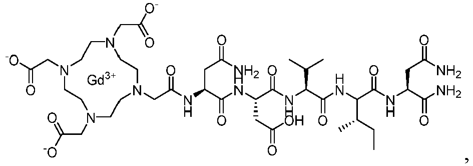 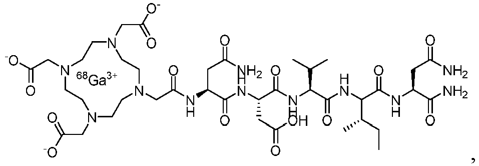 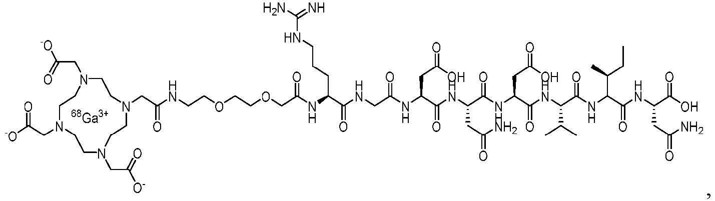 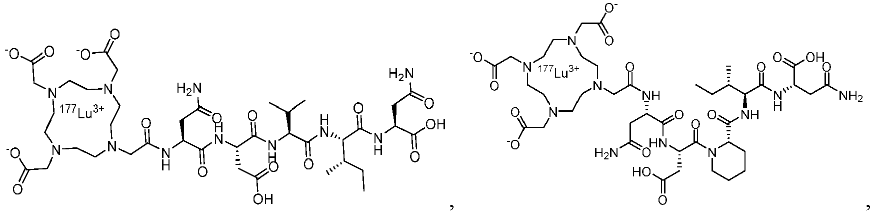 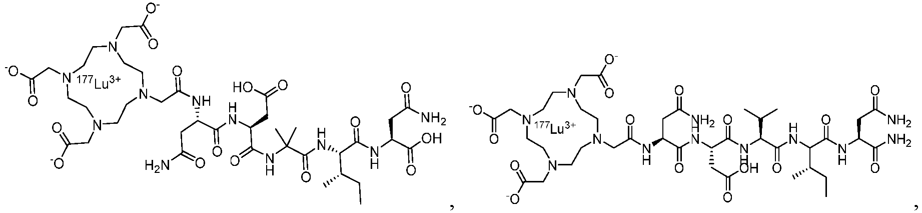 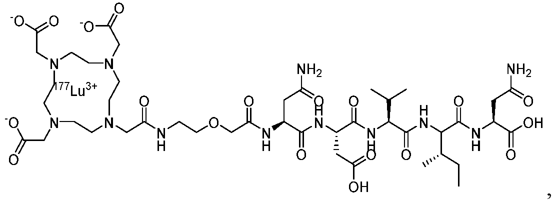 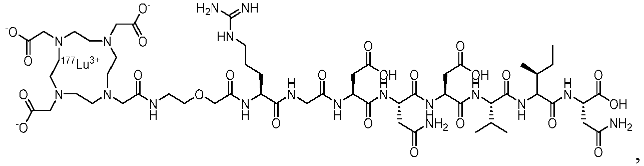 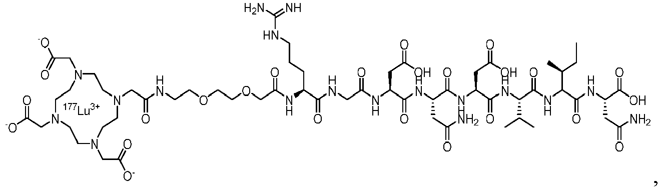 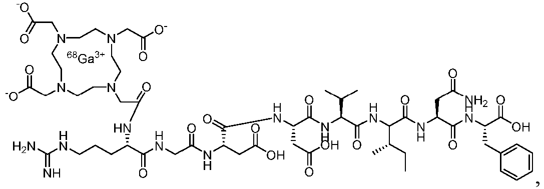 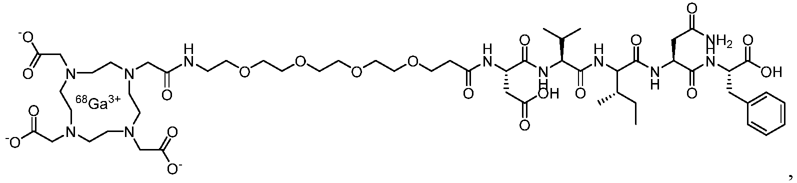 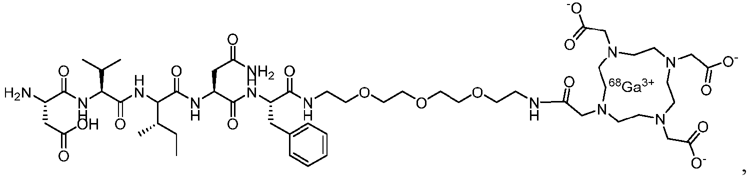 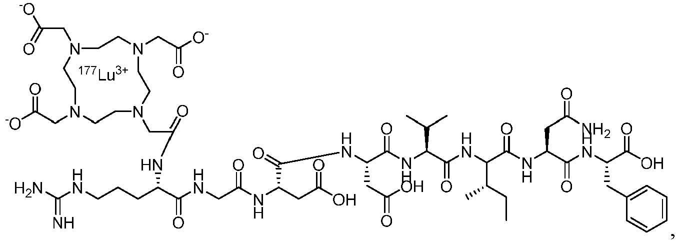 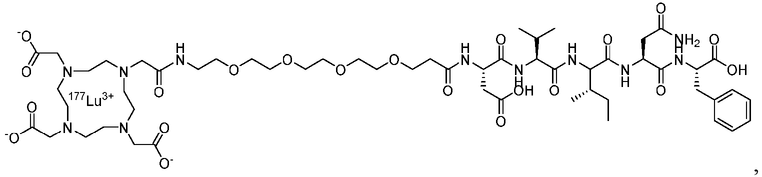 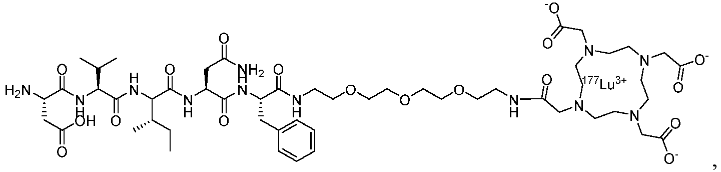 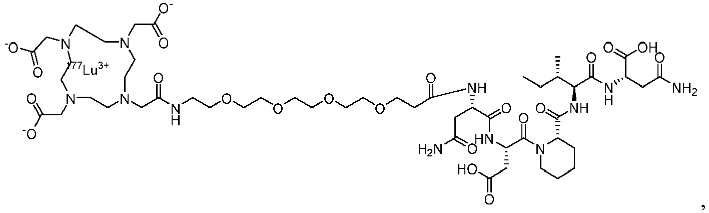 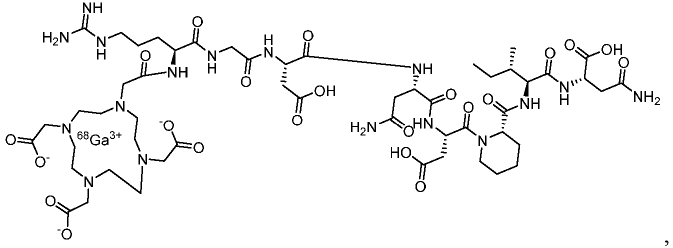 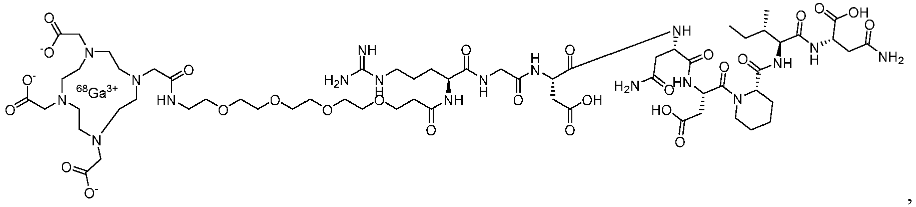 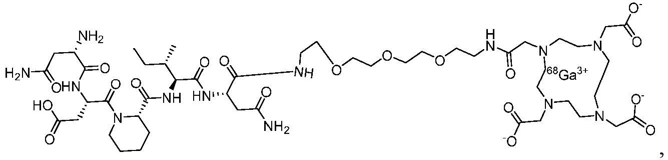 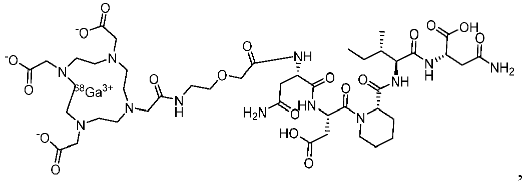 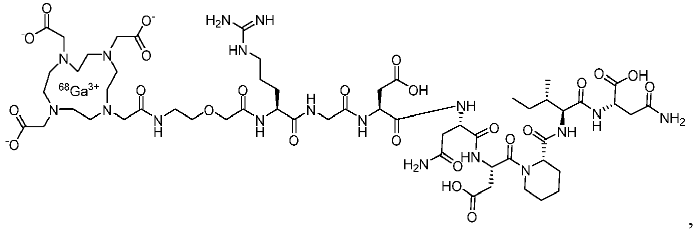 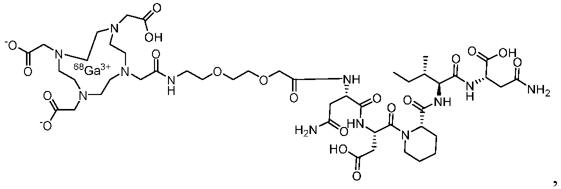 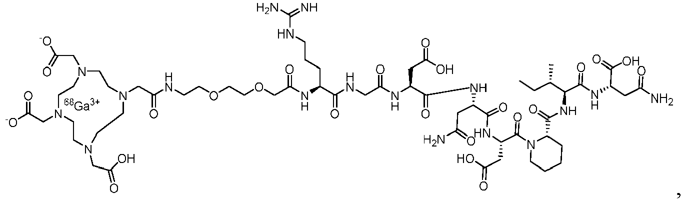 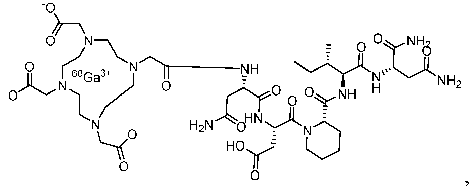 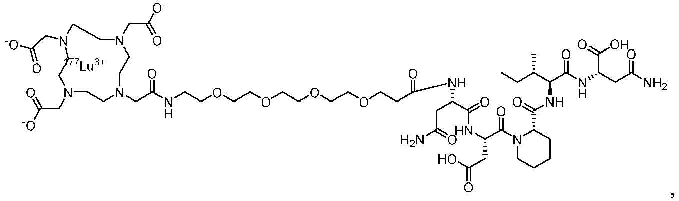 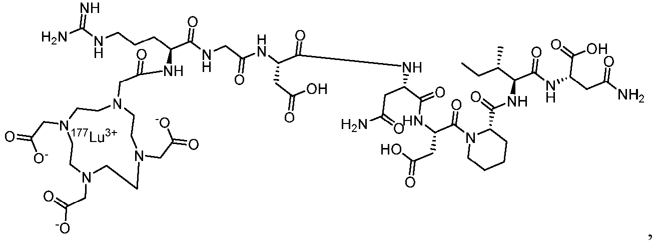 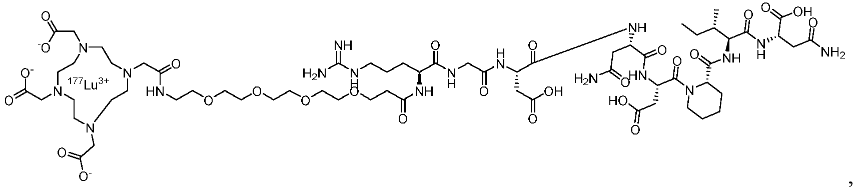 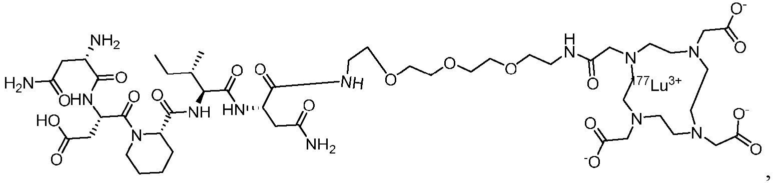 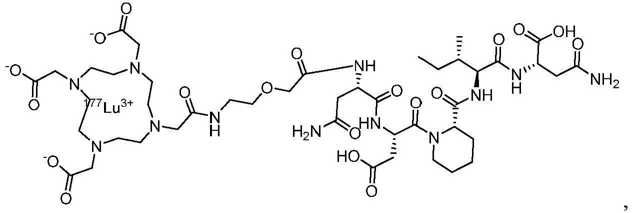 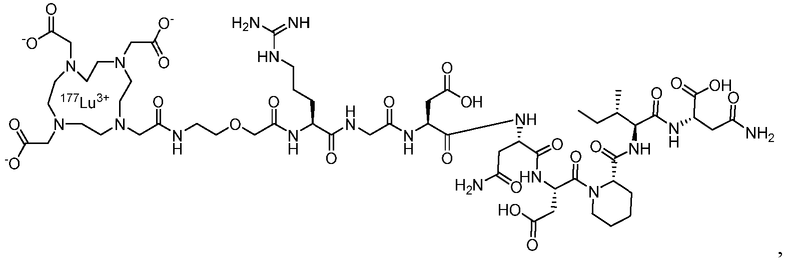 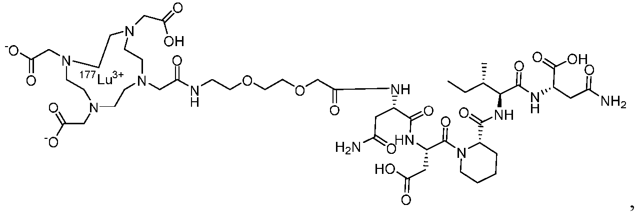 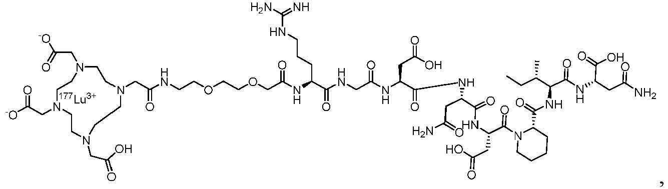 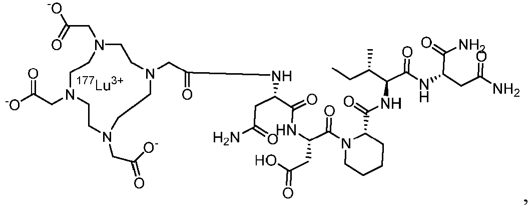 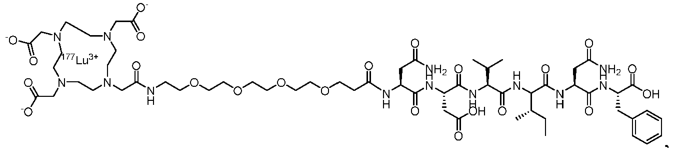 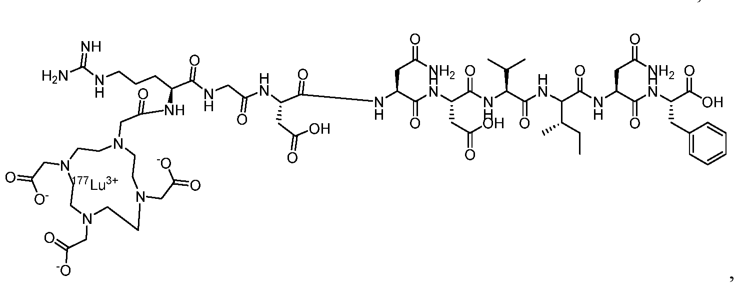 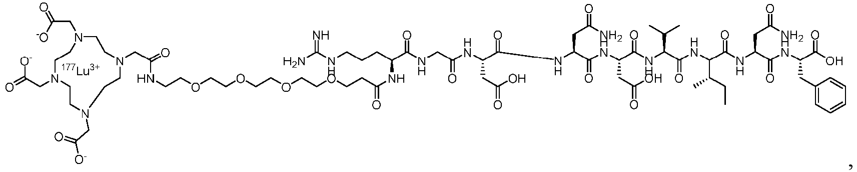 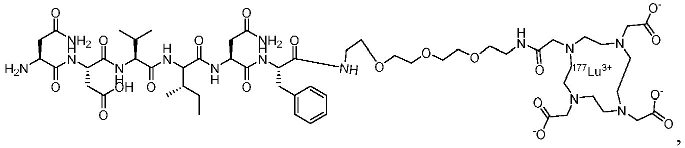 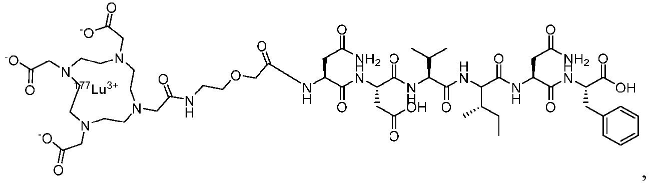 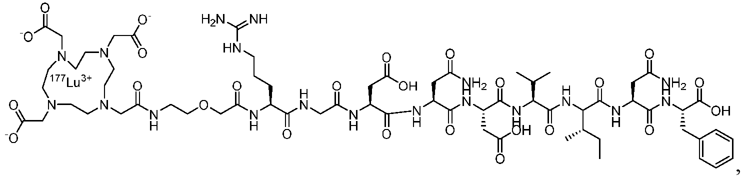 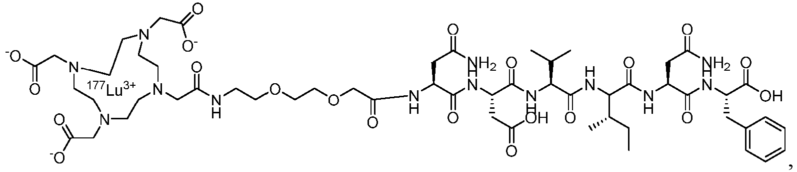 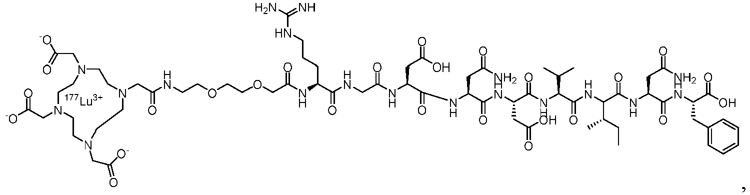 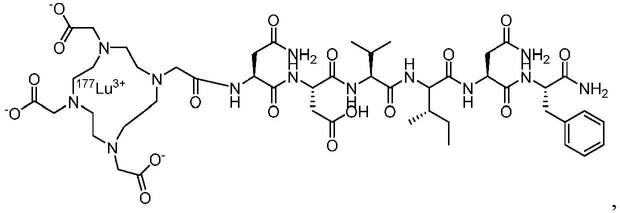 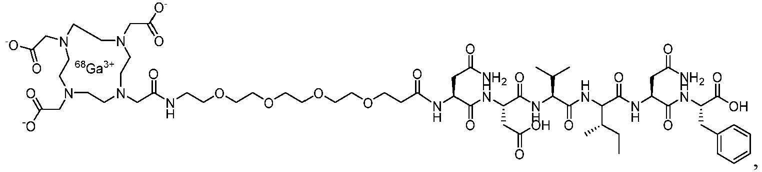 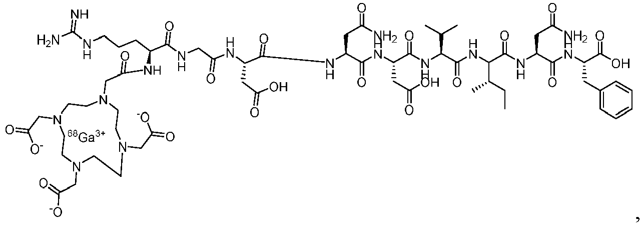 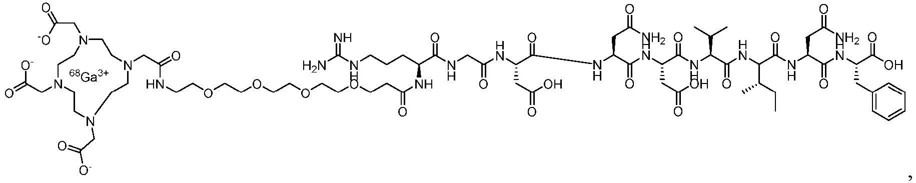 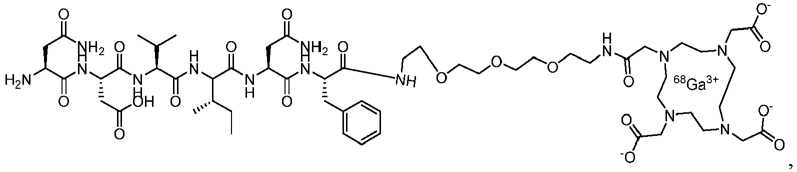 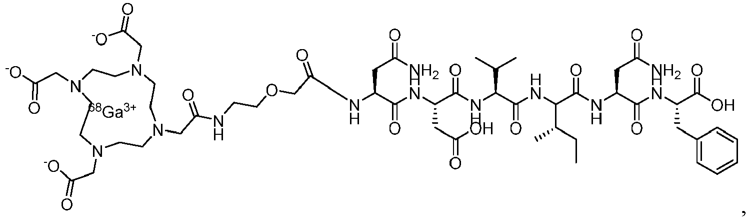 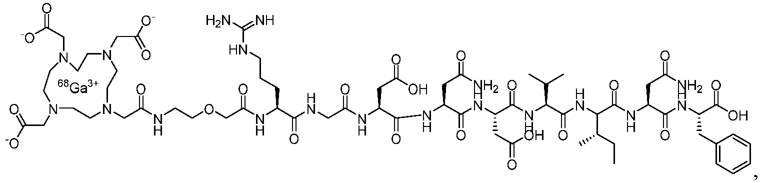 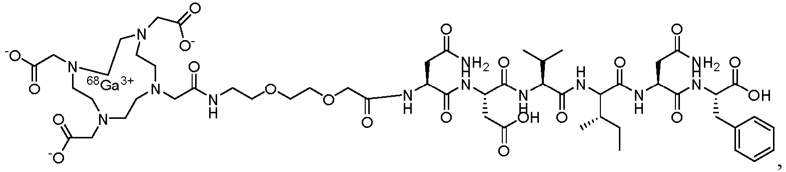 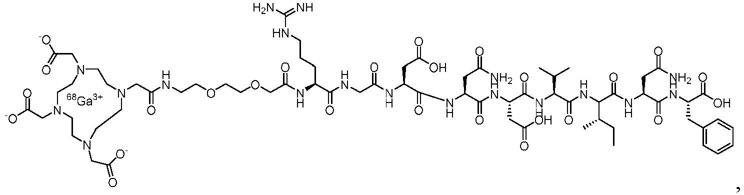 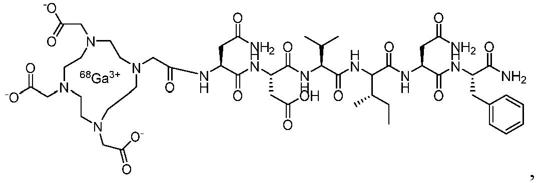 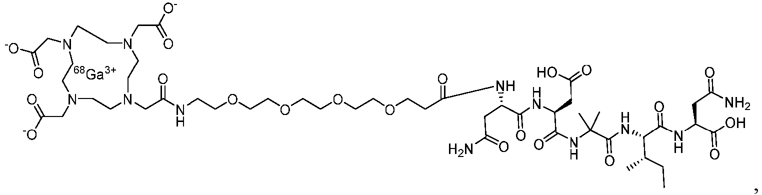 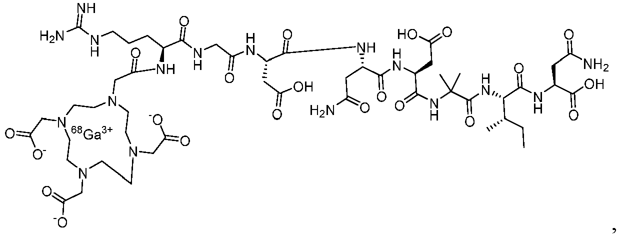 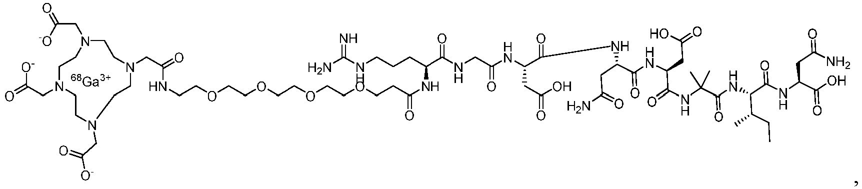 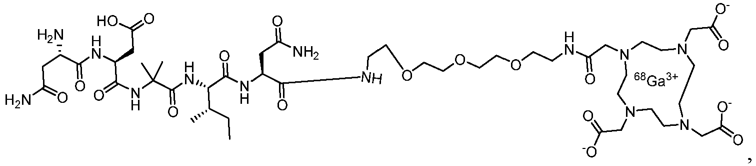 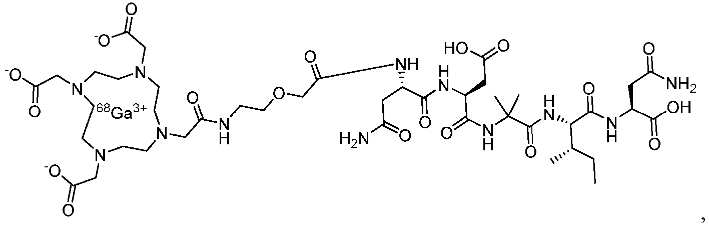 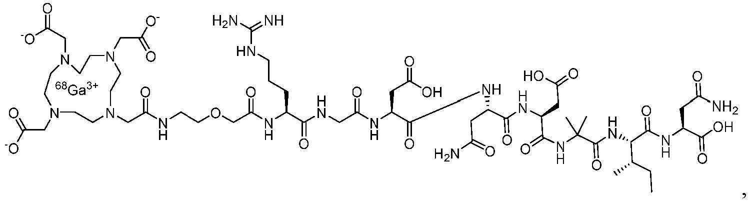 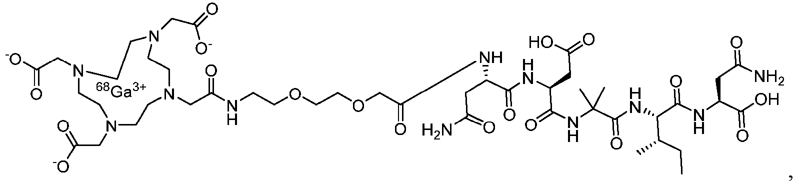 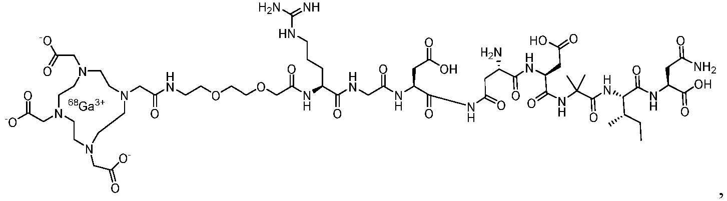 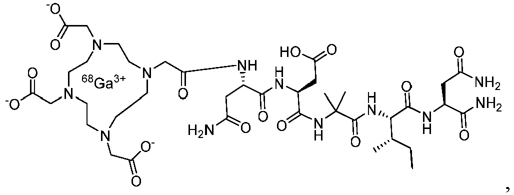 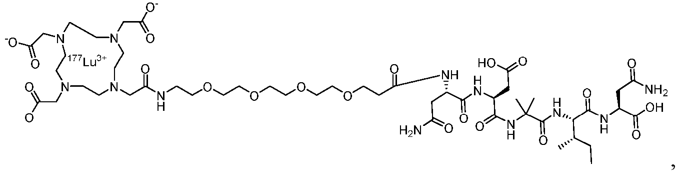 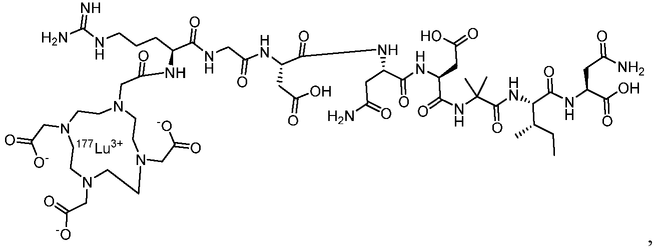 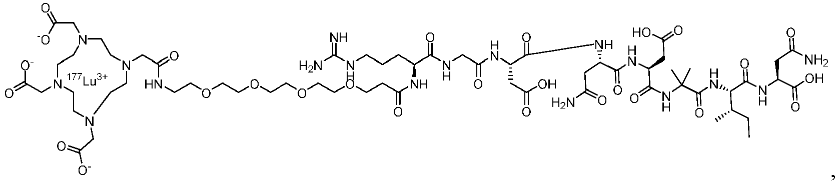 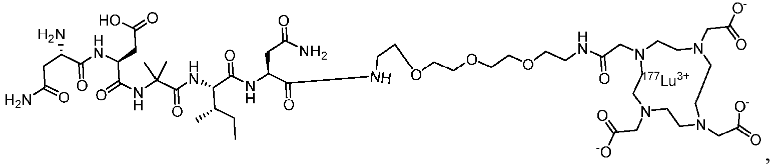 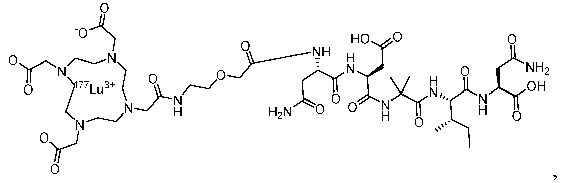 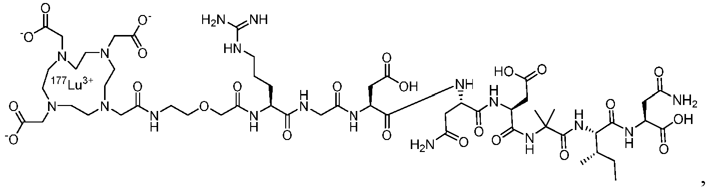 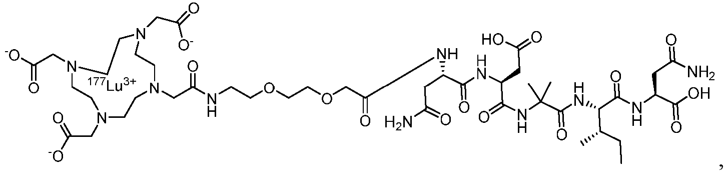 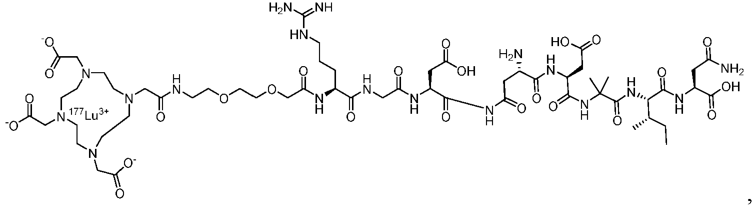 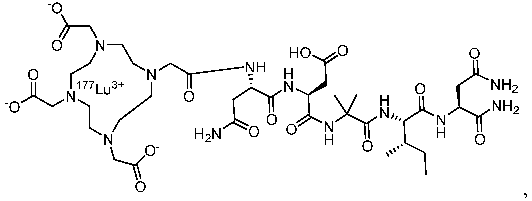 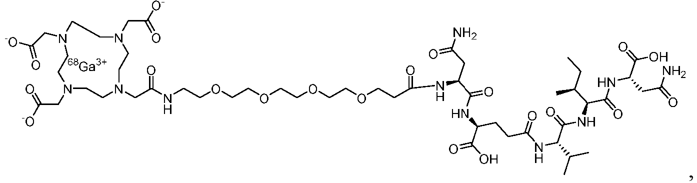 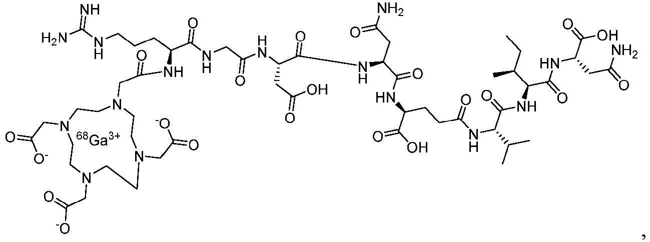 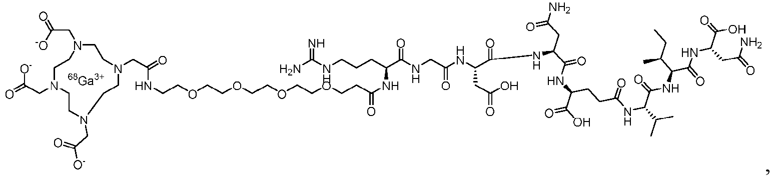 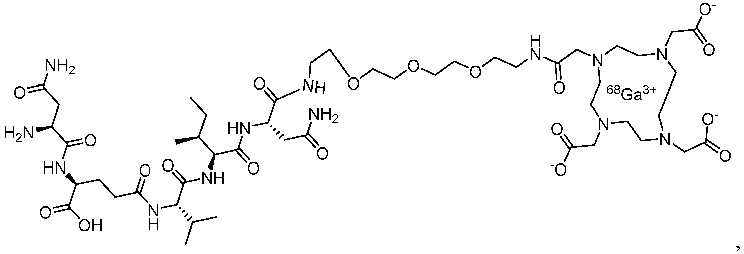 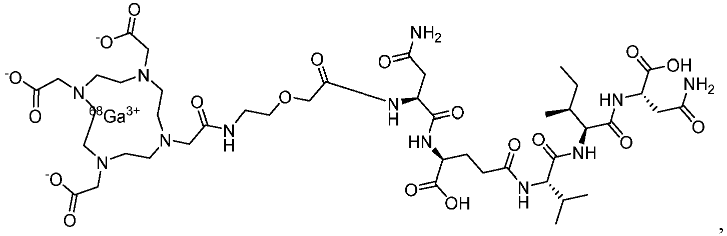 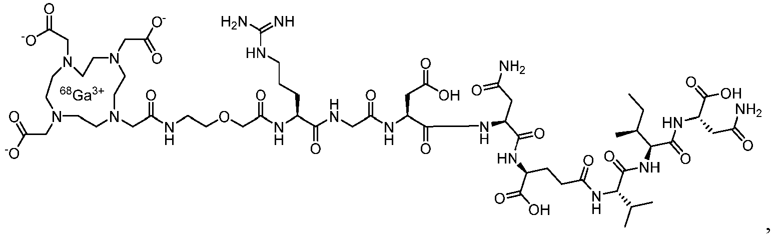 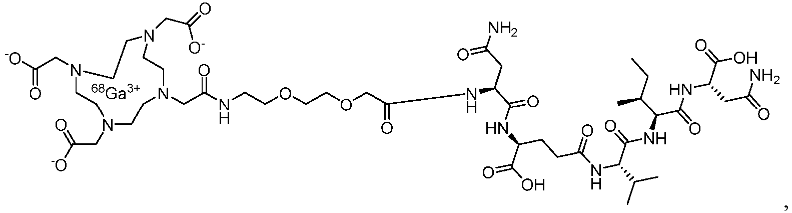 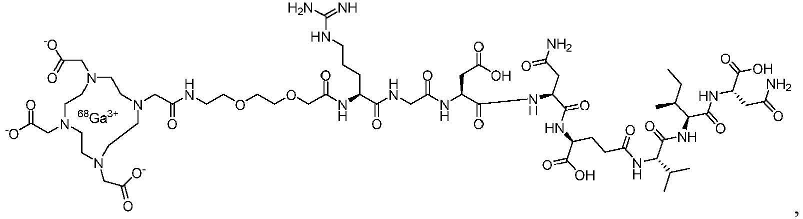 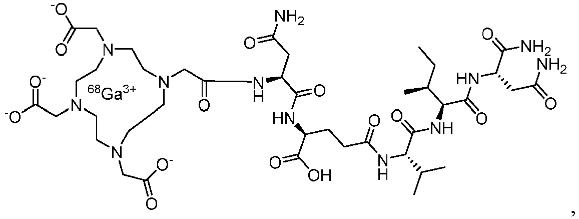 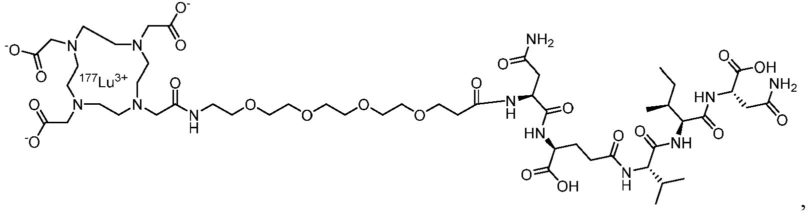 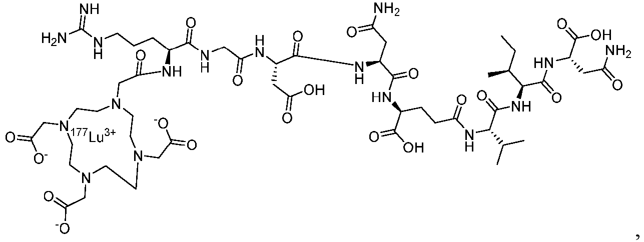 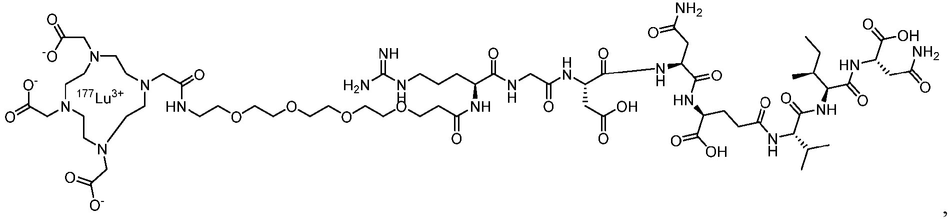 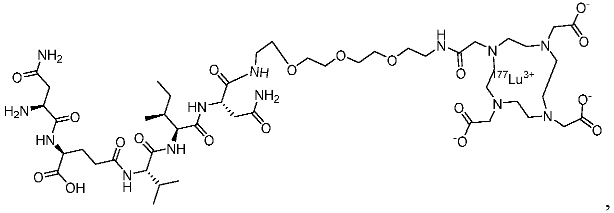 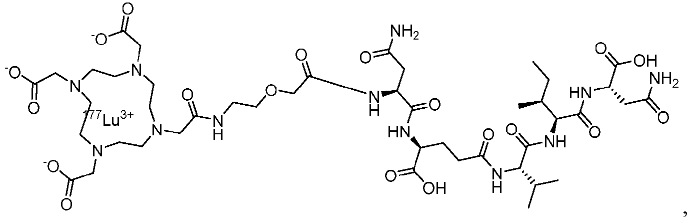 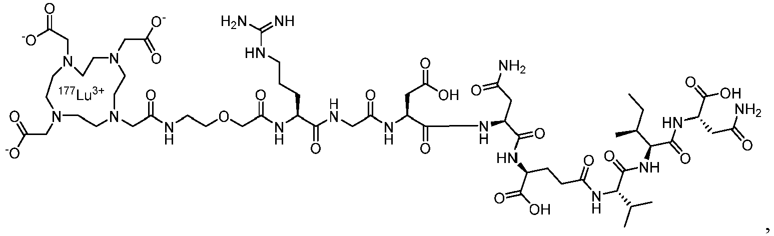 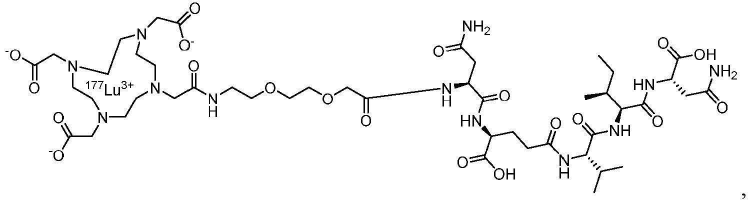 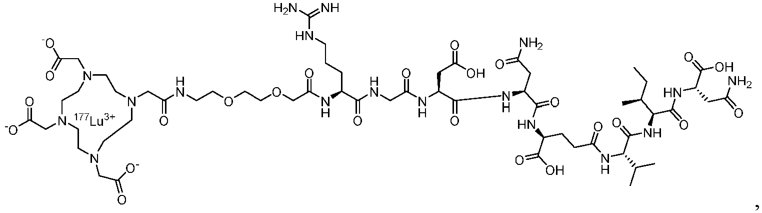 and

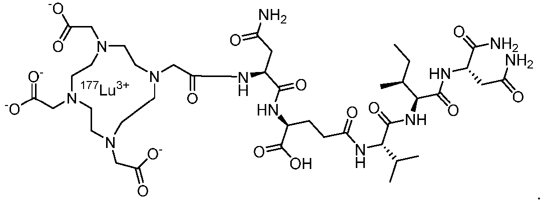

Preferably, the polypeptide-like coordination compound preferably consists of the polypeptide-like compound chelated with a metal. The preparation method therefor preferably comprises the following step of reacting the polypeptide-like compound and a metal compound, preferably mixing the polypeptide-like compound and a metal chloride or a metallic acid salt for reaction. Wherein, the metal in the metallic acid salt is preferably selected from the group consisting of Gd, ¹⁷⁷Lu, ⁶⁴Cu, ⁶⁸Ga, ¹¹¹In , ²¹¹At, ²²⁵Ac, ⁹⁰Y, ²¹³Bi, At, and an isotope thereof, further preferably selected from the group consisting of ¹⁷⁷Lu, Gd, ⁶⁸Ga, and an isotope thereof.

When the polypeptide-like compound is mixed with the metal chloride for reaction, the metal chloride and the polypeptide-like compound have an equivalence ratio of (1-10): 1; the reaction is preferably carried out at a temperature of 20-150°C, at a pH of 3-11; the reaction is further preferably carried out at a temperature of 60-70°C, at a pH of 5.5-6.0, and for a time of 2-3 h.

Alternatively, when the polypeptide-like compound is mixed with the metallic acid salt for reaction, the metallic acid salt and the polypeptide-like compound have an equivalence ratio of (1-10): 1, preferably (1-4): 1; the metallic salt is preferably a nitrate, more preferably gallium nitrate; the reaction is preferably carried out at a temperature of 20-150°C, at a pH of 3-11; the reaction is further preferably carried out at a temperature of 60-70°C, at a pH of 6.5-7.0, and for a time of 2-3 h.

Preferably, the preparation method of the polypeptide-like coordination compound preferably comprises the following steps:
dissolving the polypeptide-like compound in a solvent, mixing it with a base solution, adjusting the pH to 3-11, then adding metal chloride, adjusting the pH to 3-11, and then precipitating the excess metal ions to obtain the polypeptide-like coordination compound;
or, dissolving the polypeptide-like compound in a solvent, heating to 20-150°C, mixing it with a solution of metal acid salt, adjusting the pH to 3-11, performing reaction for 2-3 h to obtain the polypeptide-like coordination compound. Wherein, the solvent and a solvent of the solution of metal acid salt are preferably water, and KOH or NaOH are preferably used to adjust pH.

After mixing the polypeptide-like compound and the metal chloride or the metal acid salt for reaction, the system is preferably purified by high performance liquid chromatography (HPLC).

The present disclosure further provides use of the polypeptide-like compound and/or the polypeptide-like coordination compound in the manufacture of a medicament for diagnosing and/or treating a tumor.

The present disclosure further provides an integrin inhibitor comprising the polypeptide-like compound and/or the polypeptide-like coordination compound.

The present disclosure further provides a pharmaceutical composition comprising the polypeptide-like compound, and/or the polypeptide-like coordination compound, and a pharmaceutically acceptable excipient.

Preferably, the pharmaceutical composition preferably further comprises a carrier. The carrier is preferably a pharmaceutically acceptable carrier.

The polypeptide-like compounds provided by the present disclosure can target integrin-like proteins such as αvβ5, α5β1, and αvβ3, and are enriched in tumor cells with high expression of integrins, which can achieve tumor killing by inhibiting the growth and proliferation of tumor cells, or can provide a carrier for cytotoxic molecules to target tumors and enhance the precise killing of tumors by cytotoxic molecules. In addition, it can also provide a targeting carrier for nuclides and fluorescent molecules to achieve the precise diagnosis and/or treatment of tumors. As confirmed by the test examples of the present disclosure, the KD indicating affinity of the polypeptide-like compound of the present disclosure towards α5β1, αvβ3, and avp5 is nM level.

### BRIEF DESCRIPTION OF DRAWINGS

FIG. 1 is the HPLC chromatogram of the compound (TM1) prepared in example 1 of the present disclosure.
FIG. 2 is the mass spectrogram of the compound (TM1) prepared in example 1 of the present disclosure.
FIG. 3 is the HPLC chromatogram of the compound (TM8) prepared in example 8 of the present disclosure.
FIG. 4 is the mass spectrogram of the compound (TM8) prepared in example 8 of the present disclosure.
FIG. 5 is the HPLC chromatogram of the compound (T1) prepared in example 18 of the present disclosure.
FIG. 6 is the mass spectrogram of the compound (T1) prepared in example 18 of the present disclosure.
FIG. 7 is the HPLC chromatogram of the compound (T3) prepared in example 19 of the present disclosure.
FIG. 8 is the mass spectrogram of the compound (T3) prepared in example 19 of the present disclosure.
FIG. 9 shows the PET/CT imaging results of ⁶⁸Ga-TM11 in ovarian cancer model.
FIG. 10 shows the PET/CT imaging results of ⁶⁸Ga-TM13 in ovarian cancer model.
FIG. 11 shows the PET/CT imaging results of ⁶⁸Ga-TM11 in colon cancer model.
FIG. 12 shows the PET/CT imaging results of ⁶⁸Ga-TM13 in colon cancer model.
FIG. 13 shows the PET/CT imaging results of ⁶⁸Ga-TM4 in lung adenocarcinoma model.
FIG. 14 shows the PET/CT imaging results of ⁶⁸Ga-TM5 in lung adenocarcinoma model.
FIG. 15 shows the PET/CT imaging results of ⁶⁸Ga-TM7 in lung adenocarcinoma model.
FIG. 16 shows the PET/CT imaging results of ⁶⁸Ga-TM11 in lung adenocarcinoma model.
FIG. 17 shows the PET/CT imaging results of ⁶⁸Ga-TM13 in lung adenocarcinoma model.
FIG. 18 shows the MR image of mice.
FIG. 19 shows the statistical values of T1 signals.
FIG. 20 shows the statistical values of T2 signals.

### DETAILED DESCRIPTION

Hereinafter, the technical solutions of the present disclosure will be described clearly and completely in conjunction with the examples of the present disclosure. Apparently, the described examples are only part of the embodiments of the present disclosure, rather than all the embodiments. Based on the examples in the present disclosure, all other embodiments obtained by a person of ordinary skill in the art without making inventive effort are within the scope of protection of the present disclosure.

In order to further describe the present disclosure, examples will be given below for detailed description. The raw materials used in examples below of the present disclosure are all commercially available. The metals used in examples of the present disclosure are radioactive or non-radioactive metals.

### Example 1

The polypeptide-like compound NOTA-Asp-Val-Ile-Asn-Phe-OH in this example, numbered TM1, has the structure shown below:

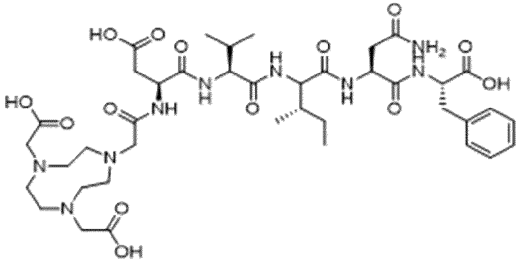

The synthesis route of TM1 is shown below:

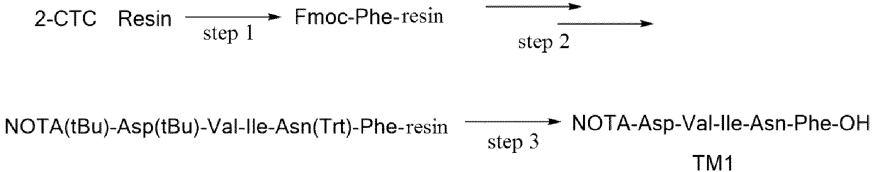

The preparation method includes the following steps:
(1) Step 1. Synthesis of Fmoc-Phe-resin: The 2-CTC Resin (0.503 g, 0.5 mmol) was swelled with DCM (5 ml) in a polypeptide synthesis tube at room temperature for 15 min, and the solvent was drained. The resin was added with a mixed solution of Fmoc-Phe-OH (390 mg, 1 mmol) and DIEA (195 mg, 1.5 mmol) in DCM (5 mL), sparged with nitrogen at room temperature for 2 h, and subsequently added with 0.5 ml of methanol and 0.3 ml of DIEA to continue the reaction for 15 min. After the reaction solution was drained, this system was washed with DCM (5 mL) three times, and finally washed with DMF (5 mL) three times. The solvent was drained to obtain Fmoc-Phe-resin, which was used directly in the next reaction.
(2) Synthesis of NOTA(tBu)-Asp(tBu)-Val-Ile-Asn(Trt)-Phe-resin: Deprotection of Fmoc protective groups: The Fmoc-Asn(Trt)-resin obtained above was added with piperidine/DMF at a volume ration of 1:4 (5mL), and reacted for 5 min at room temperature, drained, added with piperidine/DMF at a volume ration of 1:4 (5 mL) again, and reacted for 5 min. After reaction, the reaction solution was drained and the residue was washed 6 times with DMF (5 mL) and drained for the next reaction.

Coupling amino acids: HOBT (136 mg, 1 mmol), HBTU (380 mg, 1 mmol) and DIEA (195 mg, 1.5 mmol) were each added into the DMF (5 ml) solution of Fmoc-Asn(Trt)-OH (605 mg, 1 mmol) for activation reaction in an ice bath for 15 min. After activation was completed, the activation solution was added into the H-Phe-resin above for 1 h of reaction at room temperature. The reaction solution was drained, and the resin was washed with DMF (5 mL) for 6 times to obtain the Fmoc-Asn(Trt)-Phe-resin.

The above operation was repeated to couple amino acids NOTA(tBu)-OH, Fmoc-Asp(tBu)-OH, Fmoc-Val-OH and Fmoc-Ile-OH one by one to obtain NOTA(tBu)-Asp(tBu)-Val-Ile-Asn(Trt)-Phe-resin, which was washed twice with DCM (5 mL) and MeOH (5 mL) alternately, finally washed twice with MeOH (5 mL), and then dried in a vacuum drying oven at 30°C for 3 h to obtain 1.558 g of NOTA(tBu)-Asp(tBu)-Val-Ile-Asn(Trt)-Phe-resin.

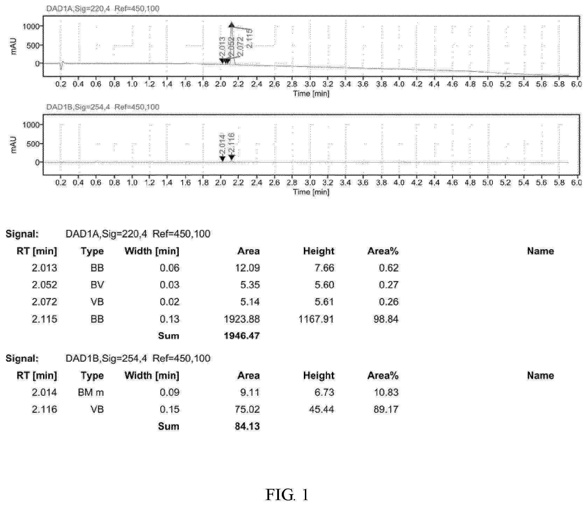

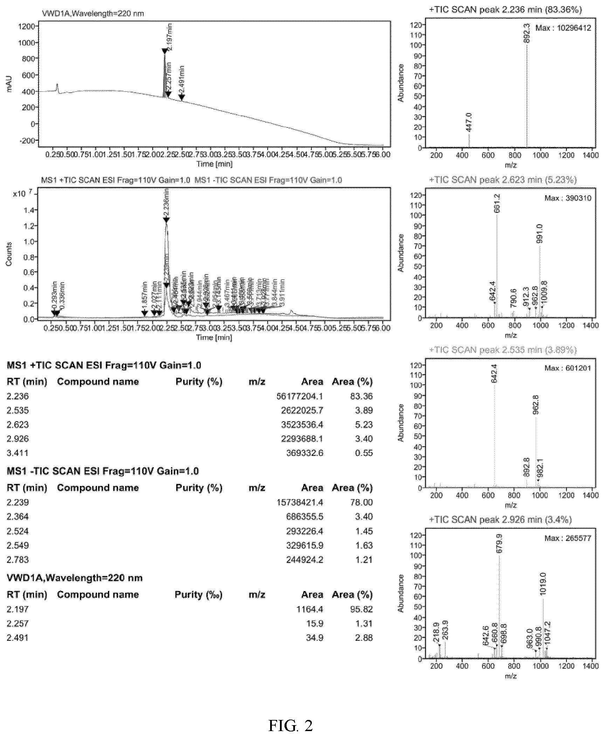

(3) Synthesis of NOTA-Asp-Val-Ile-Asn-Phe-OH: The NOTA(tBu)-Asp(tBu)-Val-Ile-Asn(Trt)-Phe-resin prepared above (1.558g) and the mixed solution of TFA:TIS:H₂O at a volume ration of 95:2.5:2.5 (10ml), were each added into a 50ml reaction flask, and reacted at room temperature for 2 h. After the reaction was completed, the two reaction systems were filtered and washed three times with 5 ml of TFA. The two solutions were combined and concentrated to about 2 ml. The concentrated solution was added dropwise into 40 ml of methyl tert-butyl ether, left for sedimentation in an ice bath for 30 min, centrifuged and washed three times with methyl tert-butyl ether (10 ml) to obtain a solid. The solid was placed into a vacuum oven and dried overnight to obtain 427 mg of white solid. The white solid was directly purified by Pre-HPLC (as shown in FIG. 1) to obtain the purified solution. The purified solution was concentrated and freeze-dried to obtain 30.52 mg of target product TM1 as a white solid, with a purity of 98.84%. The mass spectrometry of TM1 showed a result of MS (ESI): m/z 892.3 [M+H]⁺ (as shown in FIG. 2).

### Examples 2 to 7

Referring to the method in example 1, the deprotection of Fmoc protecting group and the amino acid coupling were repeated using different amino acids to obtain the polypeptide-like compound TM2 to TM7 in Table 1.

### Example 8

The polypeptide-like compound of this example has a structure shown below:

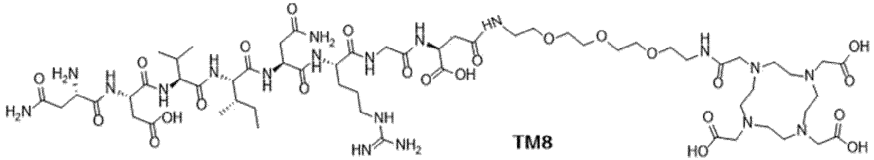

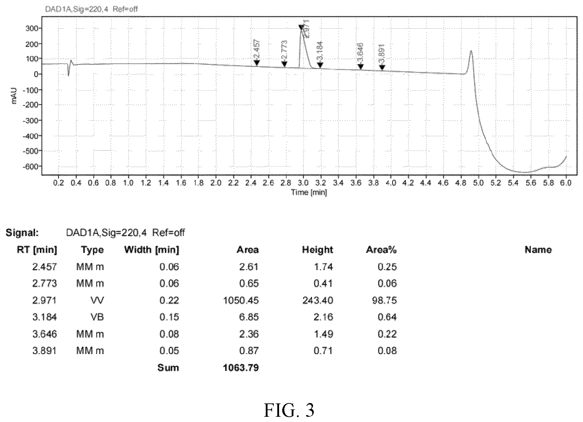

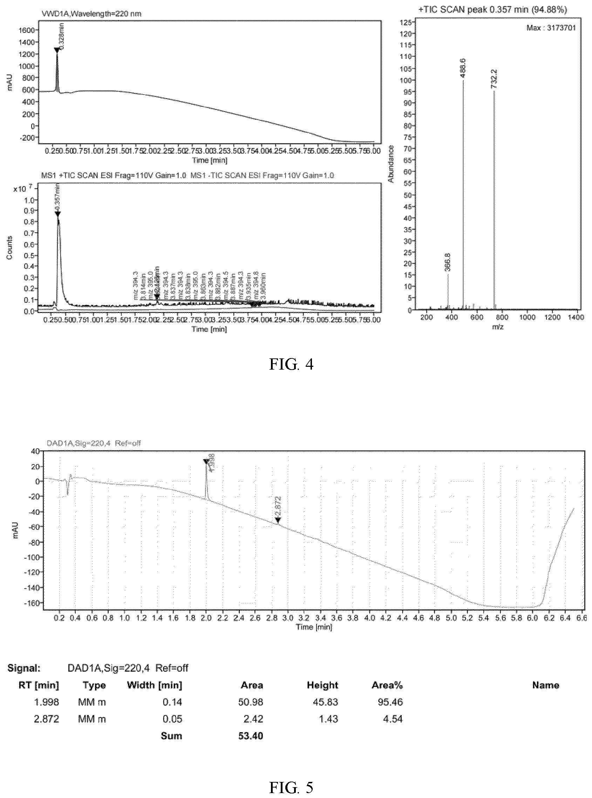

The preparation method comprises the following steps:
(1) Referring to the approach in step (1) and step (2) in example 1, Boc-Asn(Trt) - Asp(OtBu)-Val-Ile-Asn(Trt)-Arg(Pbf)-Gly-Asp(OAll)-CTC resin was prepared. A solution of the above resin (0.5 mmol) in DCM (20 ml) was added with 35 mg of PdCl₂ (dppf) and TIS (0.5 ml) to react under nitrogen protection at room temperature for 1 h. After the reaction was completed, the resin was washed three times with DCM (10 ml), and finally washed three times with DMF (10 ml) to obtain an intermediate M2.
(2) A solution of HOBT (135 mg, 1 mmol), HBTU (382 mg, 1 mmol), DIEA (198 mg, 1.5 mmol) and M3 (503 mg, 0.66 mmol) in DMF (5 ml) was added with the intermediate M2, and reacted at room temperature for 1 h. The reaction solution was drained, and the resin was washed with DMF (5 mL) for 6 times, then washed alternately with DCM (5 mL) and MeOH (5 mL) for twice, and finally washed with MeOH (5 mL) twice. The obtained resin was dried in a vacuum drying oven at 30°C for 3 h to obtain an intermediate M4 (1.788 g).
(3) M4 (1.788g) prepared above and a mixed solution of TFA:TIS:H2O at a volume ratio of 95:2.5:2.5 (15ml) were each added into a 50ml reaction flask to react at room temperature for 2 h. After the reaction was completed, the two reaction systems were filtered and washed three times with 5 ml of TFA. The two solutions were combined and concentrated to about 2 ml. The concentrated solution was added dropwise into 40 ml of methyl tert-butyl ether, left for sedimentation in an ice bath for 30 min, centrifuged and washed three times with methyl tert-butyl ether (10 ml) to obtain a solid. The solid was placed into a vacuum drying oven and dried overnight to obtain 456 mg of white solid. The white solid was directly purified by Pre-HPLC to obtain a purified solution. The purified solution was concentrated and freeze-dried to obtain 15.40 mg of target product TM8 as a white solid, with a purity of 98.75% (as shown in FIG. 3). The mass spectrometry showed a result of MS (ESI): m/z 732.2 [M/2+H]⁺ (as shown in FIG. 4). The reaction process was shown below:

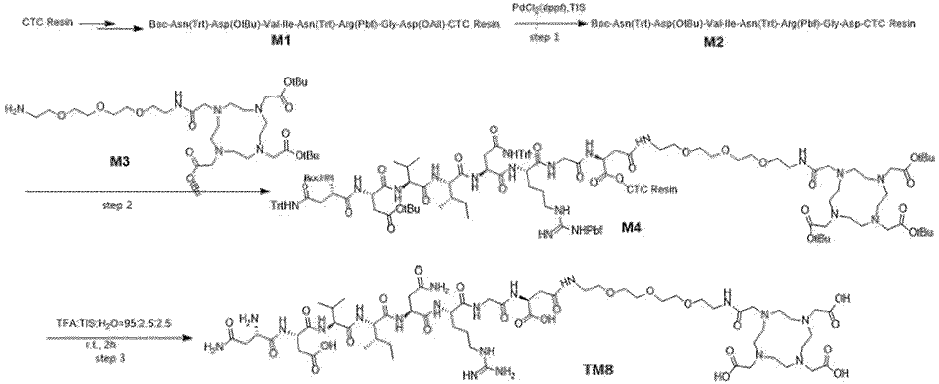

### Examples 9 to 18

Referring to the method in example 1, the deprotection of Fmoc protecting group and the amino acid coupling were repeated using different amino acids to obtain the polypeptide-like compound TM9 to TM18 in Table 1.

**Table 1 Structure and related characterization of polypeptide-like compounds prepared in example 1 to example 17**

| Number of compound | Structure formula | MS(ESI+):m/z [M+H]⁺ | Mass (mg) | Purity (%) |
|---|---|---|---|---|
| TM1 | 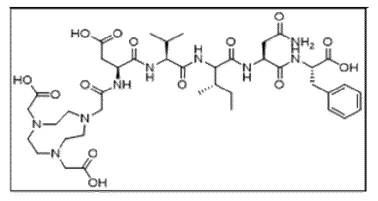 | 892.3 | 30.52 | 98.84 |
| TM2 | 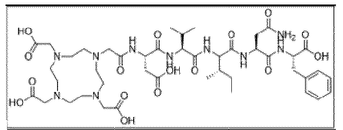 | 993.8 | 11.04 | 97.40 |
| TM3 | 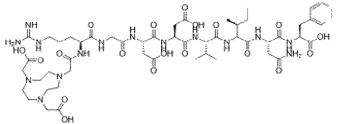 | 1222.0 | 71.52 | 98.97 |
| TM4 | 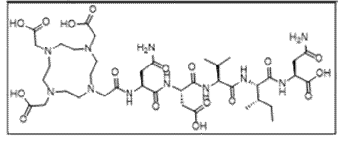 | 960.4 | 62.10 | 97.97 |
| TM5 | 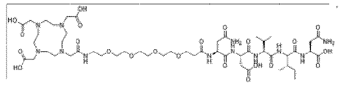 | 1207.3 | 100.5 0 | 97.27 |
| TM6 | 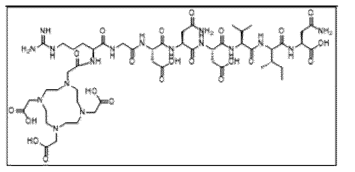 | 1288.2 | 74.51 | 96.18 |
| TM7 | 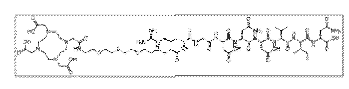 | [M/2+H]⁺=768. 6 | 127.5 0 | 94.20 |
| TM8 | 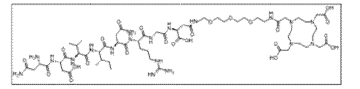 | [M/2+H]⁺=732. 2 | 15.40 | 98.75 |
| TM9 | 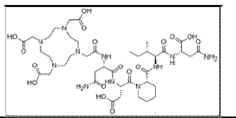 | 972.3 | 32.51 | 95.54 |
| TM10 | 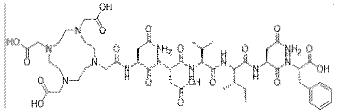 | 1107.2 | 29.92 | 97.09 |
| TM11 | 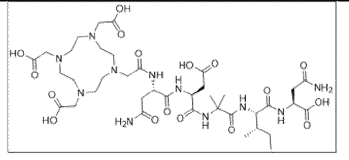 | 946.2 | 39.28 | 98.16 |
| TM12 | 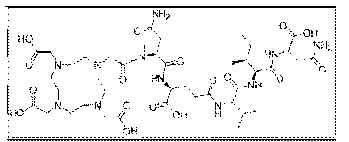 | 974.2 | 1.87 | 95.82 |
| TM13 | 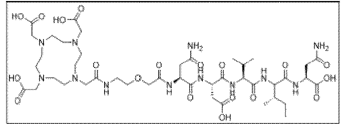 | 1061.2 | 222.1 2 | 93.66 |
| TM14 | 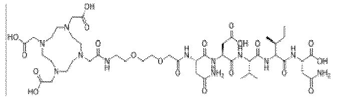 | 1105.3 | 220.7 3 | 95.01 |
| TM15 | 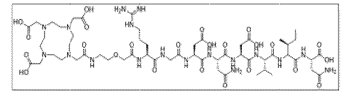 | 1389.3 | 189.8 6 | 93.17 |
| TM16 | 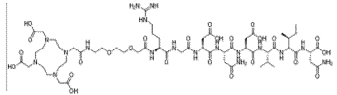 | [M/2+H]⁺=717. 7 | 195.0 7 | 95.51 |
| TM17 | 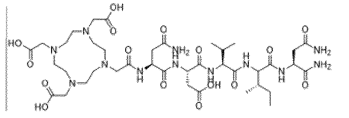 | 959.4 | 112.1 0 | 94.84 |
| TM18 (HTPM70 04-036) | 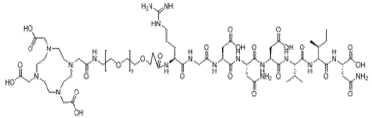 | M/2+H+=900.8 | 53.2 | 97.28 |
| | | M/3+H+=600.9 | | |

### Example 19

The polypeptide-like compound of this example has a structure shown below:

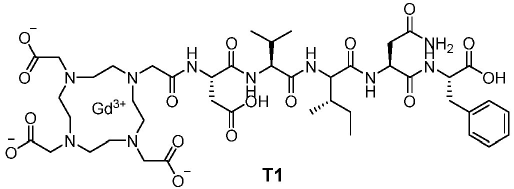

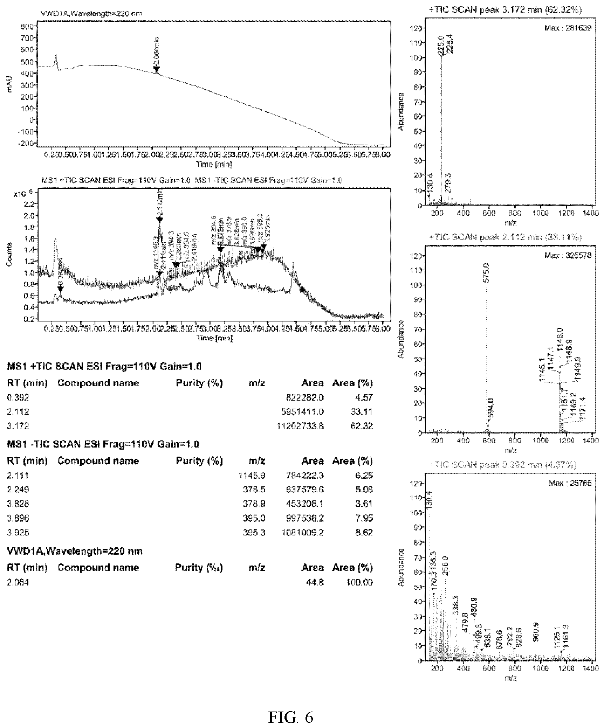

The preparation method comprises the following steps:
30 mg (0.03 mmol) of TM1 was dissolved in 2 mL of H₂O, and added with 0.5 mL of 0.5 M NaOH aqueous solution (8eq) to obtain a reaction solution. The reaction solution was controlled at a pH of 6-8, stirred for 2.5 h at room temperature, and added with GdCl₃ · 6H₂O (0.012 g, 1.1eq). The pH of the reaction solution was reduced to 5.5 with 5 w/v % HCI. The solution was stirred overnight, and the pH of the solution was raised to 12 with 0.1 M NaOH to precipitate excess Gd (III). The reaction mixture was centrifuged, and the resulting supernatant was freeze-dried to obtain a white powder, which was purified by preparative HPLC and freeze-dried to obtain 14.30 mg of the target product T1 with a purity of 95.46%. The purification process is shown in FIG. 5. The mass spectrometry showed a result of MS(ESI): m/z 1148.0 [M+H]⁺, 575.0 [M/2+H]⁺ (as shown in FIG. 6).

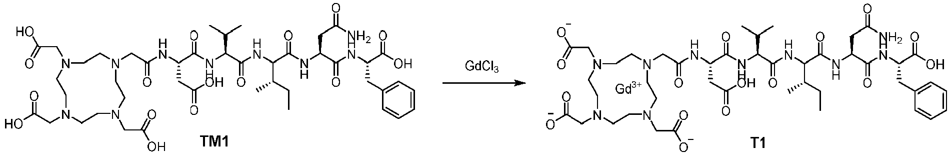

### Example 20

The polypeptide-like compound of this example has a structure shown below:

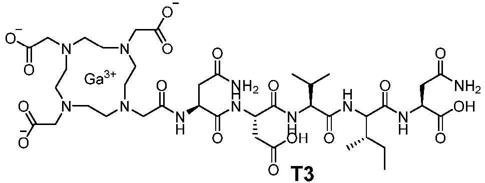

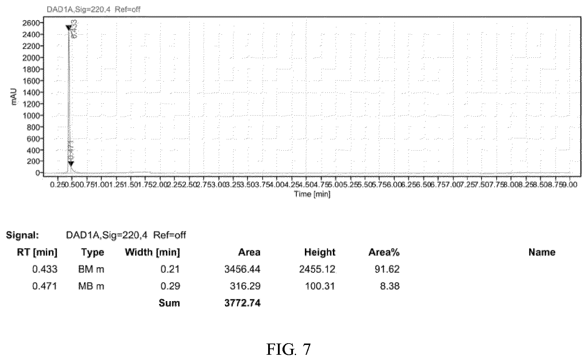

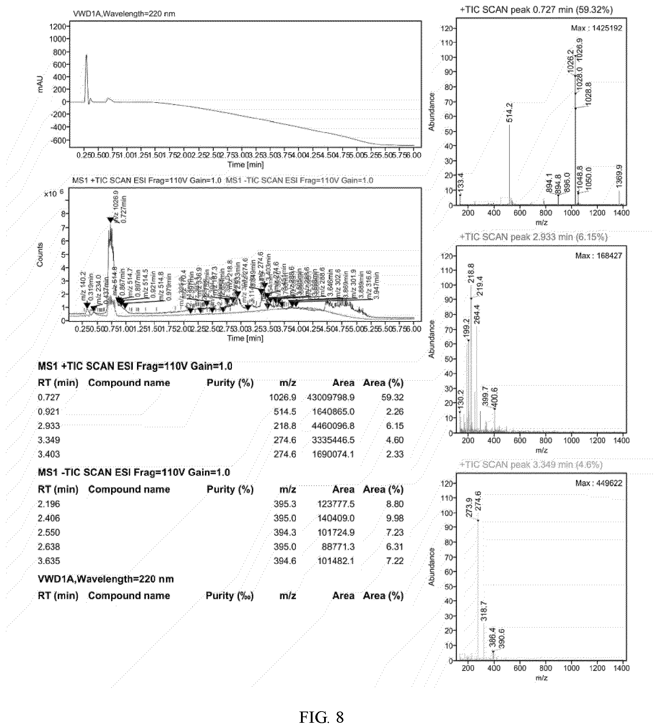

The preparation method comprises the following steps:
TM4 (80.01 mg) was weighted and dissolved in 7 ml of purified water, and heated to 70°C. The obtained solution was added dropwise into 1 ml of aqueous solution dissolved with 22.31 mg of gallium nitrate to obtain a mixture. The mixture was stirred for reaction, and the pH of the mixture was adjusted to 7.0 with KOH aqueous solution (1 mM) for 2 h of reaction. After the reaction was completed, the reaction system was desalted by using the inverse purification system to obtain the desired target compound T3 (60.01 mg), with a purity of 91.62%, and a yield of 70.8%. The purification process is shown in FIG. 7. The mass spectrometry showed a result of MS(ESI): m/z 1028.8 [M+H]⁺, 514.2 [M/2+H]⁺ (as shown in FIG. 8).

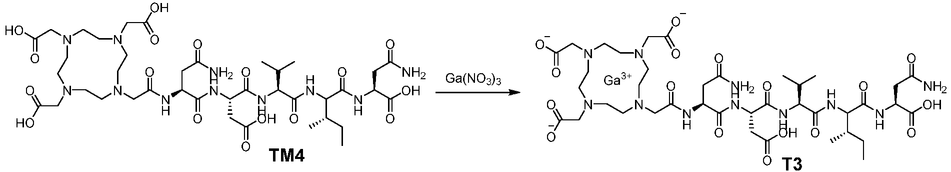

### Examples 21 to 26

Referring to the method of Example 19, the chelation of Gd was performed to obtain the polypeptide-like coordination compounds of Gd³⁺, namely T4, T6, T8, T10, T12, and T13, as shown in Table 2 below.

### Examples 27 to 33

Referring to the method of Example 20, the chelation of Ga was performed to obtain the polypeptide-like coordination compounds of Ga³⁺, namely T2, T5, T7, T9, T11, T12, T14, and T15, as shown in Table 2 below.

**Table 2 Structure and related characterization of polypeptide-like coordination compounds prepared in examples 18 to 32**

| Numb er | Structure | Result of mass spectrome try | Mass (mg) | Purity (%) |
|---|---|---|---|---|
| T1 | 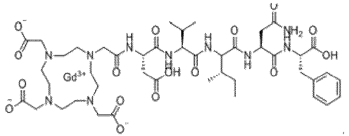 | 1148.0 | 14.30 | 95.46 |
| T2 | 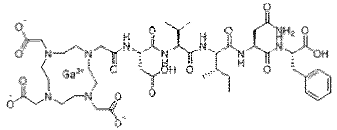 | 1509.2 | 17.35 | 96.73 |
| T3 | 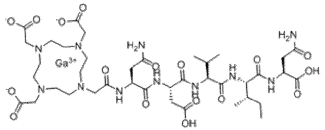 | 1026.2 | 60.10 | 91.62 |
| T4 | 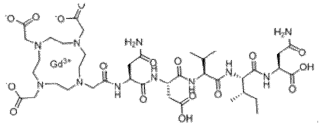 | 1115.3 | 26.77 | 97.76 |
| T5 | 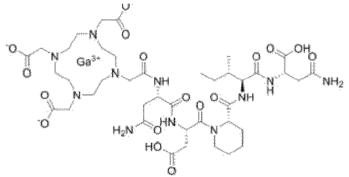 | 1038.2 | 11.38 | 93.56 |
| T6 | 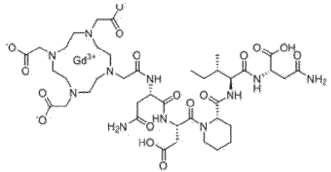 | 1127.1 | 28.61 | 93.31 |
| T7 | 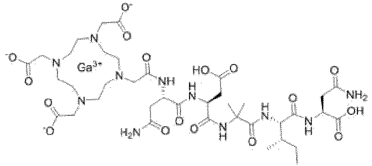 | 1012.2 | 20.05 | 98.63 |
| T8 | 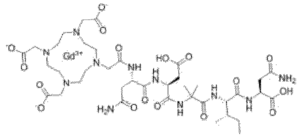 | 1101.1 | 21.22 | 96.78 |
| T9 | 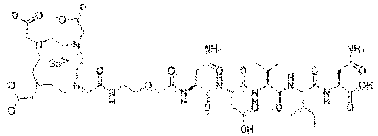 | 1127.2 | 26.15 | 98.81 |
| T10 | 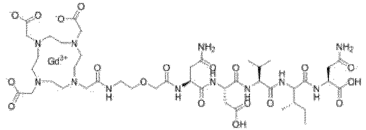 | 1216.2 | 23.33 | 98.87 |
| T11 | 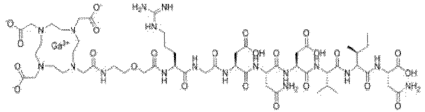 | 1455.4 | 15.56 | 95.55 |
| T12 | 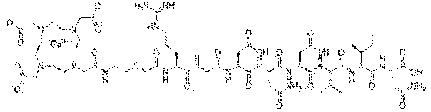 | 1544.4 | 17.32 | 95.78 |
| T13 | 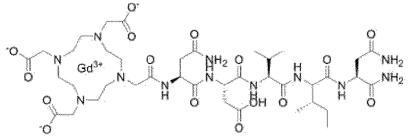 | 1114.2 | 34.38 | 97.76 |
| T14 | 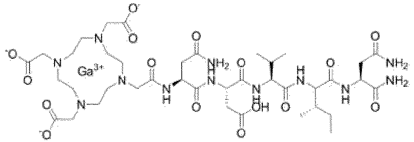 | 1025.2 | 36.76 | 97.57 |
| T15 | 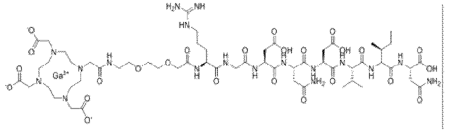 | 1499.5 | 15.59 | 95.88 |

### Test example 1 Target affinity test

Surface plasmon resonance (SPR) technique was used to test the affinity of the compound in the examples to the target protein integrin α5β1. Specifically, the following operations were performed.

The recombinant integrin α5β1 or αvβ3 proteins were immobilized on a CM5 chip (Biacore), and an SPR instrument (Biacore T200) was used to test the binding constants (Ka) and dissociation constants (Kd) of the compounds to integrin α5β1 or αvβ3 at 25°C, wherein the loading volume of the solution to be tested was 30 µL and the flow rate was 30 µL/min. The affinity constants (KD) of the compounds to the target proteins integrin α5β1, αvβ3, and αvβ5 were calculated according to the formula of KD=Kd/Ka (the lower the value of KD, the higher the affinity of the compounds of the examples to the target protein integrin α5β1). The results of affinity test of the compounds to integrin α5β1 are shown in Table 2, which shows the affinity constants of the compounds to integrin α5β1 or αvβ3.

The test results of the compounds prepared in examples are shown in Table 3 below.

**Table 3 Affinity parameter of the polypeptide-like compounds prepared in examples to integrin α5β1 or αvβ3**

| Number of polypeptide-like compound | KD (nM) | | |
|---|---|---|---|
| | α5β1 | αvβ3 | αvβ5 |
| TM3 | 1.92 | / | / |
| TM4 | 16 | 2.88 | / |
| TM5 | 2.77 | / | / |
| TM6 | 239 | / | / |
| TM7 | 43.9 | / | / |
| TM9 | 5.58 | 1.8 | / |
| TM11 | 0.0681 | 190 | / |
| TM13 | 1450 | 0.646 | / |
| TM14 | 9.43 | 36.7 | / |
| TM15 | 0.992 | 1.69 | / |

As can be seen from the above experimental data, the affinity constant (KD) of polypeptide-like compounds TM3, TM5, TM9, TM14, TM15 and TM11 to integrin α5β1 is better than nM level. In addition, the affinity constant (KD) of the polypeptide-like compounds TM4, TM9, TM13, and TM15 to integrin αvβ3 is of nM level.

In addition, all the polypeptide-like coordination compounds T1 to T15 have an affinity constant (KD) of nM level for target proteins integrin αvβ3 and α5β1, as detected by surface plasmon resonance.

### Test example 2 Radioactive labeling of compound

For example, labeling TM4 with radioactive ⁶⁸Ga: An aqueous solution of TM4 (DOTA-Asn-Asp-Val-Ile-Asn-OH) (20 µg) was added to the prepared ⁶⁸GaCl₃ solution, and the pH was adjusted to 3-5 with aqueous sodium acetate and acetic acid. This system was reacted at 70-95°C for 15 min, and then purified after the reaction was completed to obtain a labeled solution of ⁶⁸Ga-DOTA-Asn-Asp-Val-Ile-Asn-OH (⁶⁸Ga-TM4).

All compounds were labeled with ⁶⁸Ga in the same way as described above.

### Positron emission tomography-computerized tomography (PET/CT) assay

### 1) Evaluation of compound in ovarian cancer model

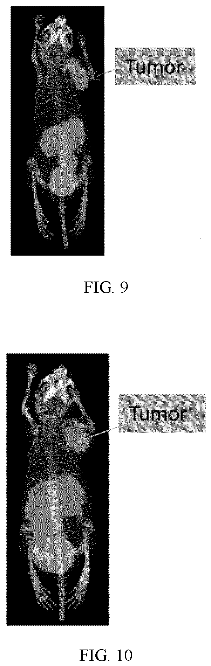

Approximately 100 µCi of ⁶⁸Ga injection was injected into the tail vein of naked mice inoculated with SKOV3. PET/CT imaging was performed 1 h after the injection, and the results are shown in FIGs. 9 and 10. FIG. 9 shows the PET/CT imaging results of ⁶⁸Ga-TM11 in ovarian cancer model, FIG. 10 shows the PET/CT imaging results of ⁶⁸Ga-TM13 in ovarian cancer model, and Table 4 was obtained by image post-processing.

**Table 4 Results of PET/CT assay**

| % ID/g | Heart | Liver | Lung | Kidney | Brain | Bone | Muscle | Intestine | Tumor | Bladder |
|---|---|---|---|---|---|---|---|---|---|---|
| ⁶⁸Ga-TM11 | 0.11 | 0.15 | 0.08 | 0.52 | 0.09 | 0.04 | 0.05 | 0.33 | 0.42 | 4.7 |
| ⁶⁸Ga-TM13 | 0.17 | 0.30 | 0.12 | 1.4 | 0.09 | 0.08 | 0.08 | 0.85 | 0.49 | 15.6 |

The results of the animal experiments in Table 4 showed that the tested compounds exhibited a significant enrichment in the tumor of naked mice inoculated with SKOV3, and the output ratios of tumor/muscle were both greater than 6. As can be seen from the data shown in Table 4, the tested compounds can be rapidly cleared in various tissues, and no significant enrichment was found. From the data, it can be deduced from the output values in the kidney and bladder that the excretion route of the compounds was mainly through the kidney.

### 2) Evaluation of compound in colon cancer model

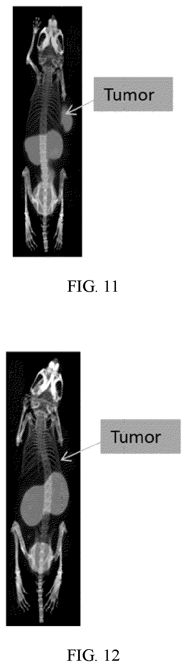

Approximately 100 µCi of ⁶⁸Ga injection was injected into the tail vein of naked mice inoculated with MC38. PET/CT imaging was performed 1 h after the injection, and the results are shown in FIGs. 11 and 12. FIG. 11 shows the PET/CT imaging results of ⁶⁸Ga-TM11 in colon cancer model, FIG. 12 shows the PET/CT imaging results of ⁶⁸Ga-TM13 in colon cancer model, and Table 5 was obtained by image post-processing.

**Table 5 Results of PET/CT assay**

| % ID/g | Heart | Liver | Lung | Kidney | Brain | Bone | Muscle | Intestine | Tumor | Bladder |
|---|---|---|---|---|---|---|---|---|---|---|
| ⁶⁸Ga -TM11 | 0.25 | 0.29 | 0.15 | 1.2 | 0.18 | 0.08 | 0.08 | 0.59 | 0.73 | 18.9 |
| ⁶⁸Ga -TM13 | 0.11 | 0.16 | 0.08 | 1.6 | 0.09 | 0.05 | 0.05 | 0.20 | 0.20 | 7.3 |

The results of the animal experiments in Table 5 showed that the tested compounds exhibited a significant enrichment in the tumor of naked mice inoculated with MC38, and the output ratios of tumor/muscle were both greater than 4. As can be seen from the data shown in Table 5, the tested compounds can be rapidly cleared in various tissues, and no significant enrichment was found. From the data, it can be deduced from the output values in the kidney and bladder that the excretion route of the compounds was mainly through the kidney.

### 3) Evaluation of compound in lung adenocarcinoma model

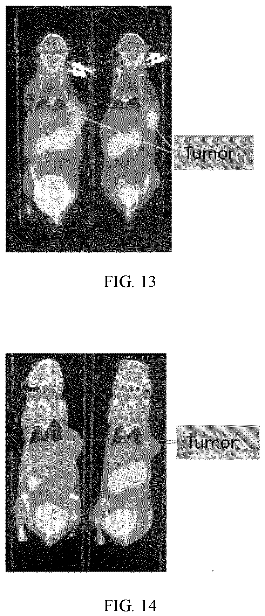

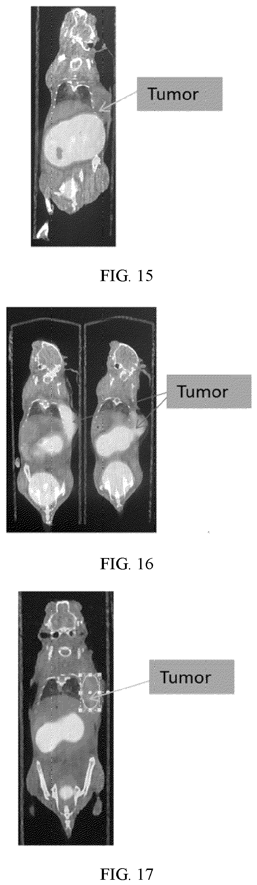

Approximately 100 µCi of ⁶⁸Ga injection was injected into the tail vein of M-NSG mice inoculated with H1975. PET/CT imaging was performed 1 h after the injection, and the results are shown in FIGs. 13, 14, 15, 16 and 17. FIG. 13 shows the PET/CT imaging results of ⁶⁸Ga-TM4 in lung adenocarcinoma model, FIG. 14 shows the PET/CT imaging results of ⁶⁸Ga-TM5 in lung adenocarcinoma model, FIG. 15 shows the PET/CT imaging results of ⁶⁸Ga-TM7 in lung adenocarcinoma model, FIG. 16 shows the PET/CT imaging results of ⁶⁸Ga-TM11 in lung adenocarcinoma model, and FIG. 17 shows the PET/CT imaging results of ⁶⁸Ga-TM13 in lung adenocarcinoma model. Table 6 was obtained by image post-processing.

**Table 6 Results of PET/CT assay**

| %ID/g | ⁶⁸Ga -TM4 | ⁶⁸Ga -TM5 | ⁶⁸Ga -TM7 | ⁶⁸Ga -TM11 | ⁶⁸Ga -TM13 |
|---|---|---|---|---|---|
| Heart | 0.96 | 0.45 | 0.50 | 0.53 | 0.49 |
| Liver | 0.79 | 0.50 | 0.53 | 0.43 | 0.50 |
| Lung | 0.47 | 0.24 | 0.30 | 0.31 | 0.32 |
| Kidney | 4.80 | 7.40 | 64.50 | 4.00 | 3.90 |
| Brain | 0.50 | 0.25 | 0.24 | 0.21 | 0.20 |
| Bone | 0.30 | 0.22 | 0.24 | 0.27 | 0.13 |
| Muscle | 0.36 | 0.21 | 0.28 | 0.31 | 0.23 |
| Intestine | 0.56 | 0.57 | 0.45 | 0.50 | 0.60 |
| Tumor | 1.4 | 1.10 | 0.75 | 0.94 | 0.97 |

The results of the animal experiments in Table 6 showed that the tested compounds exhibited a significant enrichment in the tumor of M-NSG mice inoculated with H1975, and 30 min after injection, the output ratios of tumor/lung and tumor/muscle were all greater than 2. The tested compounds can be rapidly cleared in various tissues, and no significant enrichment was found. From the data, it can be deduced from the output values in the kidney and bladder that the excretion route of the compounds was mainly through the kidney.

### 4) Evaluation of compound in breast cancer model

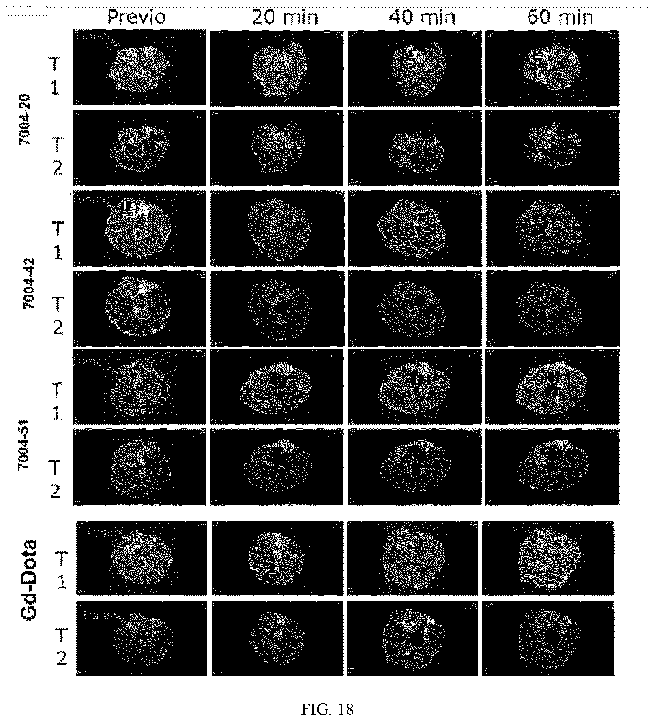

Gd-dota (CAS:72573-82-1, also known as Gd-DOTA), Gd-dtpa (CAS:80529-93-7, also known as Gd-DTPA), T1 (7004-020), T4 (7004-042), and T10 (7004-051) were dissolved in physiological saline and prepared to a concentration of 0.1 mmol/ml. The obtained solution was filtered into sterile EP tubes through sterile 0.22 µm filter tip. BALB/c naked mice of the MD-MB-231 breast cancer model were anaesthetized by intraperitoneal injection of chloral hydrate (100 µL). Magnetic resonance (MR) cross-sectional imaging of the mice was acquired prior to drug injection in all experimental groups, and the acquisition time was recorded. The mice of breast cancer model were administered with the drug by injection via tail vein at a dose of 0.1 mmol/kg, and the administration time was recorded. The MR images (T1 signal, and T2 signal) were acquired 5 min, 10 min, 20 min, 30 min, 40 min, 50 min, 60 min and 70 min after injection of the drug. After each acquisition of the MR images, the mice were kept warm and the imaging results of MR images are shown in FIG. 18, which shows the MR images of mice.

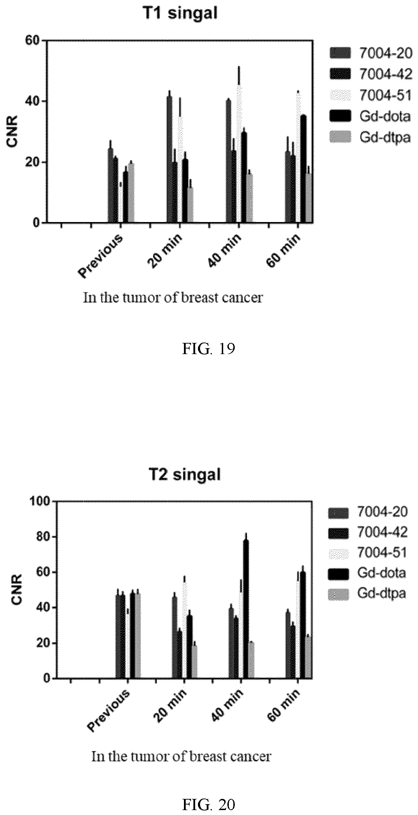

Analysis of MR image of animal: Firstly, the whole tumor (T) excluding the tumor pseudocapsule was circled to obtain its grey value by using Image J, at the same time, the muscle (M) was circled to obtain its grey value. The above count was performed three times. The average value was calculated, and the statistical analysis of the data from T1 and T2 signals in the breast cancer tumor was performed. The results are shown in FIGs. 19 and 20. FIG. 19 shows the statistical value of the T1 signals, and FIG. 20 shows the statistical value of the T2 signals. The calculation formula is CNR= (SIperiphery-SImuscle)/Sdair, wherein SIperiphery represents the grey value of the peripheral tumor tissues, SImuscle represents the grey value of the muscles, Sdair represents the grey value of air, and CNR represents the signal-to-noise ratio of the tumor tissues.

As can be seen from FIG. 19 and FIG. 20, the imaging effect of 7004-020 and 7004-051 are better than that of Gd-DOTA and Gd-DTPA within 20 min for T1 and T2 signals, the imaging effect of 7004-020 and 7004-051 are better than that of Gd-DOTA and Gd-DTPA within 40 min for imaging data of T1 signals, and the imaging effect of 7004-051 is better than that of Gd-DOTA and Gd-DTPA within 60 min for imaging data of T1 signals.

The results of animal experiments using PET/CT and MR imaging show that the polypeptide-like compound, polypeptide-like compound carriers and polypeptide-like coordination compound prepared by the present disclosure can directly target tumor cells, and can also be used as targeting carriers to precisely deliver cytotoxins, nuclides and the like to tumor cells.

The experimental results of *in vitro* enzymatic data show that the polypeptide-like compound and polypeptide-like coordination compound prepared by the present disclosure can be used in the diagnosis and treatment of various tumors such as lung adenocarcinoma, melanoma, malignant primary neuroendocrine prostate cancer and its metastatic lesions in the lungs, breast cancer, ovarian cancer, and tumors of the gastrointestinal tract.

The above-disclosed description of examples enables those skilled in the art to implement or use the present disclosure. Various modifications to these examples are obvious to those skilled in the art, the general principles defined herein may be implemented in other examples without departing from the spirit and scope of the present disclosure. Therefore, the present disclosure is not limited to the examples described herein, but should be in accordance with the broadest scope consistent with the principle and novel features disclosed herein.

## Claims

1. A polypeptide-like compound, wherein the polypeptide-like compound is prepared from PD and ZT, or from PD, ZT and linker;
wherein, ZT has a structure represented by formula I-ZT: 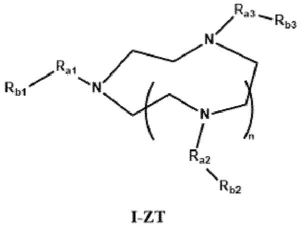
in formula I-ZT, n is an integer selected from 1 to 8,
Rₐ₁, Rₐ₂, and Rₐ₃ are each selected from the group consisting of substituted or unsubstituted alkylidene hypophosphorous acid group, substituted or unsubstituted alkylidene phosphorous acid group, substituted or unsubstituted C1-C6 alkylene, substituted or unsubstituted C5-C20 arylidene, substituted or unsubstituted C1-C20 heteroarylidene, substituted or unsubstituted C1-C6 alkyleneoxy, substituted or unsubstituted C5-C20 aryleneoxy, substituted or unsubstituted C1-C6 alkylidene sulfonyl, substituted or unsubstituted C1-C6 alkylidene sulfonic acid group, substituted or unsubstituted C1-C6 alkylidene sulfonate group, and substituted or unsubstituted C1-C6 alkylidene phosphate group,
R_{b1}, R_{b2}, and R_{b3} are each selected from the group consisting of hydrogen, hydroxyl, carboxyl, halogen, mercapto, seleno, amido, amino, cyano, nitro, substituted or unsubstituted hypophosphorous acid group, substituted or unsubstituted phosphorous acid group, substituted or unsubstituted C1-C6 hydrocarbyl, substituted or unsubstituted C5-C20 aryl, substituted or unsubstituted C1-C20 heteroaryl, substituted or unsubstituted C1-C6 alkoxy, substituted or unsubstituted C5-C20 aryloxy, substituted or unsubstituted C1-C6 sulfonyl, substituted or unsubstituted C1-C6 sulfonic acid group, substituted or unsubstituted C1-C6 sulfonate group, and substituted or unsubstituted C1-C6 phosphate group,
at least one of R_{b1} and R_{b3} is selected from the group consisting of carboxyl, amino and hydroxyl;
linker has a structure represented by formula I-linker: 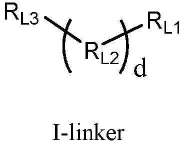
in formula I-linker, d is an integer selected from 0 to 17,
R_{L1}, and R_{L3} are each selected from the group consisting of -(CH₂)_{d1}COOH, amino, mercapto, carboxyl, and hydroxyl, wherein d1 is selected from 0 to 13,
R_{L2} is selected from the group consisting of -Arg-Gly-Asp-, -Arg-Gly-, -Arg-, imino, nitrilo, imido, substituted or unsubstituted C1-C20 alkylene, substituted or unsubstituted C1-C20 alkylidene ether, and substituted or unsubstituted C5-C20 arylidene ether;
PD is a compound and/or a derivative thereof obtained by condensing one or more of: 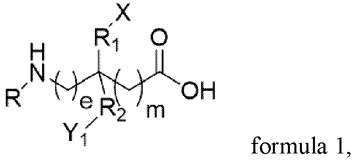 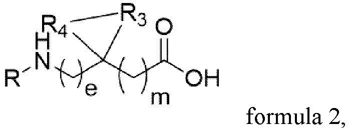 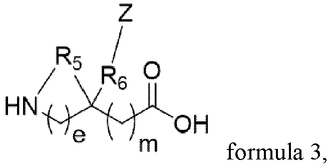 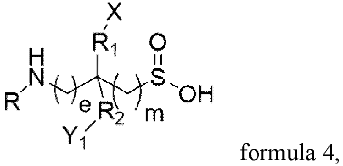 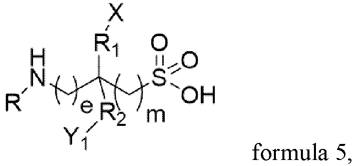 and 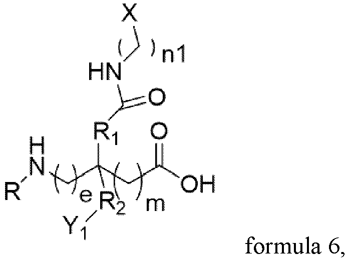
in formula 1 to formula 6, m, e and n1 are each an integer independently selected from 0 to 5,
R is selected from the group consisting of hydrogen, -OH, -NH2, substituted or unsubstituted C1-C6 hydrocarbyl, substituted or unsubstituted C5-C20 aryl, and substituted or unsubstituted C1-C20 heteroaryl,
R₁, R₂, R₃, R₄, R₅, and R₆ are independently selected from the group consisting of substituted or unsubstituted C1-C6 alkylene, substituted or unsubstituted C5-C20 arylidene, and substituted or unsubstituted C1-C20 heteroarylidene;
X, Y₁, and Z are independently selected from the group consisting of hydrogen, halogen, substituted or unsubstituted C1-C6 alkyl, substituted or unsubstituted C1-C6 alkoxy, substituted or unsubstituted C5-C20 aryloxy, -O-(CH2)q-O-R", mercapto, seleno, amido, amino, hydroxyl, cyano, nitro, carboxyl , substituted or unsubstituted C1-C6 ester, substituted or unsubstituted C1-C6 sulfonyl, substituted or unsubstituted C1-C6 sulfonic acid group, substituted or unsubstituted C1-C6 sulfonate group, sulfonamido group, phosphoric acid group, substituted or unsubstituted C1-C6 phosphate group, substituted or unsubstituted C5-C20 aryl, substituted or unsubstituted C5-C20 aryl heterocyclyl, substituted or and unsubstituted C2-C6 heterocyclyl; wherein, the substituted C1-C6 alkyl, substituted C1-C6 alkyl ether, and substituted C5-C20 aryl ether have a substituent group selected from the group consisting of -SH, SeH, hydroxyl, amino, carboxyl, sulfonic acid group , phosphoric acid group, halogen, and a combination thereof; q is 1 or 2; and R" is a C1-C6 alkyl;
when R₁, R₂, and R₆ are H, X, Y1, and Z are absent; and
when R₃, R₄, and R₅ are H, R₃ and R₄ do not form a ring, and R₅ together with the C atom to which it is bounded do not form a ring.

2. The polypeptide-like compound according to claim 1, wherein in formula I-ZT, n is an integer selected from 1 to 4;
Rₐ₁, Rₐ₂, and Rₐ₃ are each selected from the group consisting of substituted or unsubstituted alkylidene hypophosphorous acid group, substituted or unsubstituted alkylidene phosphorous acid group, substituted or unsubstituted C1-C6 alkylene, and substituted or unsubstituted C1-C6 alkyleneoxy;
R_{b1}, R_{b2}, and R_{b3} are each selected from the group consisting of hydrogen, hydroxyl, carboxyl, halogen, amino, substituted or unsubstituted hypophosphorous acid group, substituted or unsubstituted phosphorous acid group, substituted or unsubstituted C1-C6 hydrocarbyl, and substituted or unsubstituted C1-C6 alkoxy; and
at least one of R_{b1} and R_{b3} is selected from the group consisting of carboxyl, amino and hydroxyl.

3. The polypeptide-like compound according to claim 2, wherein ZT has a structure selected from formula ZT1 to ZT4: 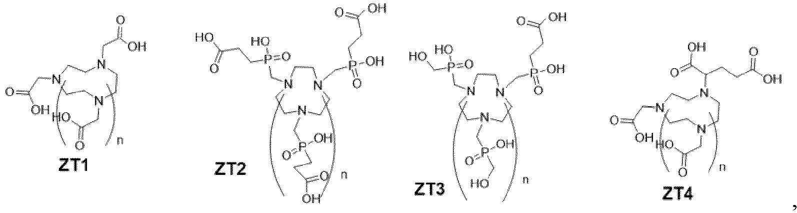 and
in formula ZT1 to ZT4, n is an integer from 1 to 4.

4. The polypeptide-like compound according to claim 1, wherein linker has a structure selected from formula linker 1 to linker 9: 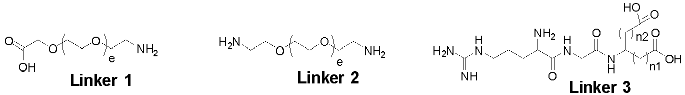 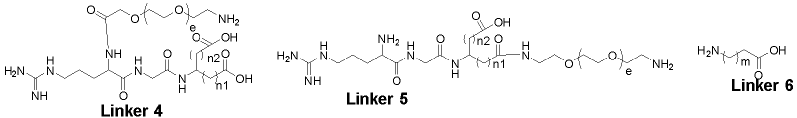 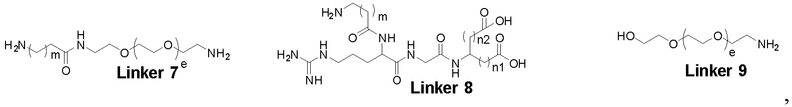 and
in formula linker 1 to linker 9, e, m, n₁, and n₂ are each an integer independently selected from 0 to 16.

5. The polypeptide-like compound according to any one of claims 1 to 4, wherein PD has a structure selected from the group consisting of: 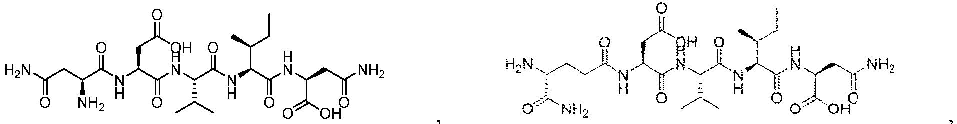 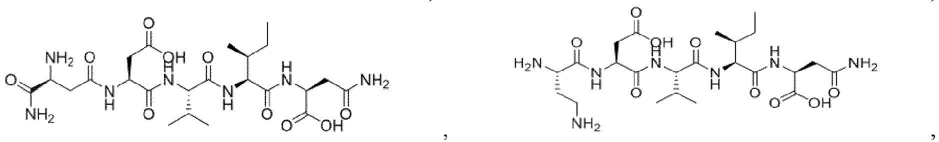 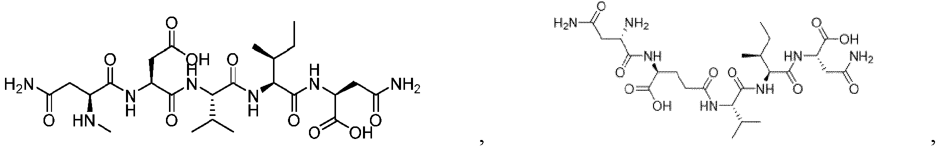 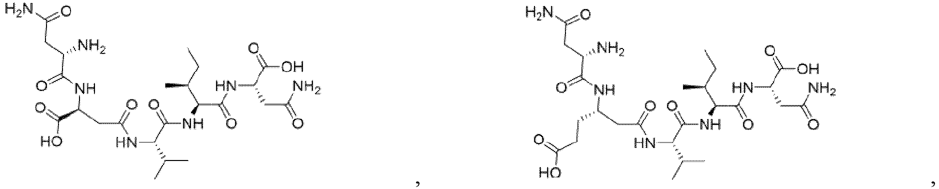 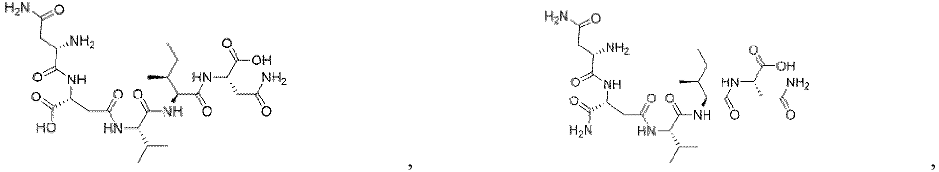 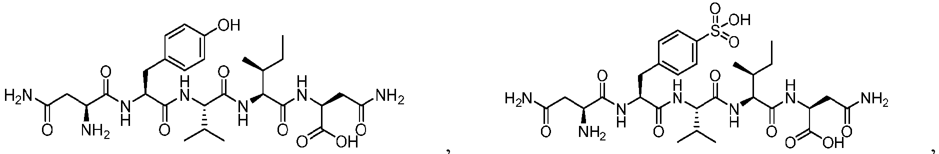 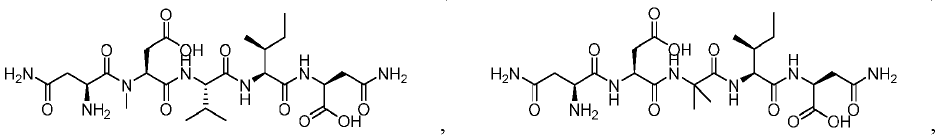 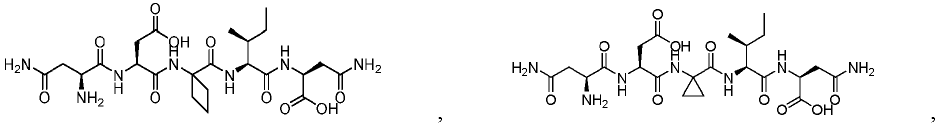 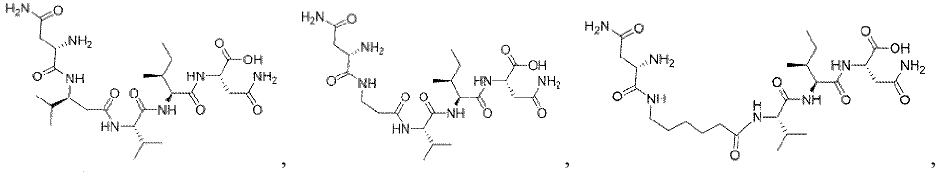 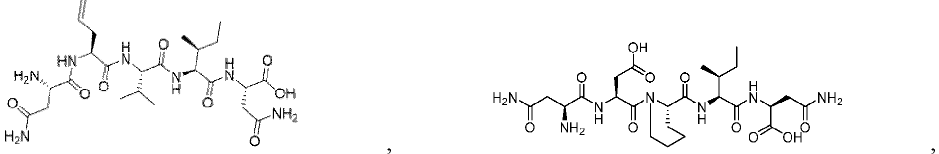 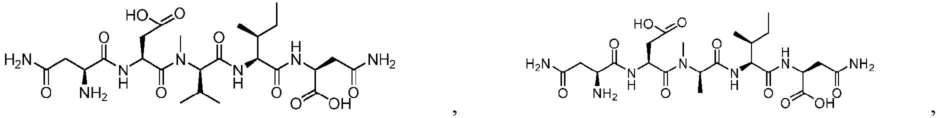 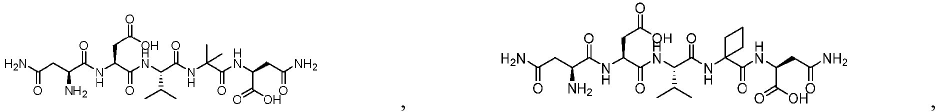 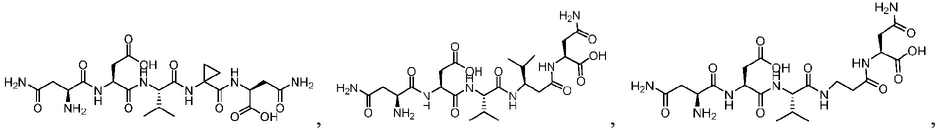 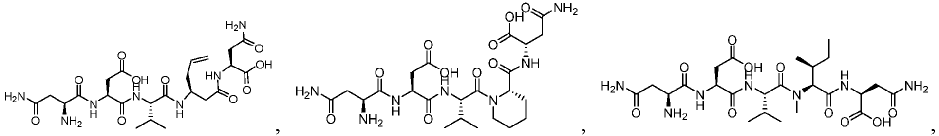 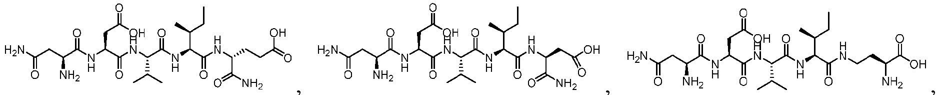 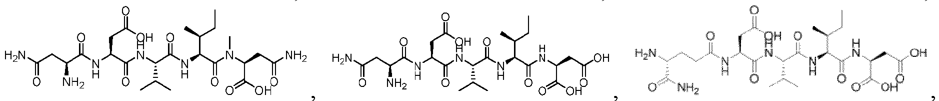 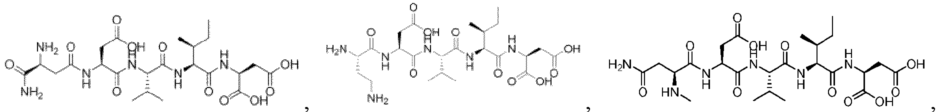 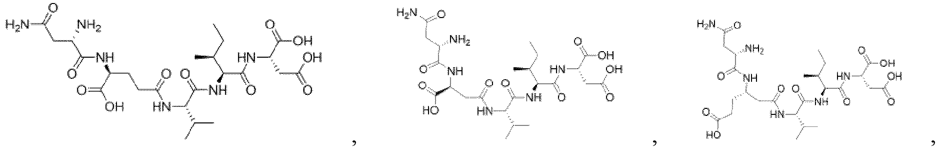 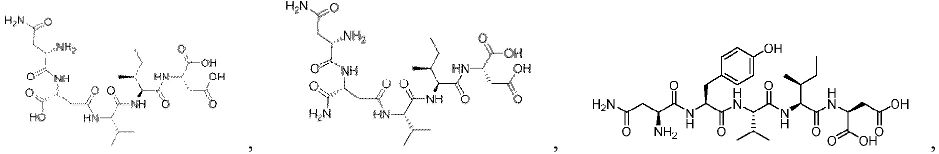 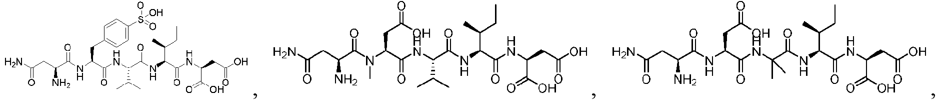 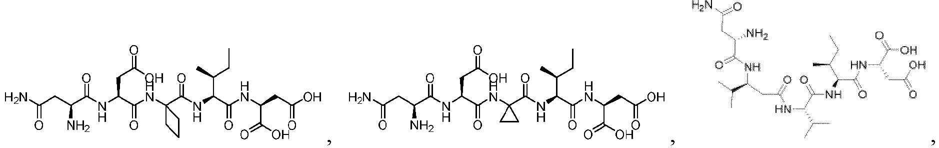 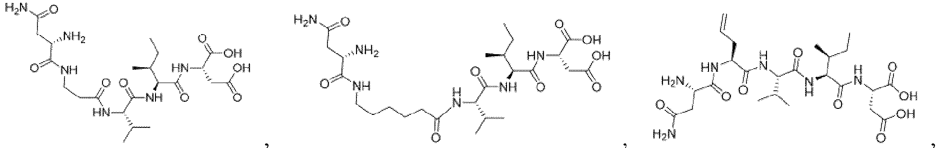 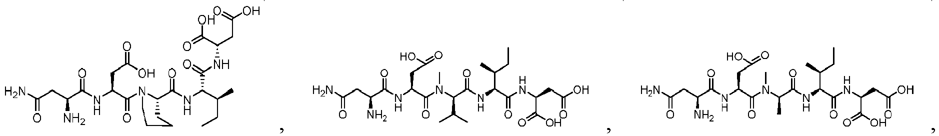 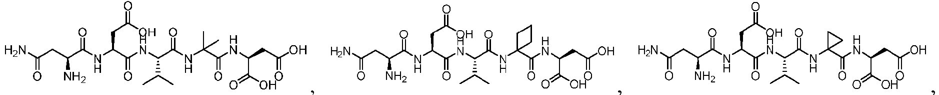 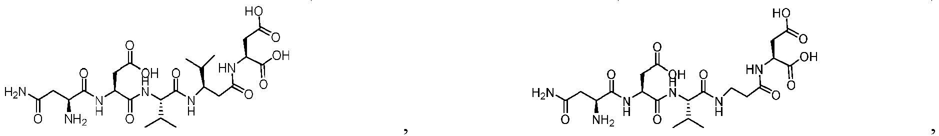 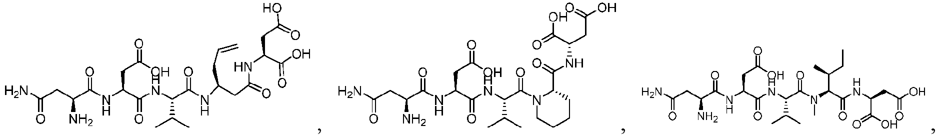 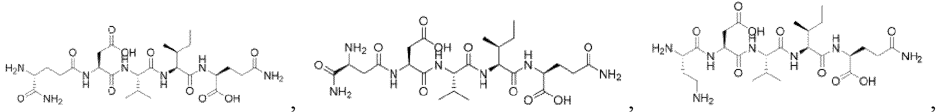 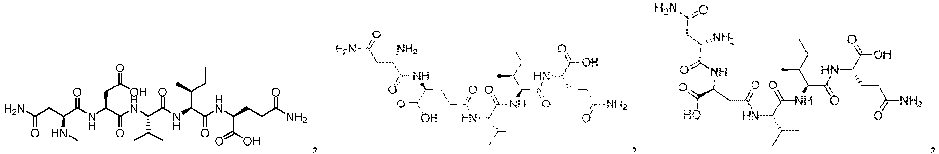 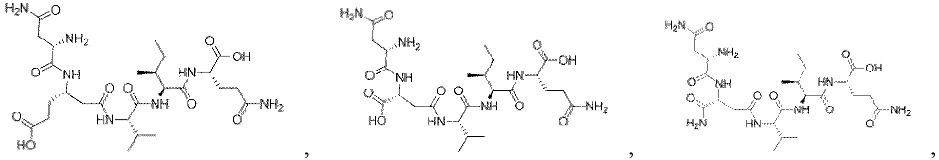 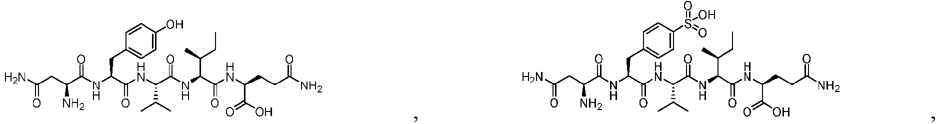 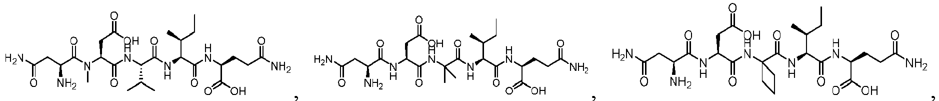 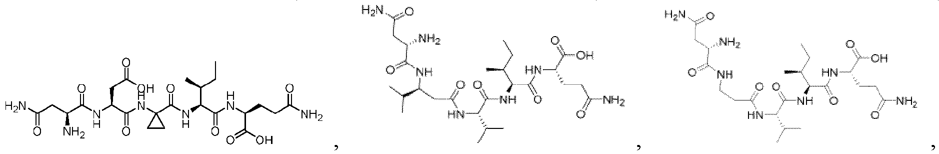 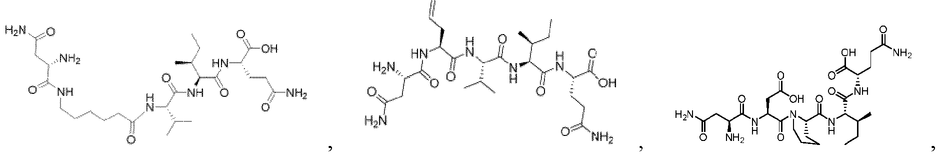 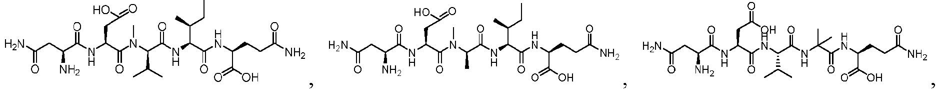 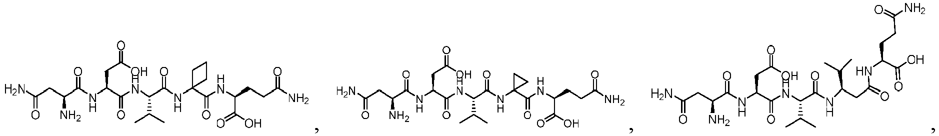 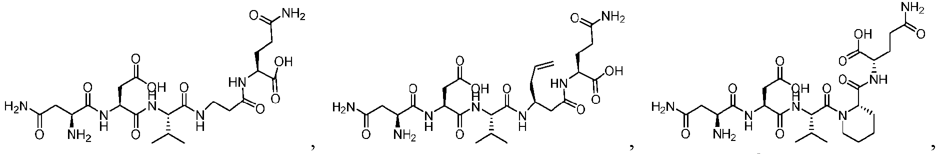 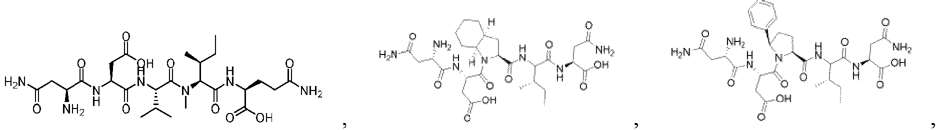 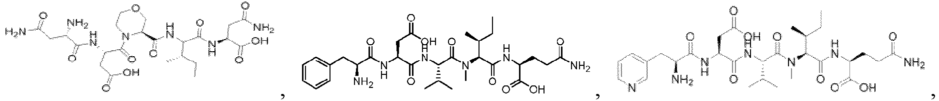 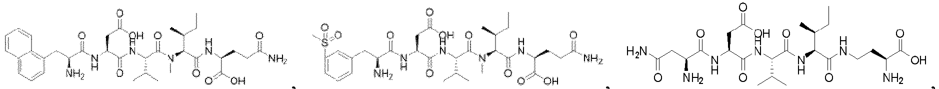 and

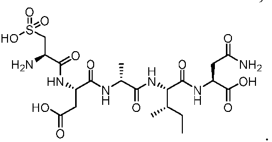

6. The polypeptide-like compound according to any one of claims 1 to 4, wherein the polypeptide-like compound has a structure selected from the group consisting of: 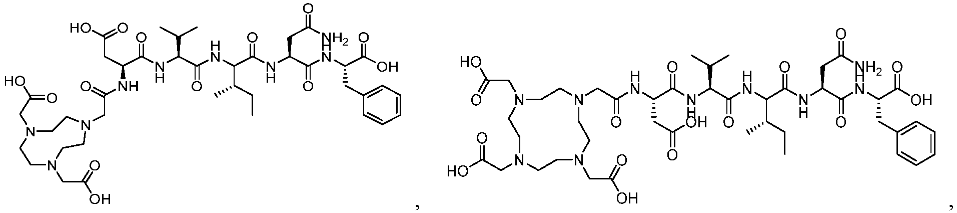 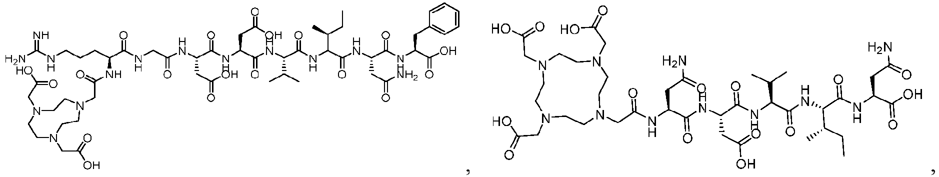 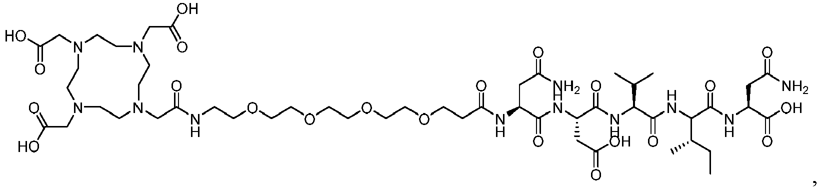 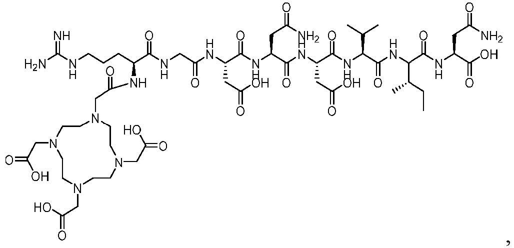 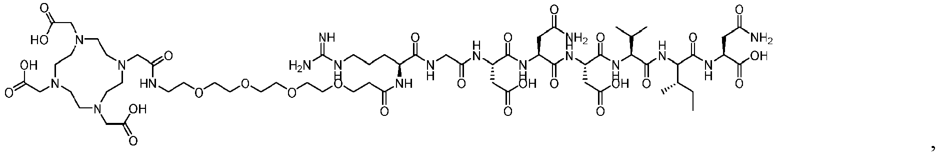 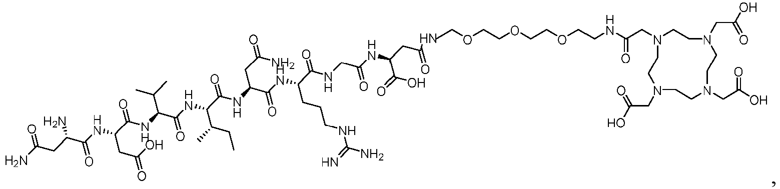 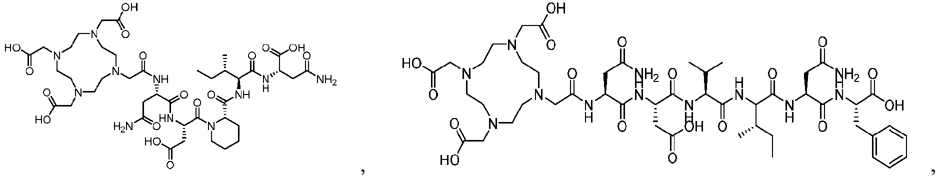 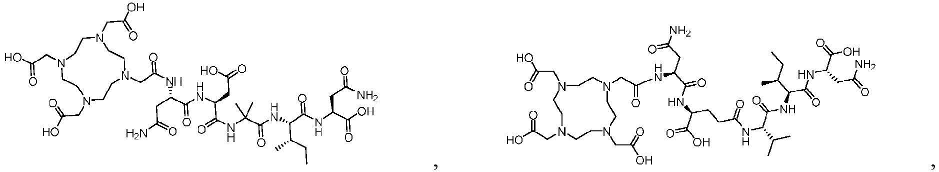 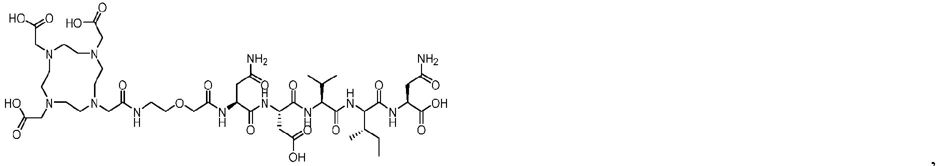 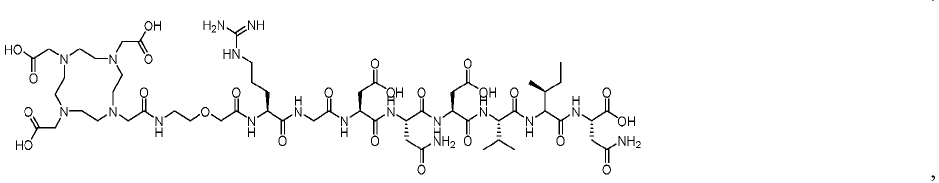 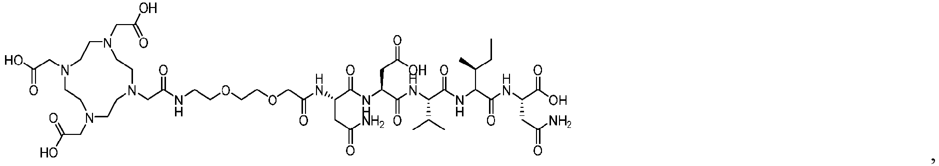 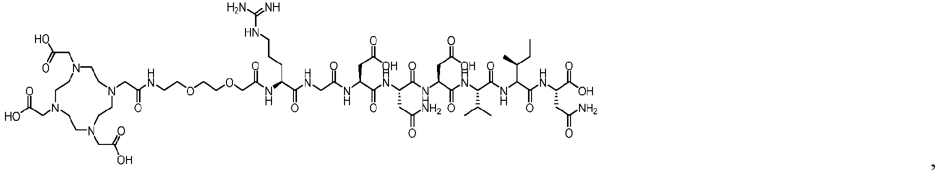 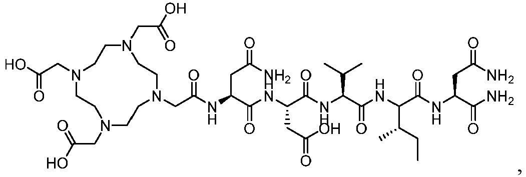 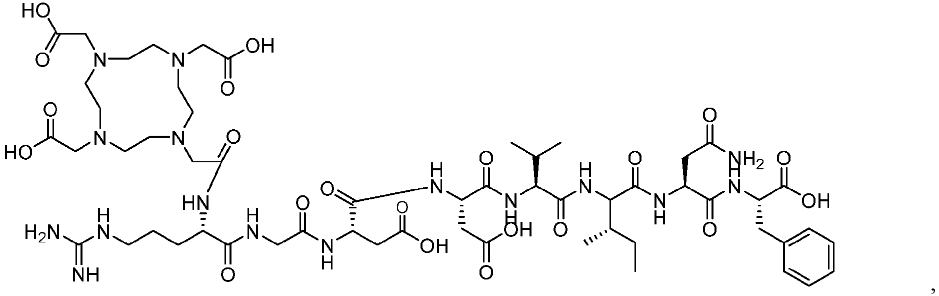 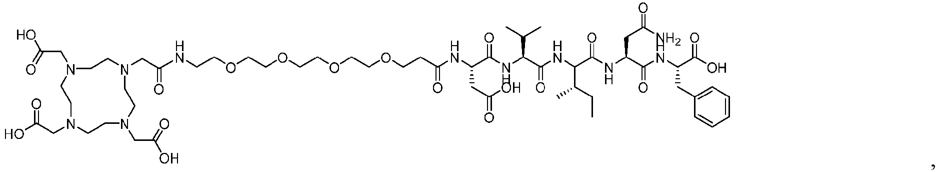 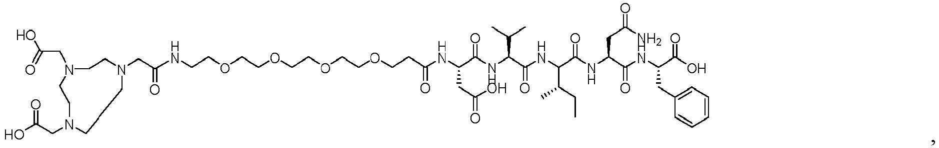 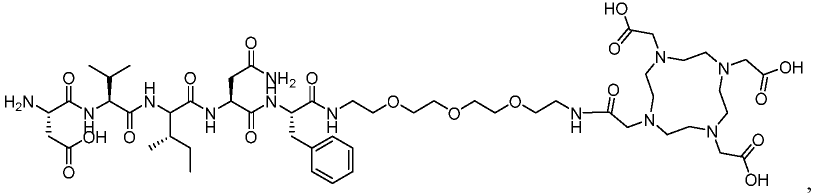 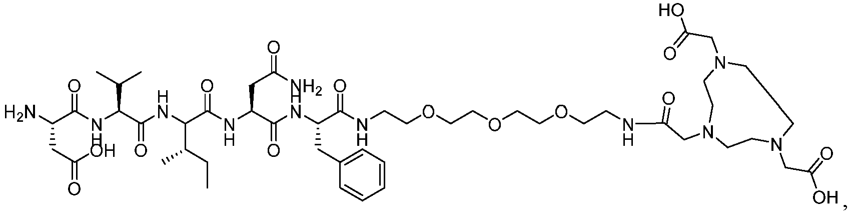 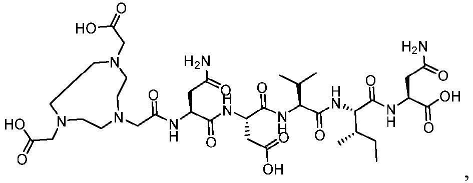 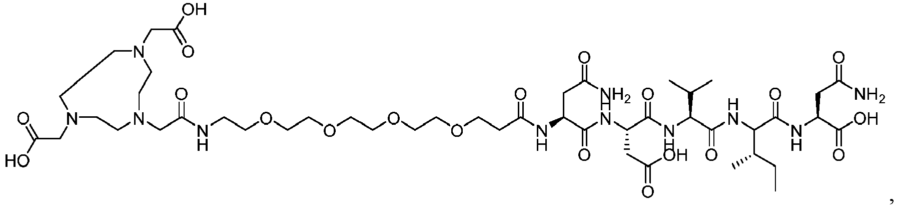 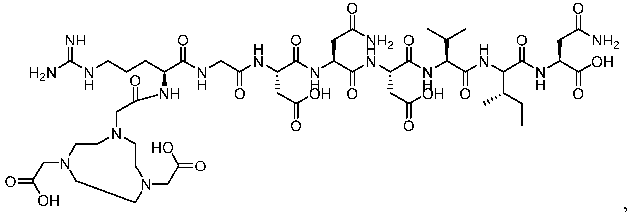 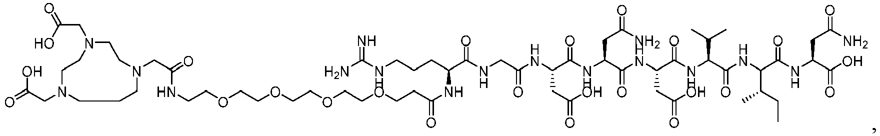 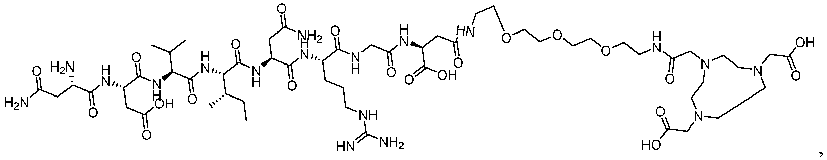 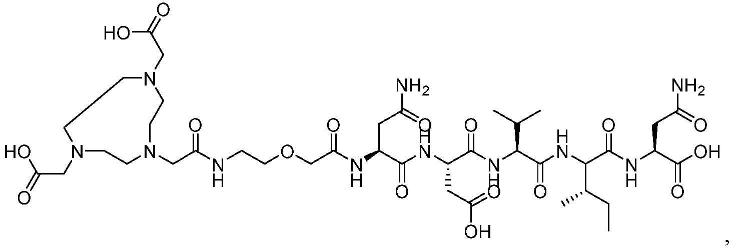 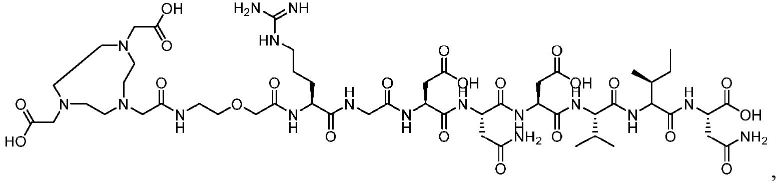 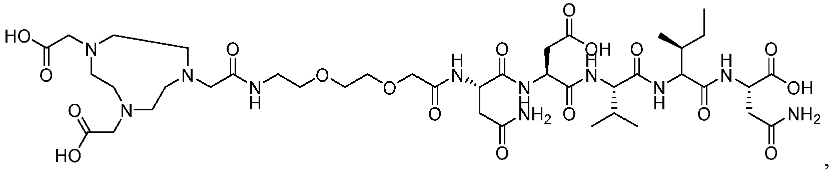 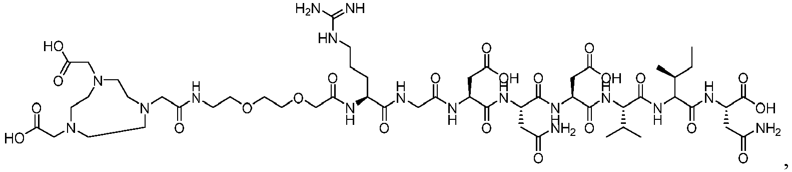 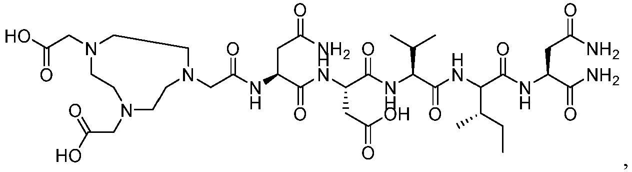 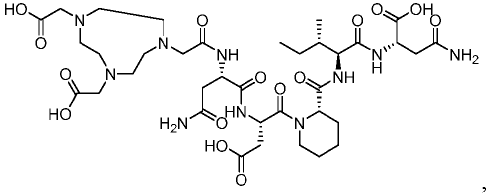 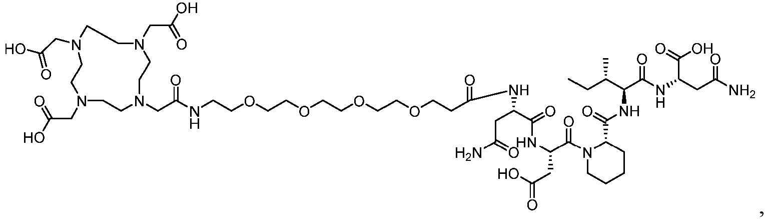 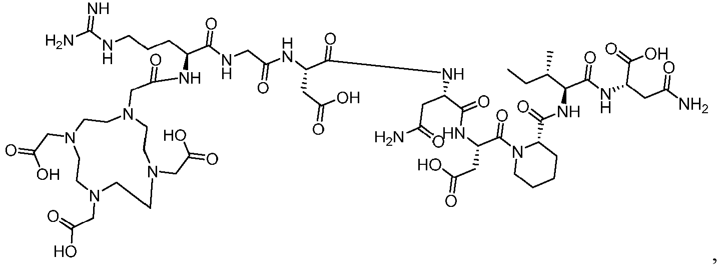 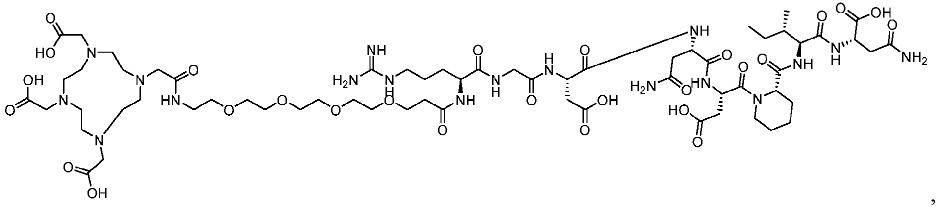 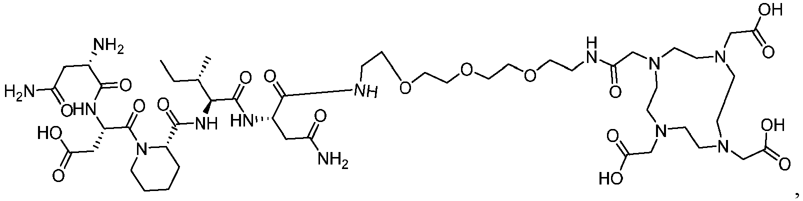 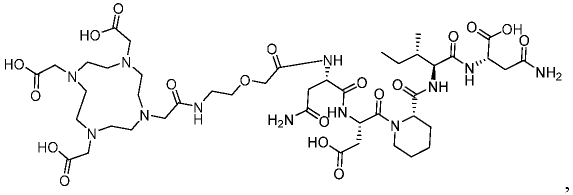 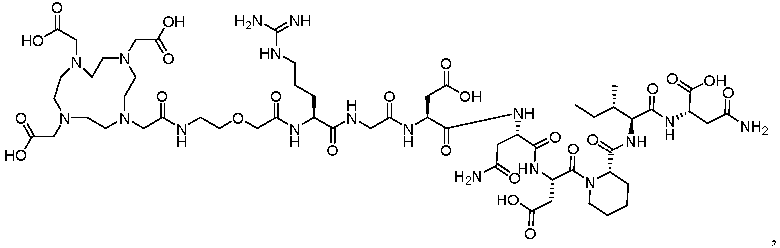 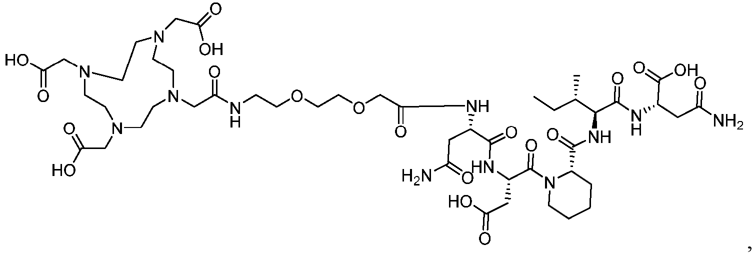 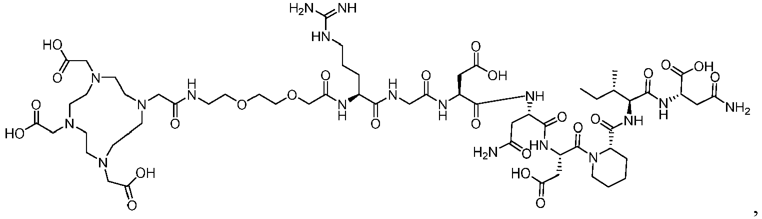 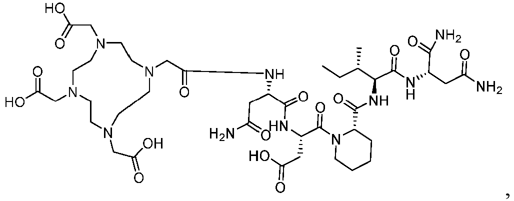 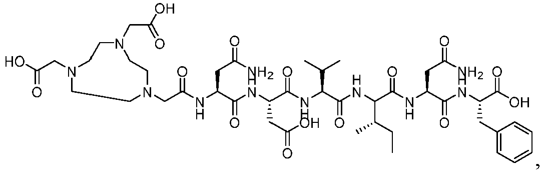 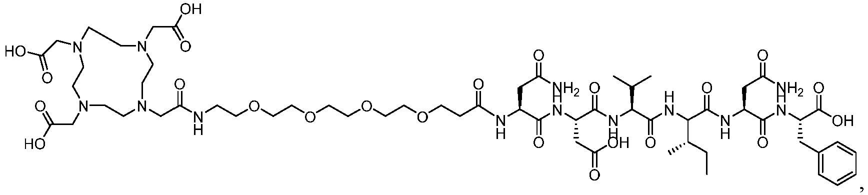 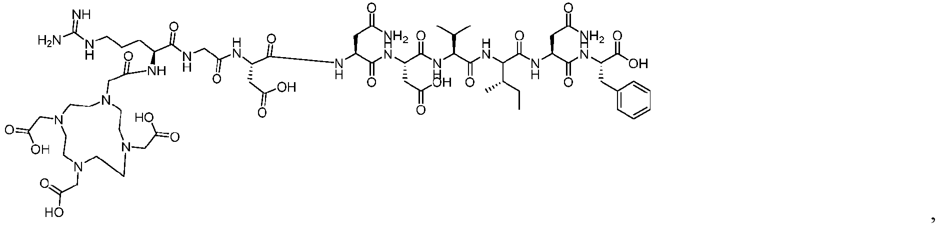 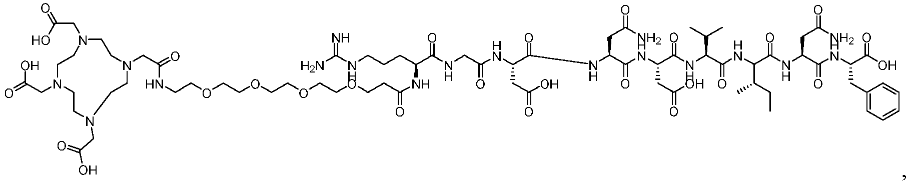 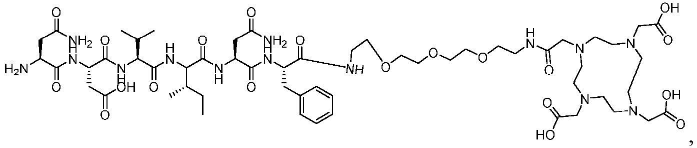 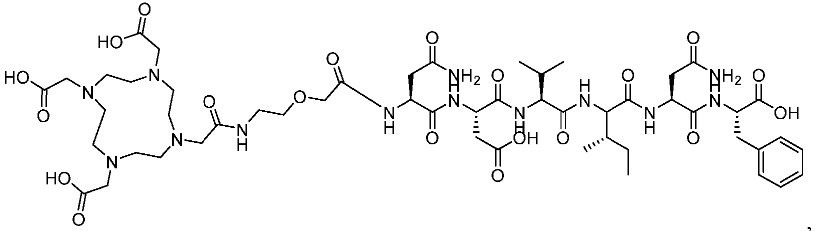 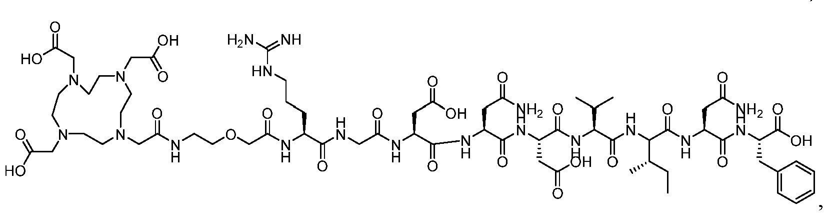 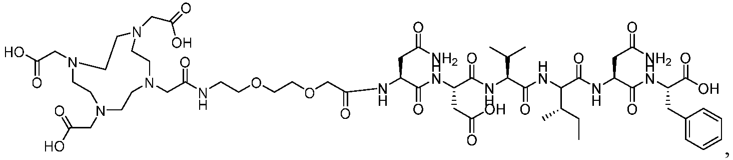 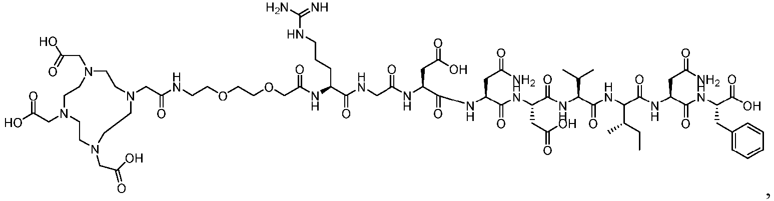 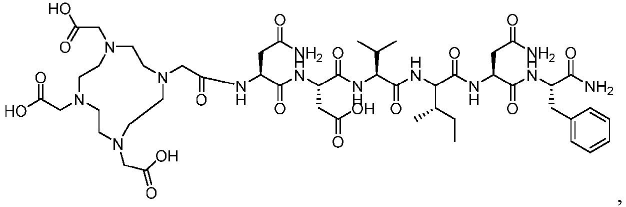 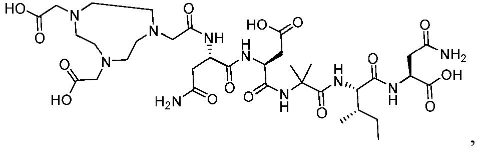 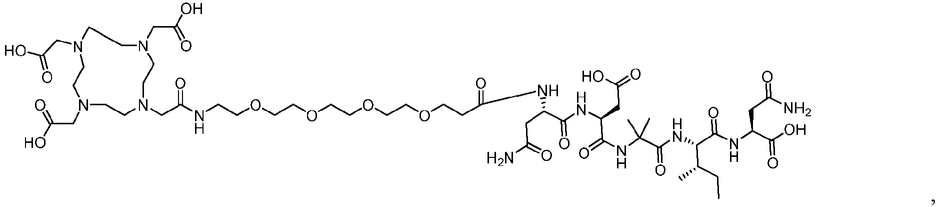 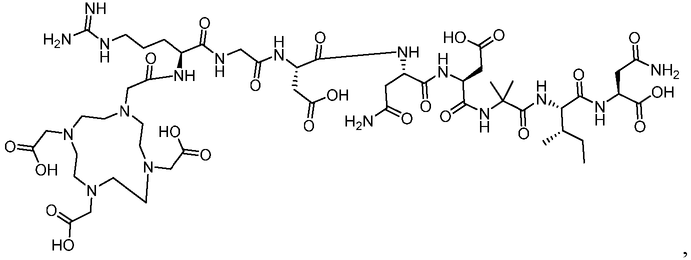 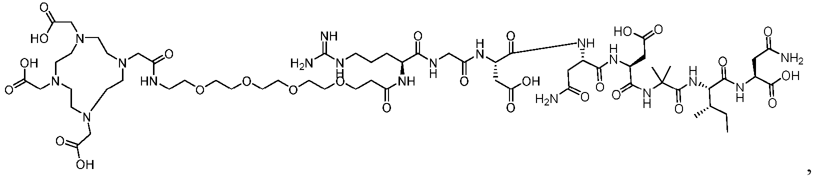 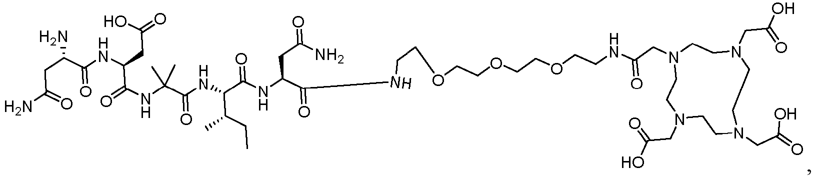 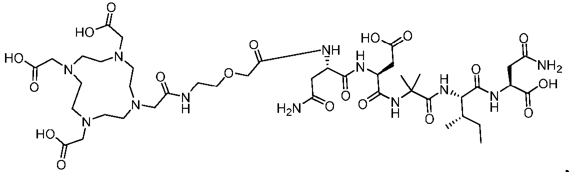 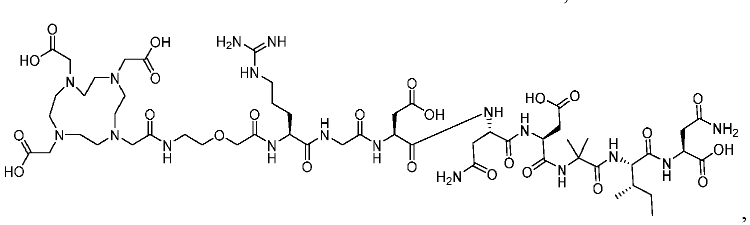 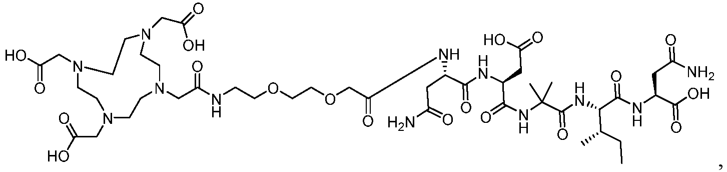 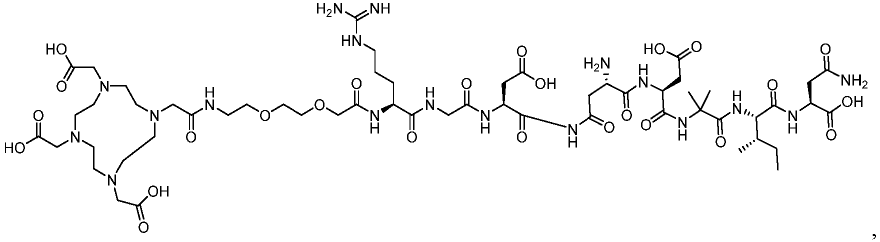 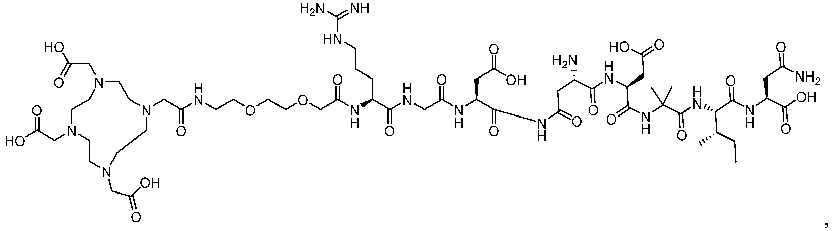 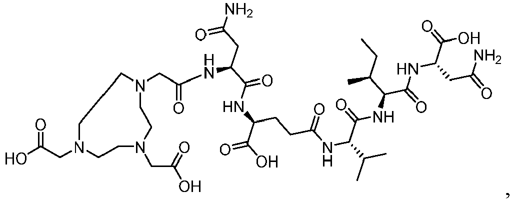 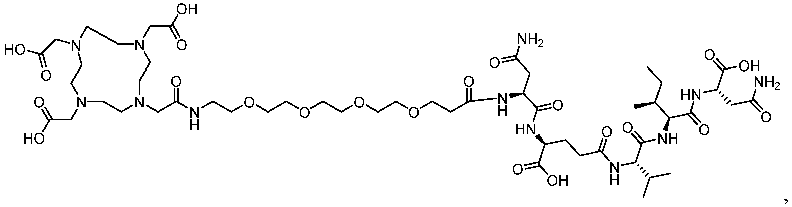 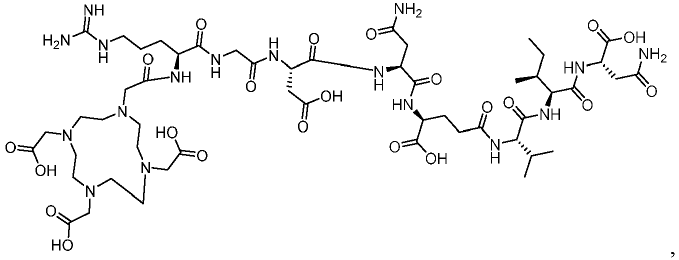 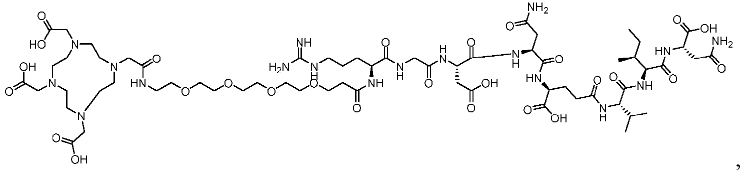 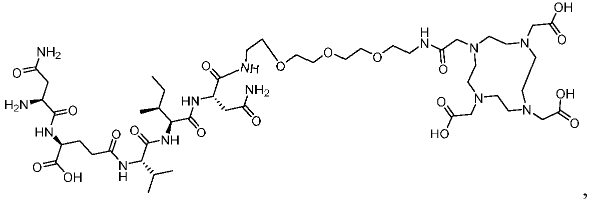 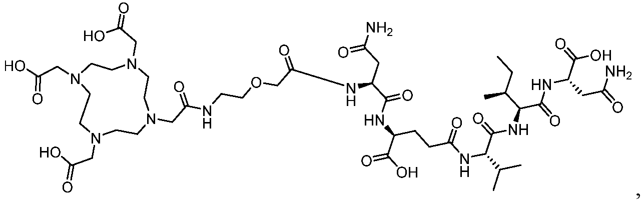 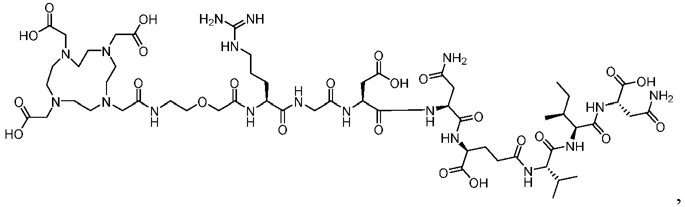 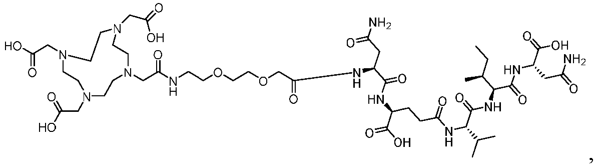 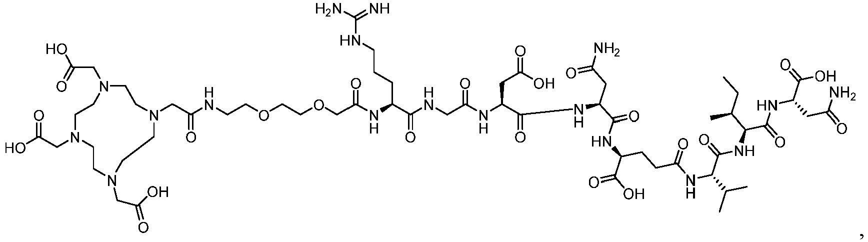 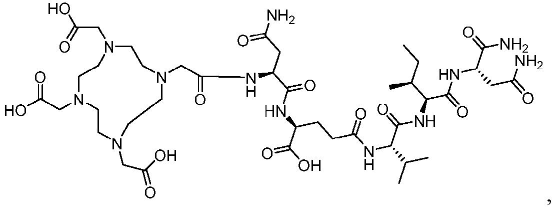 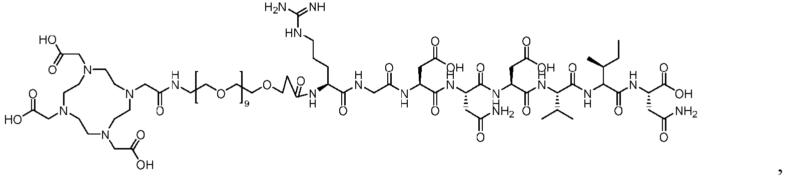 and

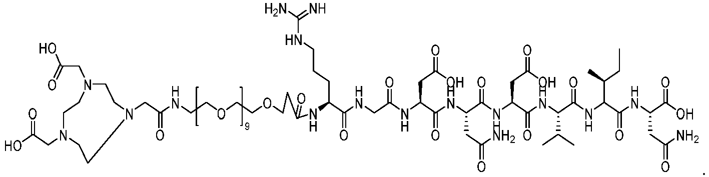

7. A polypeptide-like coordination compound formed by reacting the polypeptide-like compound according to any one of claims 1 to 6 with a metal; wherein the metal is selected from the group consisting of Cu, Ga, In, Bi, Gd, ¹⁷⁷Lu, ⁶⁴Cu, ⁶⁸Ga, ¹¹¹In , ²¹¹At, ²²⁵Ac, ⁹⁰Y, ²¹³Bi, At, and an isotope thereof.

8. The polypeptide-like coordination compound according to claim 7, wherein the polypeptide-like coordination compound has a structure selected from the group consisting of: 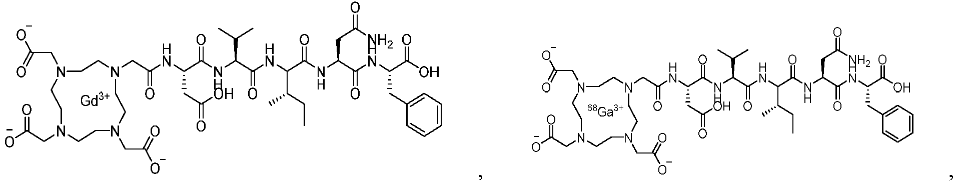 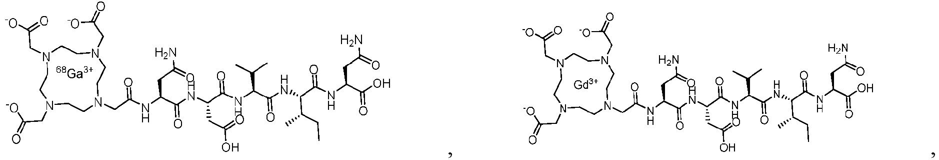 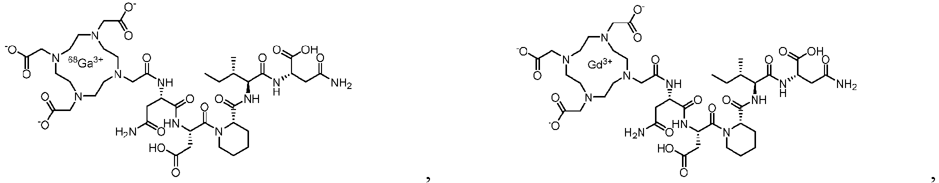 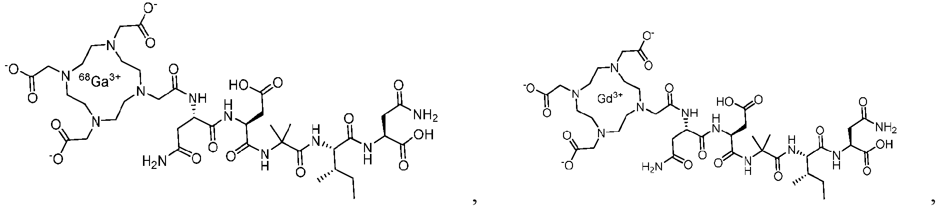 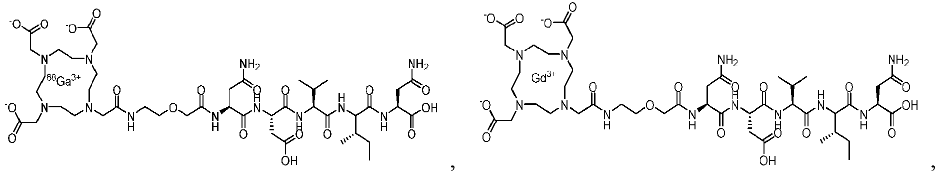 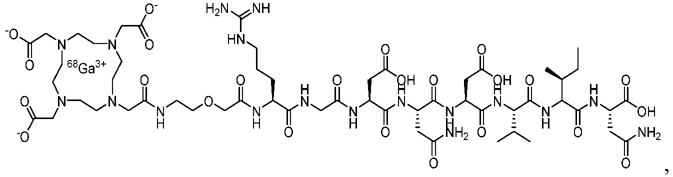 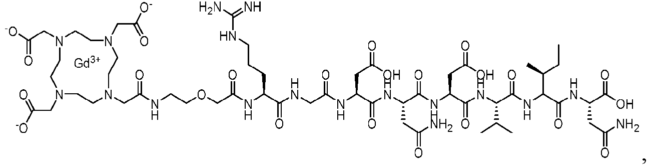 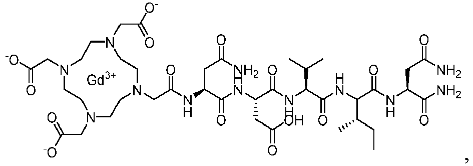 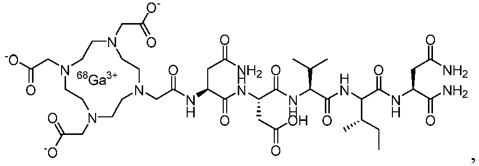 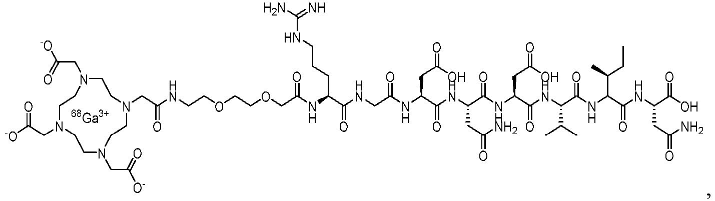 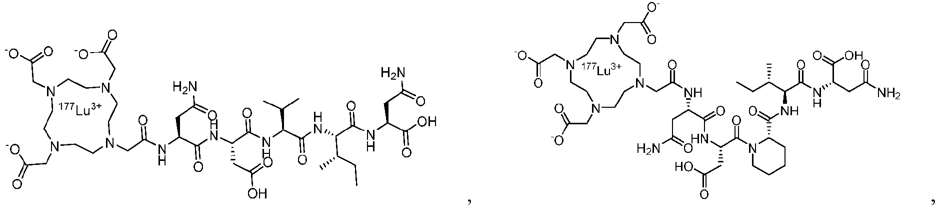 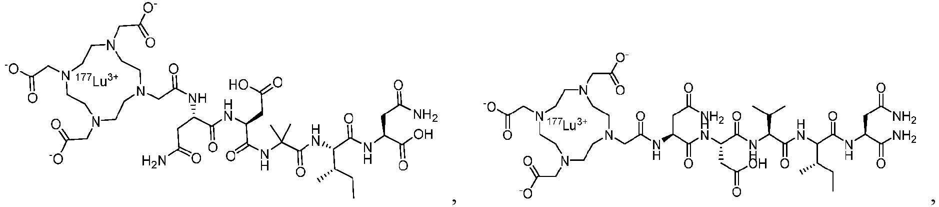 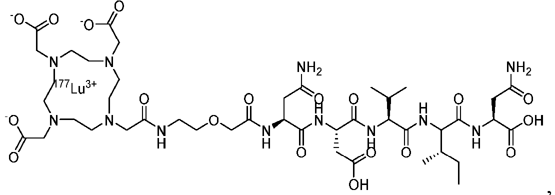 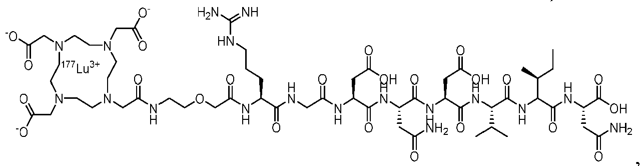 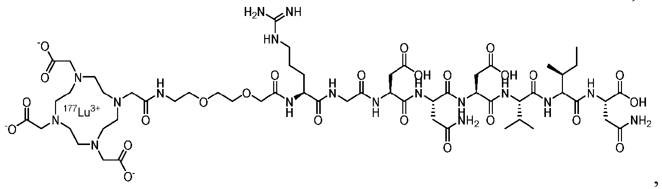 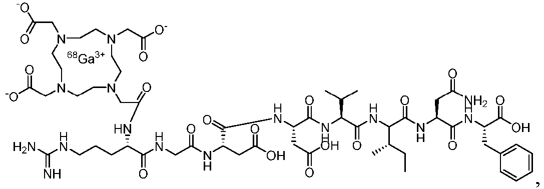 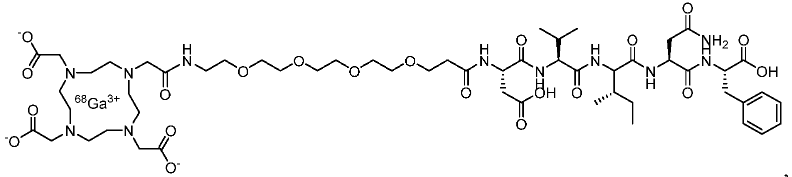 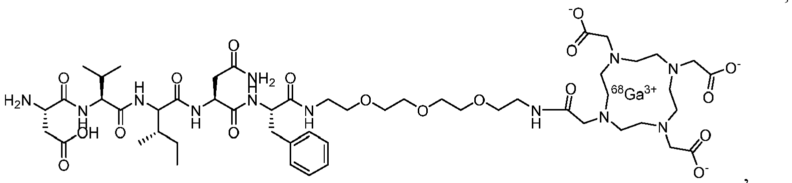 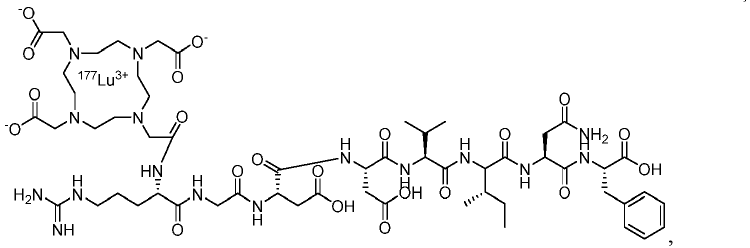 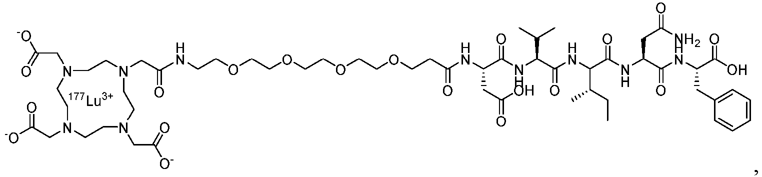 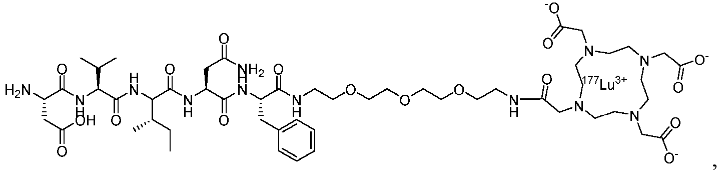 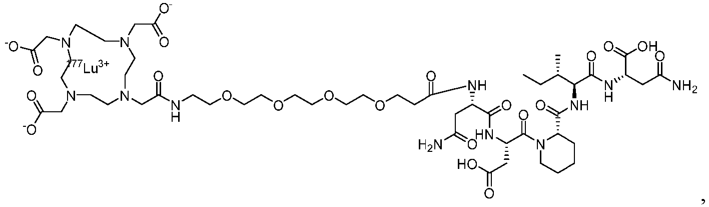 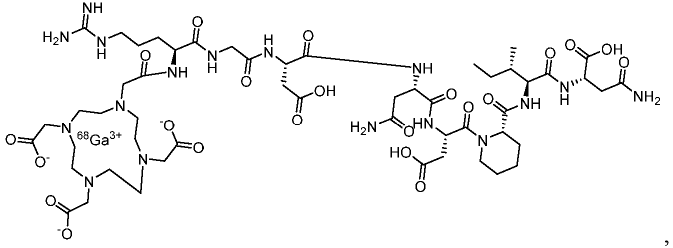 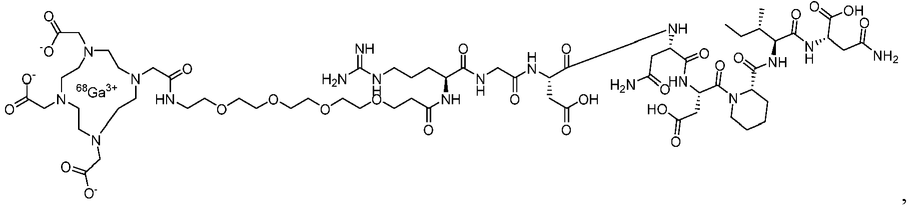 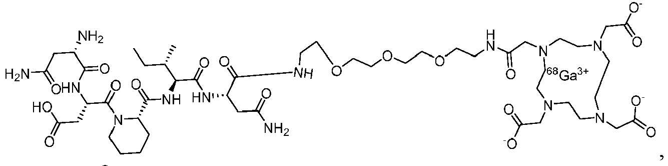 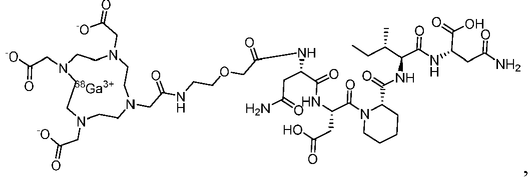 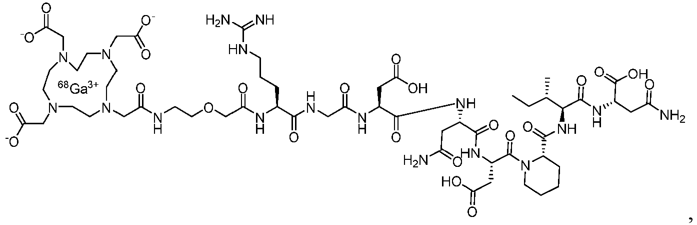 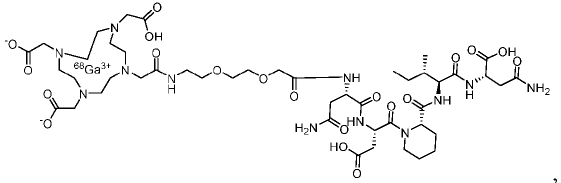 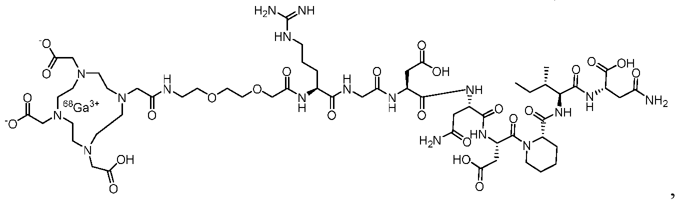 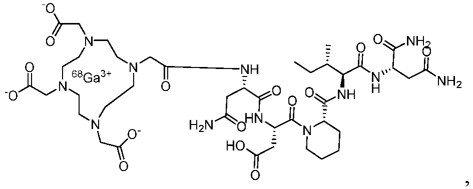 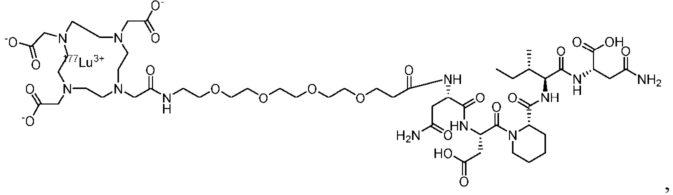 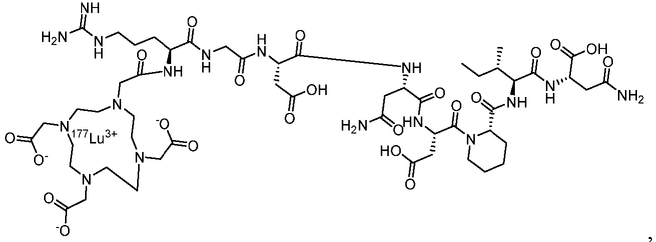 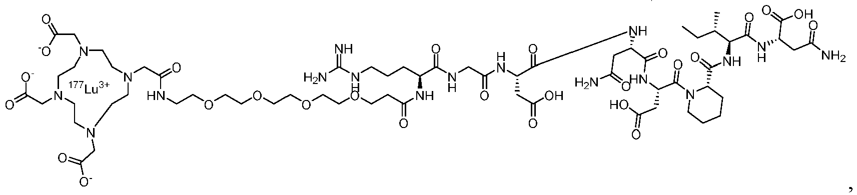 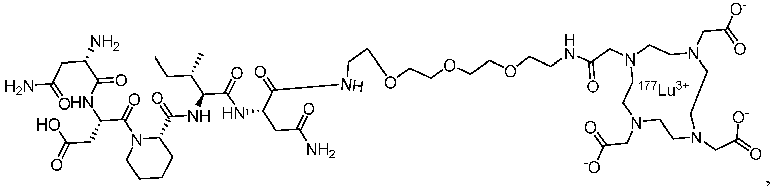 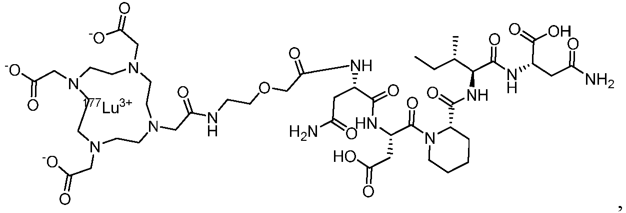 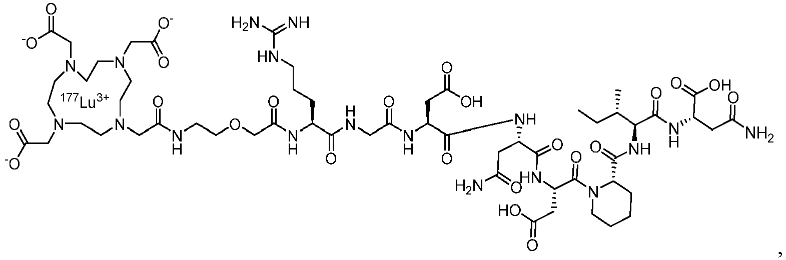 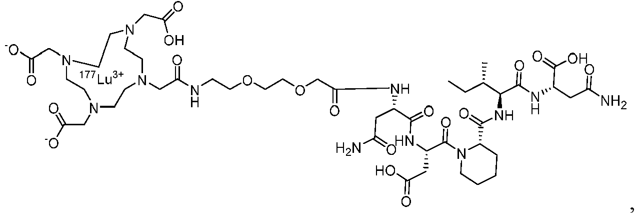 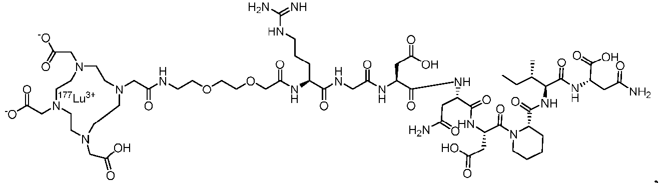 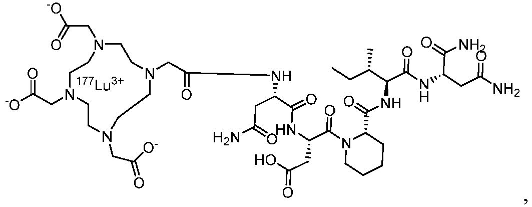 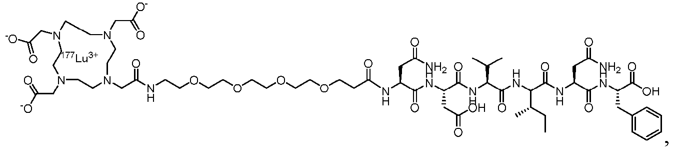 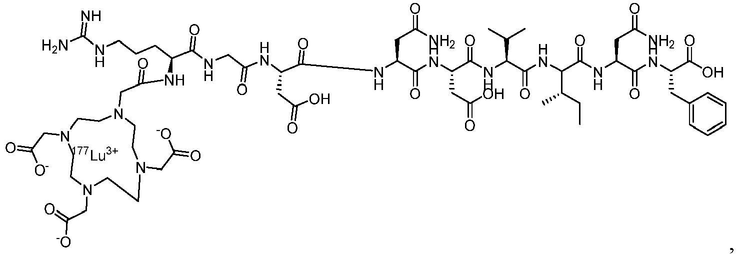 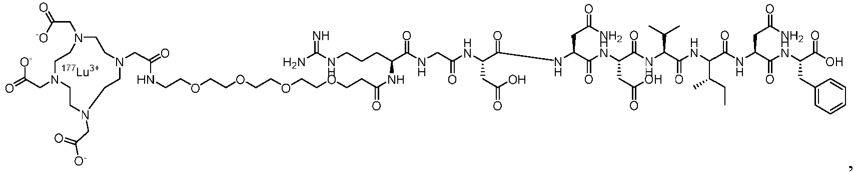 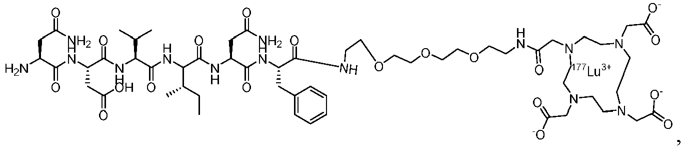 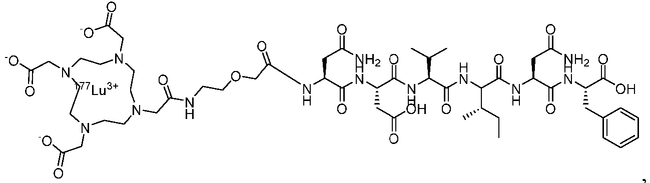 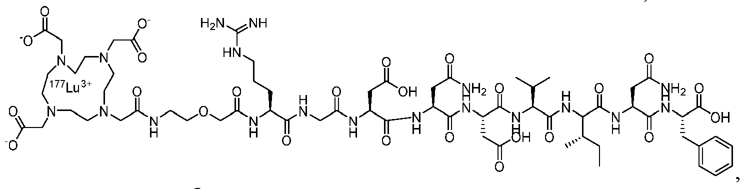 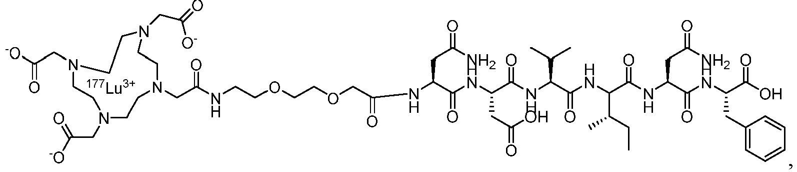 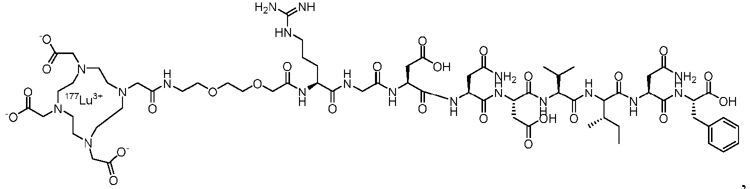 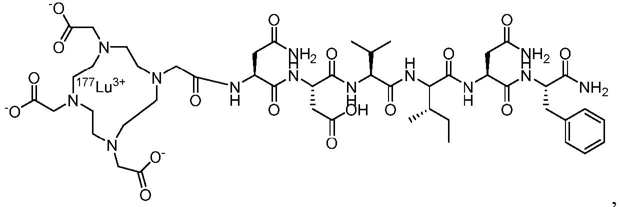 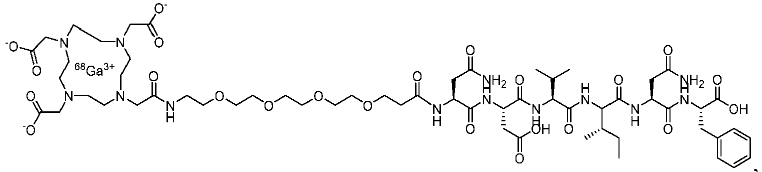 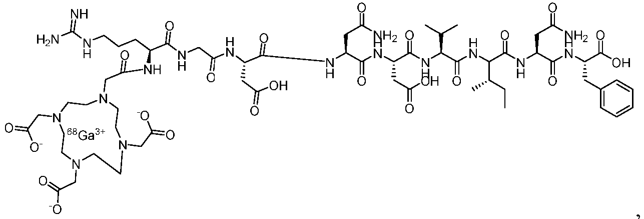 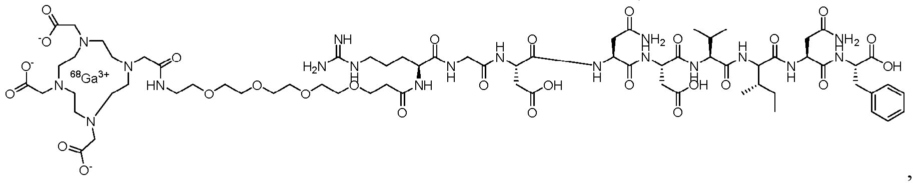 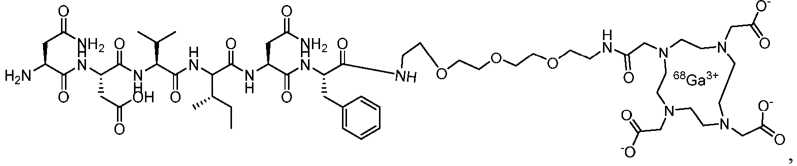 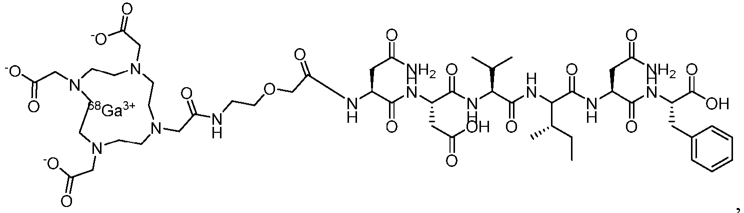 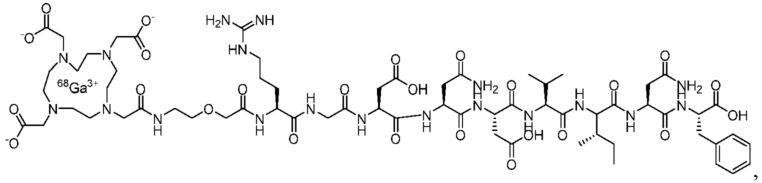 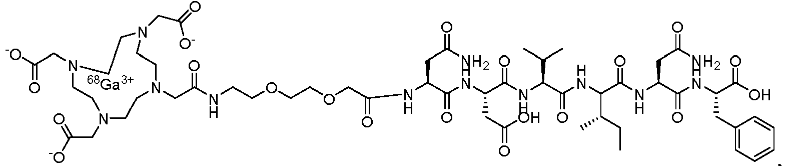 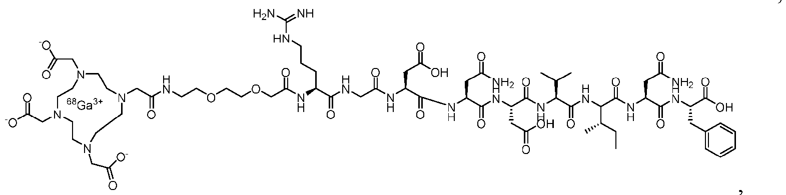 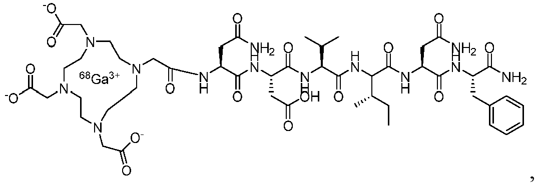 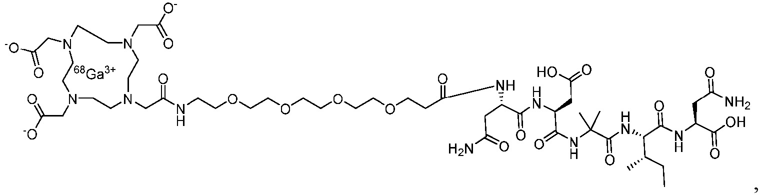 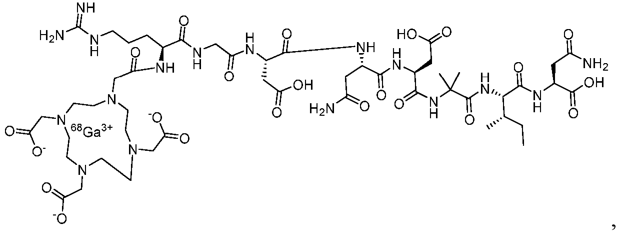 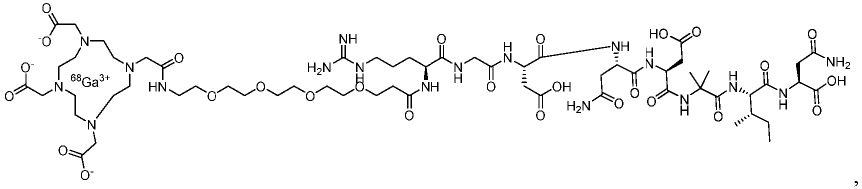 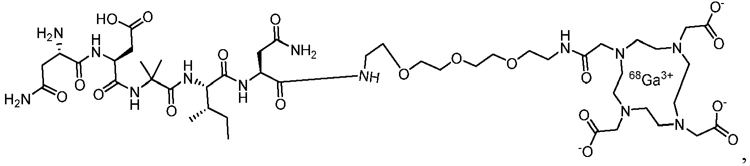 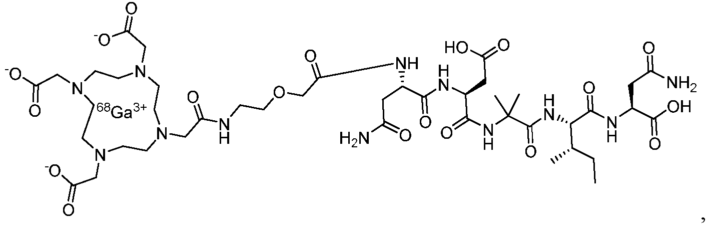 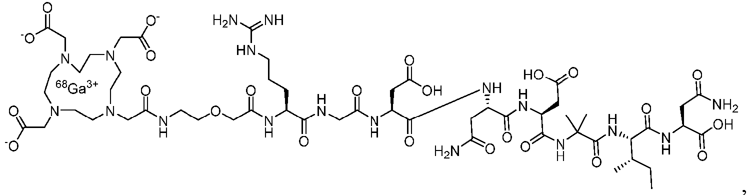 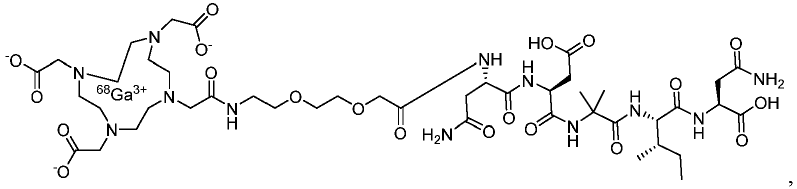 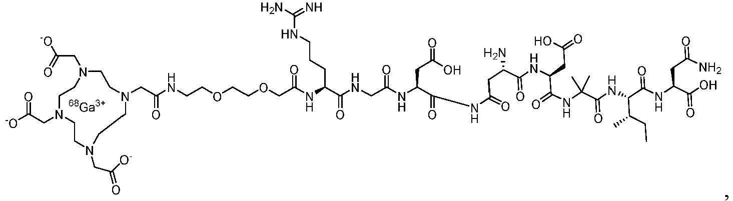 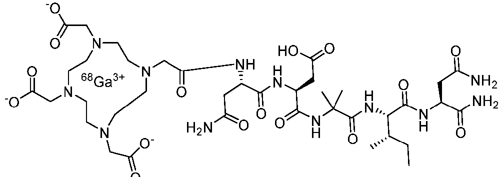 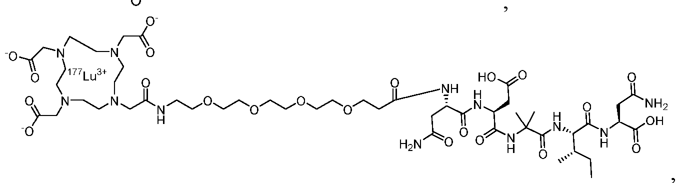 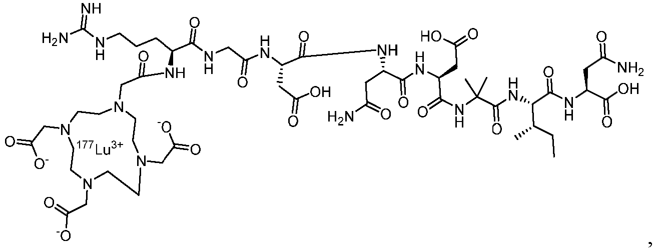 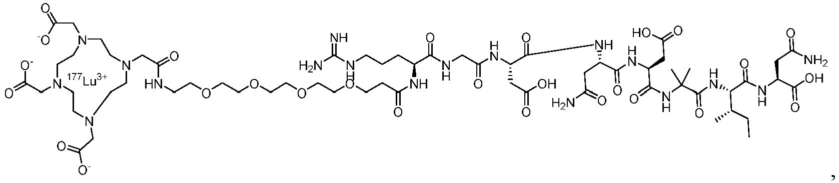 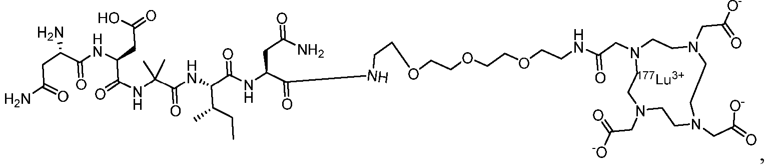 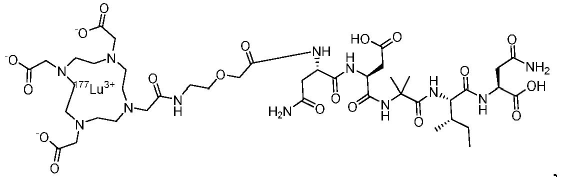 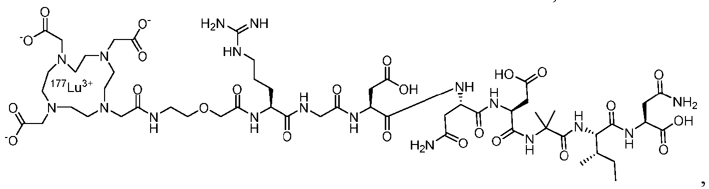 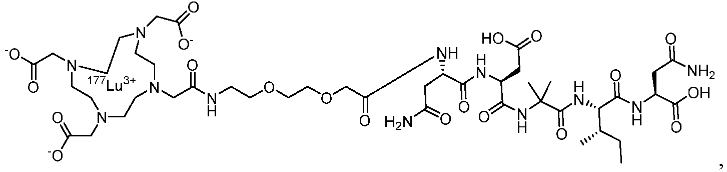 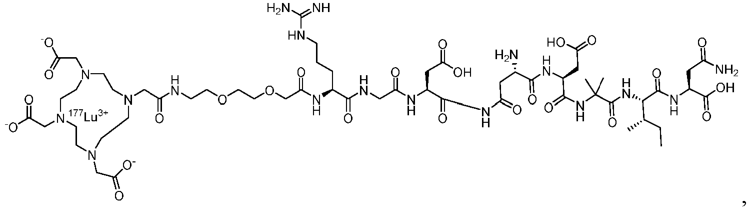 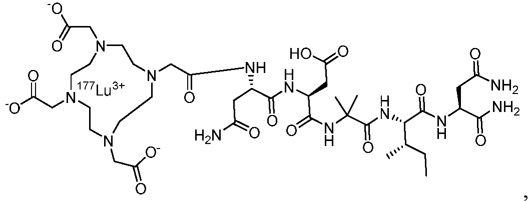 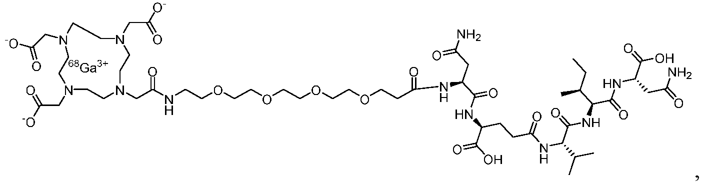 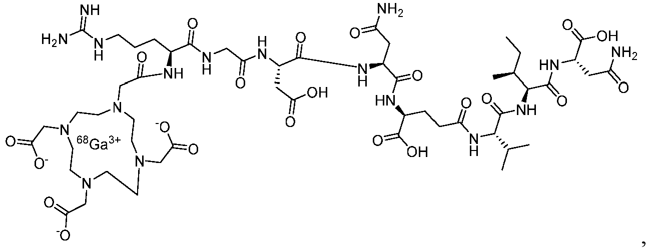 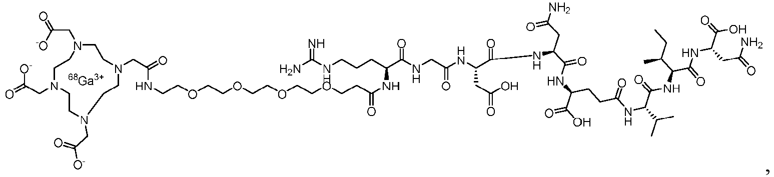 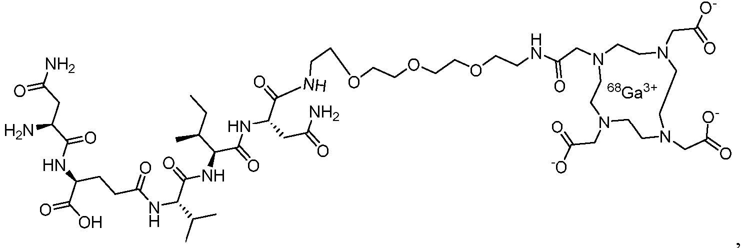 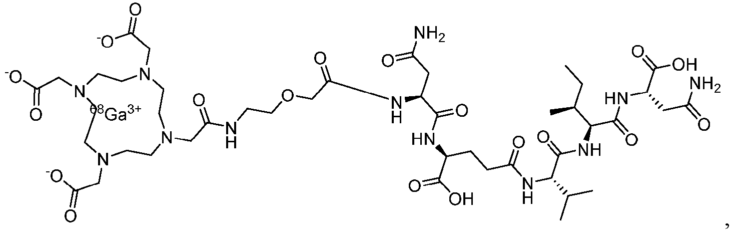 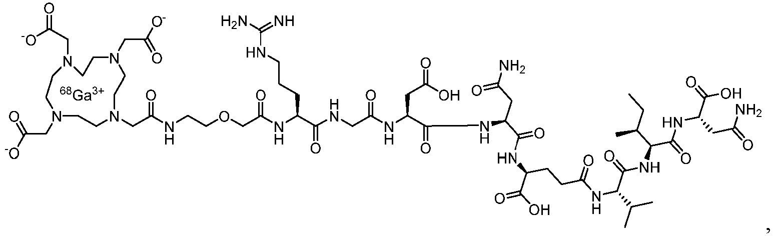 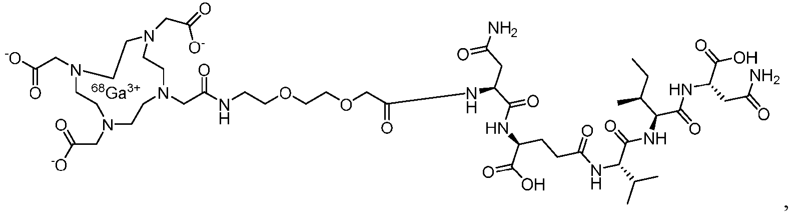 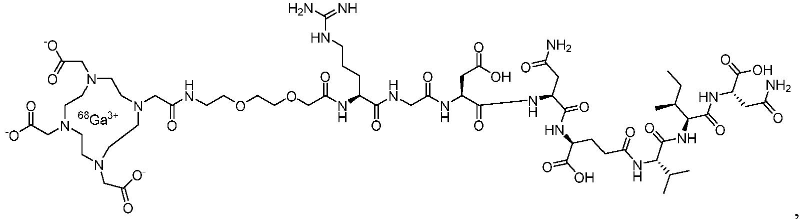 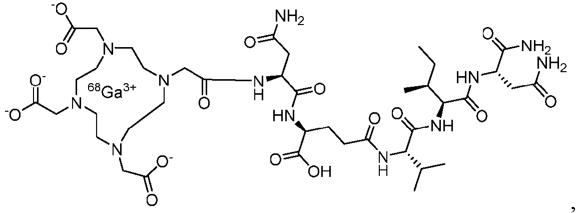 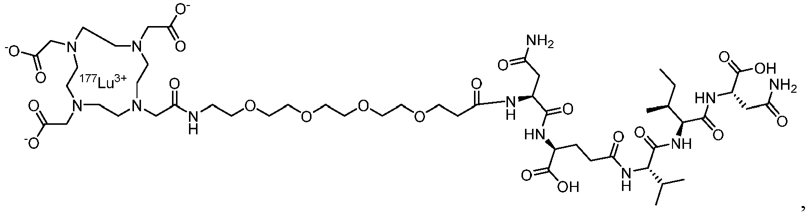 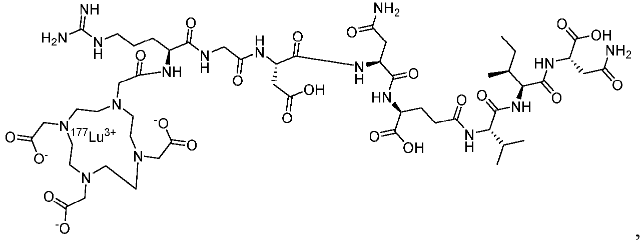 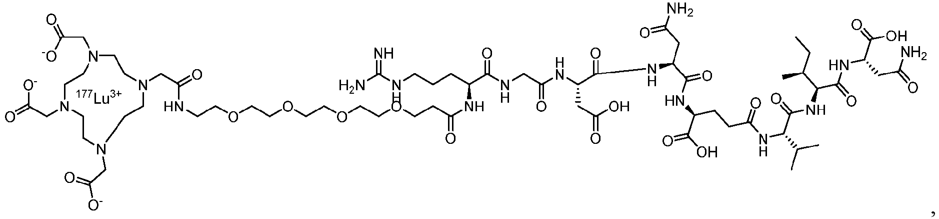 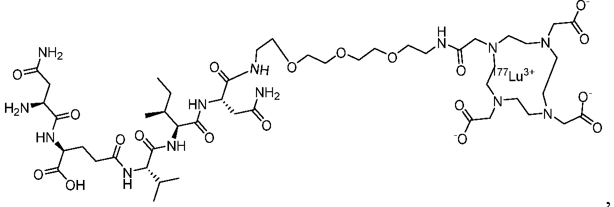 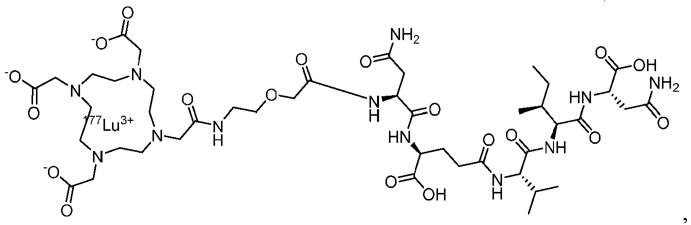 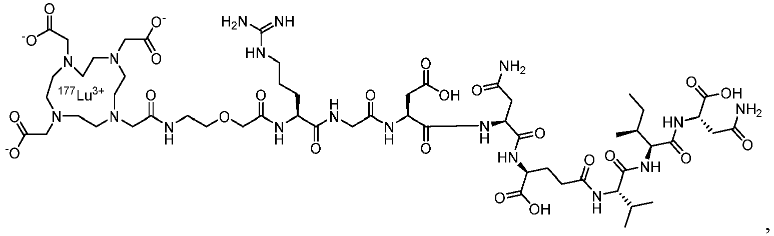 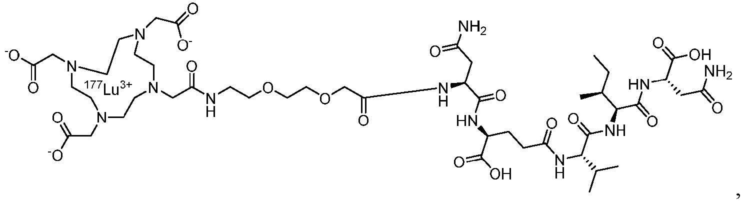 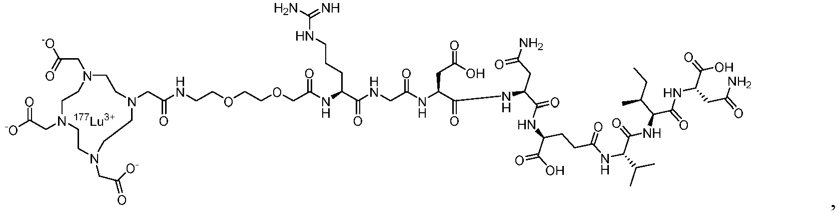 and

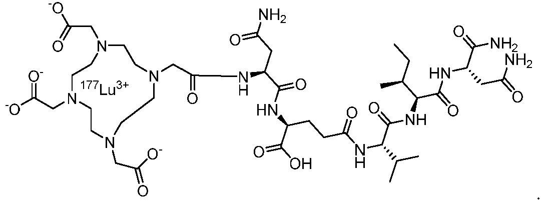

9. Use of the polypeptide-like compound according to any one of claims 1 to 6 and/or the polypeptide-like coordination compound according to claim 7 or 8 in the manufacture of a medicament for diagnosing and/or treating a tumor.

10. An integrin inhibitor comprising the polypeptide-like compound according to any one of claims 1 to 6 and/or the polypeptide-like coordination compound according to claim 7 or 8.

11. A pharmaceutical composition comprising the polypeptide-like compound according to any one of claims 1 to 6, and/or the polypeptide-like coordination compound according to claim 7 or 8, and a pharmaceutically acceptable excipient.
